(19) Europäisches Patentamt
European Patent Office
Office européen des brevets

(11) EP 1 627 870 A1

(12) **EUROPEAN PATENT APPLICATION**
published in accordance with Art. 158(3) EPC

(43) Date of publication:
**22.02.2006 Bulletin 2006/08**

(21) Application number: **04733467.7**

(22) Date of filing: **17.05.2004**

(51) Int Cl.:
***C07D 207/16*** (1985.01) ***C07D 401/12*** (1990.01)
***C07D 403/12*** (1990.01)

(86) International application number:
**PCT/JP2004/006983**

(87) International publication number:
**WO 2004/101514 (25.11.2004 Gazette 2004/48)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **15.05.2003 JP 2003137062**

(71) Applicant: **TAISHO PHARMACEUTICAL CO., LTD**
**Tokyo 170-8633 (JP)**

(72) Inventors:
- **FUKUSHIMA, Hiroshi**
**Toshima-ku, Tokyo 1708633 (JP)**
- **TAKAHASHI, Masato**
**Toshima-ku, Tokyo 1708633 (JP)**
- **MIKAMI, Ayako**
**Toshima-ku, Tokyo 1708633 (JP)**

(74) Representative: **HOFFMANN EITLE**
**Patent- und Rechtsanwälte**
**Arabellastrasse 4**
**81925 München (DE)**

(54) **CYANOFLUOROPYRROLIDINE DERIVATIVE**

(57) A cyanofluoropyrrolidine compound of Formula (I) or a pharmaceutically acceptable salt thereof or a hydrate thereof, which is useful as an agent for preventing or treating diseases or conditions capable of being improved by inhibition of dipeptidyl peptidase IV (DPPIV), diabetes mellitus, immune diseases and the like:

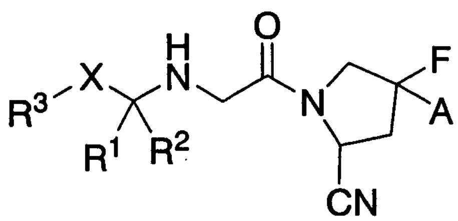

(I)

[wherein
A represents a hydrogen atom or a fluorine atom,
$R^1$ and $R^2$ are as defined in the specification,
X represents a single bond or a $C_{1-3}$ alkylene group, and
$R^3$ represents a group represented by the formula: $-N(R^4)COR^5$, $-N(R^4)SO_2R^5$, $-NR^4R^6$, $-SO_2R^5$, $-SO_2NR^4R^5$, $-OCONR^4R^5$, $-CH{=}CH-R^7$ or $-C{\equiv}C-R^7$, or represents a heteroaryl group selected from a heteroaryl group which contains at least one oxygen and/or sulfur atom and which may further contain a nitrogen atom, and a 6-membered nitrogen-containing aromatic ring or a 9- to 11-membered condensed ring thereof (wherein the heteroaryl group may be substituted with one or more substituents selected from the substituent $Y^3$ group)].

EP 1 627 870 A1

## Description

### TECHNICAL FIELD

The present invention relates to novel cyanofluoropyrrolidine derivatives.

### BACKGROUND ART

Dipeptidyl peptidase IV (DPPIV) is a kind of serine proteases that can hydrolyze a dipeptide from a peptide chain having proline or alanine at the second position from its N-terminal end. DPPIV is distributed in a wide range of tissues (e.g., kidney, liver) and plasma, and is involved in the metabolism of various physiologically active peptides.

Recent studies have indicated that DPPIV acts on the metabolism of glucagon-like peptide-1 (GLP-1). Namely, DPPIV inactivates GLP-1 by hydrolyzing the N-terminal His-Ala dipeptide in GLP-1, and the resulting inactivated product serves as an antagonist of GLP-1 receptor.

GLP-1 has been known to have physiological actions such as an accelerating action on insulin secretion from the pancreas, an prolonging action on gastric emptying time and an inhibitory action on eating. Thus, DPPIV inhibition leads to an increase in GLP-1 action, enhancement of insulin action and improvement of glucose metabolism; DPPIV inhibition is therefore expected to be useful in treating type 2 diabetes mellitus.

Likewise, DPPIV has been known to contribute to the metabolism of neuropeptide Y (a kind of neuropeptides), activation of immunocompetent T cells, cancer cell adhesion to the endothelium, and invasion of HIV virus into lymphocytes. Thus, DPPIV inhibition is believed to be useful in treating immune diseases, etc.

Moreover, a high level of DPPIV expression has been found in fibroblasts of the skin of human subjects with psoriasis, rheumatoid arthritis and lichen planus, and a high DPPIV activity has been found in subjects with benign prostatic hypertrophy. Thus, DPPIV inhibition is also expected to be effective for skin diseases and benign prostatic hypertrophy.

Compounds previously reported as DPPIV inhibitors are cyanopyrrolidine derivatives (International Publication No. WO98/19998) and 4-fluoro-2-cyanopyrrolidine derivatives (International Publication No. WO02/38541), etc.

### DISCLOSURE OF THE INVENTION

An object of the present invention is to provide novel cyanofluoropyrrolidine derivatives having an excellent DPPIV inhibition activity. Another object of the present invention is to provide novel cyanofluoropyrrolidine derivatives having prolonged DPPIV inhibition activity.

As a result of extensive and intensive efforts, the inventors of the present invention have found that cyanofluoropyrrolidine derivatives of Formula (I) achieve the objects stated above, and thereby have completed the present invention.

Namely, according to one embodiment of the present invention, there is provided a cyanofluoropyrrolidine compound of the following Formula (I) or a pharmaceutically acceptable salt thereof or a hydrate thereof (hereinafter referred to as "the compound of the present invention"):

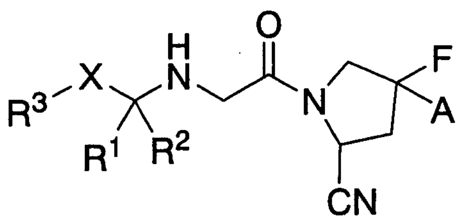

(I)

[wherein

A represents a hydrogen atom or a fluorine atom,

$R^1$ and $R^2$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; a $C_{2-6}$ alkenyl group which may be substituted with one or more substituents selected from the substituent $Y^2$ group; a $C_{3-6}$ cycloalkenyl group which may be substituted with one or more substituents selected from the substituent $Y^2$ group; or a $C_{4-9}$ cycloalke-

nylalkyl group which may be substituted with one or more substituents selected from the substituent $Y^2$ group; or $R^1$ and $R^2$ may form, together with the adjacent carbon atom, a $C_{3-10}$ cycloalkyl group which may be substituted with one or more substituents selected from the substituent $Y^2$ group,

X represents a single bond or a $C_{1-3}$ alkylene group,

$R^3$ represents a group represented by the formula: $-N(R^4)COR^5$, $-N(R^4)SO_2R^5$, $-NR^4R^6$, $-SO_2R^5$, $-SO_2NR^4R^5$, $-OCONR^4R^5$, $-CH=CH-R^7$ or $-C \equiv C-R^7$, or represents a heteroaryl group selected from a heteroaryl group which contains at least one oxygen and/or sulfur atom and which may further contain a nitrogen atom, and a 6-membered nitrogen-containing aromatic ring or a 9- to 11-membered condensed ring thereof (wherein the heteroaryl group may be substituted with one or more substituents selected from the substituent $Y^3$ group)

(wherein $R^4$ and $R^6$, which may be the same or different, each represent a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or an arylalkyl group which may be substituted with one or more substituents selected from the substituent $Y^3$ group,

$R^5$ represents a $C_{1-10}$ alkyl group which may be substituted with one or more substituents selected from the substituent $Y^4$ group, or -($C_{1-3}$ alkylene)-Q or Q, wherein $C_{1-3}$ alkylene may be substituted with one or more substituents selected from a halogen atom and a hydroxyl group, and Q represents an aliphatic or aromatic hydrocarbon selected from a $C_{3-10}$ cycloalkyl group which may be substituted with one or more substituents selected from the substituent $Y^3$ group; a $C_{4-10}$ bridged cyclic alkyl group which may be substituted with one or more substituents selected from the substituent $Y^3$ group; a $C_{2-10}$ alkenyl group which may be substituted with one or more substituents selected from the substituent $Y^3$ group; a $C_{3-10}$ cycloalkenyl group which may be substituted with one or more substituents selected from the substituent $Y^3$ group; a $C_{4-10}$ bridged cyclic alkenyl group which may be substituted with one or more substituents selected from the substituent $Y^3$ group; and an aryl group which may be substituted with one or more substituents selected from the substituent $Y^3$ group; or alternatively, Q represents a heterocyclic ring which may be substituted with one or more substituents selected from the substituent $Y^5$ group, wherein in the aryl group or heterocyclic ring in $R^5$, adjacent substituents attached to the ring member atoms may together form a 5- to 8-membered ring which may contain one or more heteroatoms in its ring,

in $R^4$, $R^5$ or $R^6$, $R^4$ and $R^5$, $R^4$ and $R^6$, as well as $R^5$ and $R^6$ may form, together with the adjacent heteroatom(s), a 4-to 10-membered heterocyclic ring which may be substituted with one or more substituents selected from the substituent $Y^5$ group, and

$R^7$ represents a hydrogen atom; a $C_{1-6}$ alkyl group which may be substituted with one or more substituents selected from the substituent $Y^1$ group; an aryl group which may be substituted with one or more substituents selected from the substituent $Y^3$ group; or a heteroaryl group which may be substituted with one or more substituents selected from the substituent $Y^3$ group),

the substituent $Y^1$ group represents a group consisting of a halogen atom, a hydroxyl group, a carboxyl group, a cyano group, an amino group, an aminocarbonyl group, a $C_{3-5}$ cycloalkyloxy group and a $C_{1-6}$ alkoxy group,

the substituent $Y^2$ group represents a group consisting of a halogen atom, a hydroxyl group, a carboxyl group, a cyano group, an amino group, an aminocarbonyl group, a $C_{3-5}$ cycloalkyloxy group, a $C_{1-6}$ alkoxy group and a $C_{1-6}$ alkyl group,

the substituent $Y^3$ group represents a group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or a phenyl group which may be substituted with one or more substituents selected from the substituent $Y^2$ group), as well as a $C_{1-6}$ alkyl group which may be substituted with one or more substituents selected from the substituent $Y^1$ group and a phenyl group which may be substituted with one or more substituents selected from the substituent $Y^2$ group,

the substituent $Y^4$ group represents a group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or a phenyl group which may be substituted with one or more substituents selected from the substituent $Y^2$ group), as well as a phenyl group which may be substituted with one or more substituents selected from the substituent $Y^2$ group, and

the substituent $Y^5$ group represents a group consisting of an oxo group, a halogen atom, a hydroxyl group, a cyano

group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$,$-N(R^9)SO_2R^{10}$, $-NR^9R^{10}$, $-SO_2R^9$, $-SO_2NR^9R^{10}$, $-SO_2N=CHNR^9R^{10}$ and-$OCONR^9R^{10}$ (wherein $R^9$, $R^{10}$ and $R^{11}$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or a phenyl group which may be substituted with one or more substituents selected from the substituent $Y^2$ group), as well as a $C_{1-6}$ alkyl group which may be substituted with one or more substituents selected from the substituent $Y^1$ group and a phenyl group which may be substituted with one or more substituents selected from the substituent $Y^2$ group].

MODE FOR CARRYING OUT THE INVENTION

According to another embodiment of the present invention, there is provided a compound of Formula (I-2) or a salt thereof or a hydrate thereof:

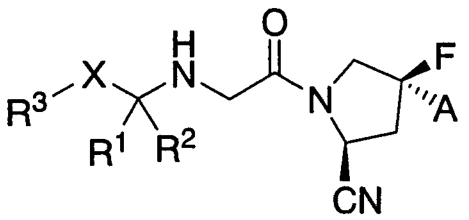

(I-2)

(wherein A, $R^1$, $R^2$, $R^3$ and X are as defined in Formula (I)).

According to another embodiment of the present invention, there is provided a compound of Formula (I) or Formula (I-2) or a salt thereof or a hydrate thereof, wherein $R^1$ and $R^2$, which may be the same or different, each represent a $C_{1-6}$ alkyl group which may be substituted with one or more substituents selected from the substituent $Y^1$ group.

According to another embodiment of the present invention, there is provided a compound of Formula (I) or Formula (I-2) or a salt thereof or a hydrate thereof, wherein $R^1$ and $R^2$ are each a methyl group, an ethyl group or a hydroxymethyl group.

According to another embodiment of the present invention, there is provided a compound of Formula (I) or Formula (I-2) or a salt thereof or a hydrate thereof, wherein

$R^1$ and $R^2$ are as defined in Formula (I), preferably are each a methyl group, an ethyl group or a hydroxymethyl group, and more preferably are each a methyl group or a hydroxymethyl group,

X is a methylene group or an ethylene group,

$R^3$ is a group represented by the formula $-N(R^4)COR^5$,

$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; or a $C_{3-6}$ cycloalkyl group which may be substituted with one or more substituents selected from the substituent $Y^2$ group, and more preferably a hydrogen atom,

$R^5$ is a $C_{1-10}$ alkyl group which may be substituted with one or more substituents selected from the substituent $Y^4$ group, or -($C_{1-3}$ alkylene)-Q or Q, wherein $C_{1-3}$ alkylene may be substituted with one or more substituents selected from a halogen atom and a hydroxyl group, and Q is an aliphatic or aromatic hydrocarbon selected from a $C_{3-10}$ cycloalkyl group which may be substituted with one or more substituents selected from the substituent $Y^3$ group; a $C_{4-10}$ bridged cyclic alkyl group which may be substituted with one or more substituents selected from the substituent $Y^3$ group; a $C_{2-10}$ alkenyl group which may be substituted with one or more substituents selected from the substituent $Y^3$ group; a $C_{3-10}$ cycloalkenyl group which may be substituted with one or more substituents selected from the substituent $Y^3$ group; a $C_{4-10}$ bridged cyclic alkenyl group which may be substituted with one or more substituents selected from the substituent $Y^3$ group; and an aryl group which may be substituted with one or more substituents selected from the substituent $Y^3$ group, in the aryl group in $R^5$, adjacent substituents attached to the ring member atoms may together form a 5- to 8-membered ring which may contain one or more heteroatoms in its ring, and

$R^4$ and $R^5$ may form, together with the adjacent heteroatom(s), a 4- to 10-membered heterocyclic ring which may be substituted with one or more substituents selected from the substituent $Y^5$ group.

According to another embodiment of the present invention, there is provided a compound of Formula (I) or

Formula (I-2) or a salt thereof or a hydrate thereof, wherein
$R^1$ and $R^2$ are as defined in Formula (I), preferably are each a methyl group, an ethyl group or a hydroxymethyl group, and more preferably are each a methyl group or a hydroxymethyl group,
X is a methylene group or an ethylene group,
$R^3$ is a group represented by the formula $-N(R^4)COR^5$,
$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; or a $C_{3-6}$ cycloalkyl group which may be substituted with one or more substituents selected from the substituent $Y^2$ group, and more preferably a hydrogen atom, and
$R^5$ is a $C_{1-6}$ alkyl group which may be substituted with one or more substituents selected from the substituent $Y^1$ group, or a $C_{3-6}$ cycloalkyl group which may be substituted with one or more substituents selected from the substituent $Y^2$ group.

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group, and more preferably are each a methyl group or a hydroxymethyl group,
X is a methylene group or an ethylene group,
$R^3$ is a group represented by the formula $-N(R^4)COR^5$,
$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; or a $C_{3-6}$ cycloalkyl group which may be substituted with one or more substituents selected from the substituent $Y^2$ group, and more preferably a hydrogen atom, and
$R^5$ is an aryl group which may be substituted with one or more substituents selected from the substituent $Y^3$ group, wherein in the aryl group, adjacent substituents attached to the ring member atoms may together form a 5- to 8-membered ring which may contain one or more heteroatoms in its ring.

According to another embodiment of the present invention, there is provided a compound of Formula (I) or Formula (1-2) or a salt thereof or a hydrate thereof, wherein
$R^1$ and $R^2$ are as defined in Formula (I), preferably are each a methyl group, an ethyl group or a hydroxymethyl group, and more preferably are each a methyl group or a hydroxymethyl group,
X is a methylene group or an ethylene group,
$R^3$ is a group represented by the formula $-N(R^4)COR^5$,
$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; or a $C_{3-6}$ cycloalkyl group which may be substituted with one or more substituents selected from the substituent $Y^2$ group, and more preferably a hydrogen atom, and
$R^5$ is a heteroaryl group which may be substituted with one or more substituents selected from the substituent $Y^3$ group.

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group, and more preferably are each a methyl group or a hydroxymethyl group,
X is a methylene group or an ethylene group,
$R^3$ is a group represented by the formula $-N(R^4)COR^5$,
$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group, and more preferably a hydrogen atom, and
$R^5$ is a monocyclic heteroaryl group which may be substituted with one or more substituents selected from the substituent $Y^3$ group.

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group,

and more preferably are each a methyl group or a hydroxymethyl group,
X is a methylene group or an ethylene group,
$R^3$ is a group represented by the formula $-N(R^4)COR^5$,
$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group, and more preferably a hydrogen atom, and
$R^5$ is a thienyl group which may be substituted with one or more substituents selected from the substituent $Y^3$ group.

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group, and more preferably are each a methyl group or a hydroxymethyl group,
X is a methylene group or an ethylene group,
$R^3$ is $-N(R^4)SO_2R^5$,
$R^4$ is a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, $R^4$ is preferably a hydrogen atom; a $C_{1-10}$ alkyl group which may be substituted with one or more substituents selected from the substituent $Y^4$ group; or a $C_{3-6}$ cycloalkyl group which may be substituted with one or more substituents selected from the substituent $Y^2$ group, and more preferably a hydrogen atom,
$R^5$ is a $C_{1-10}$ alkyl group which may be substituted with one or more substituents selected from the substituent $Y^4$ group, or $-(C_{1-3}$ alkylene)-Q or Q, wherein $C_{1-3}$ alkylene may be substituted with one or more substituents selected from a halogen atom and a hydroxyl group, and Q is an aliphatic or aromatic hydrocarbon selected from a $C_{3-10}$ cycloalkyl group which may be substituted with one or more substituents selected from the substituent $Y^3$ group; a $C_{4-10}$ bridged cyclic alkyl group which may be substituted with one or more substituents selected from the substituent $Y^3$ group; a $C_{2-10}$ alkenyl group which may be substituted with one or more substituents selected from the substituent $Y^3$ group; a $C_{3-10}$ cycloalkenyl group which may be substituted with one or more substituents selected from the substituent $Y^3$ group; a $C_{4-10}$ bridged cyclic alkenyl group which may be substituted with one or more substituents selected from the substituent $Y^3$ group; and an aryl group which may be substituted with one or more substituents selected from the substituent $Y^3$ group; or alternatively, Q represents a heterocyclic ring which may be substituted with one or more substituents selected from the substituent $Y^5$ group,
wherein in the aryl group or heterocyclic ring in $R^5$, adjacent substituents attached to the ring member atoms may together form a 5- to 8-membered ring which may contain one or more heteroatoms in its ring, and
$R^4$ and $R^5$ may form, together with the adjacent heteroatom(s), a 4- to 10-membered heterocyclic ring which may be substituted with one or more substituents selected from the substituent $Y^5$ group.

According to another embodiment of the present invention, there is provided a compound of Formula (I) or Formula (1-2) or a salt thereof or a hydrate thereof, wherein
$R^1$ and $R^2$ are as defined in Formula (I), preferably are each a methyl group, an ethyl group or a hydroxymethyl group, and more preferably are each a methyl group or a hydroxymethyl group,
X is a methylene group or an ethylene group, and
$R^3$ is $-NR^4R^6$,
(wherein $R^4$ and $R^6$, which may be the same or different, each represent a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or an arylalkyl group which may be substituted with one or more substituents selected from the substituent $Y^3$ group, or
$R^4$ and $R^6$ may form, together with the adjacent nitrogen atom, a 4- to 10-membered nitrogen-containing ring which may be substituted with one or more substituents selected from the substituent $Y^5$ group,
wherein $R^4$ and $R^6$, which may be the same or different, preferably each represent a hydrogen atom or a $C_{1-10}$ alkyl group which may be substituted with one or more substituents selected from the substituent $Y^4$ group, or may preferably form, together with the adjacent nitrogen atom, a 4- to 10-membered nitrogen-containing ring which may be substituted with one or more substituents selected from the substituent $Y^5$ group, and more preferably each represent a hydrogen atom or a $C_{1-10}$ alkyl group which may be substituted with one or more (preferably 1 to 3) hydroxyl groups).

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group,

X represents a single bond or a methylene group, and
$R^3$ is a group represented by the formula $-CH=CH-R^7$ or $-C\equiv C-R^7$
(wherein $R^7$ represents a hydrogen atom; a $C_{1-6}$ alkyl group which may be substituted with one or more substituents selected from the substituent $Y^1$ group; an aryl group which may be substituted with one or more substituents selected from the substituent $Y^3$ group; or a heteroaryl group which may be substituted with one or more substituents selected from the substituent $Y^3$ group).

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group,
X represents a single bond or a methylene group, and
$R^3$ is a 5- or 6-membered heteroaryl or an 8- to 11-membered condensed ring thereof, which contains at least one oxygen and/or sulfur atom and may further contain a nitrogen atom and which may be substituted with one or more substituents selected from the substituent $Y^3$ group.

According to another embodiment of the present invention, there is provided a compound of Formula (I) or Formula (I-2) or a salt thereof or a hydrate thereof, wherein
$R^1$ and $R^2$ are as defined in Formula (I), preferably are each a methyl group, an ethyl group or a hydroxymethyl group,
X represents a single bond or a methylene group, and
$R^3$ is a 6-membered nitrogen-containing aromatic ring or a 9- to 11-membered condensed ring thereof, which may be substituted with one or more substituents selected from the substituent $Y^3$ group.

According to another embodiment of the present invention, there is provided a pharmaceutical preparation which comprises any one of the above cyanofluoropyrrolidine compounds or a pharmaceutically acceptable salt thereof or a hydrate thereof as an active ingredient.

According to another embodiment of the present invention, there is provided such a pharmaceutical preparation for use in preventing or treating a disease or condition capable of being improved by inhibition of dipeptidyl peptidase IV.

According to another embodiment of the present invention, there is provided such a pharmaceutical preparation, wherein the disease or condition capable of being improved by inhibition of dipeptidyl peptidase IV is diabetes mellitus.

According to another embodiment of the present invention, there is provided such a pharmaceutical preparation, wherein a disease or condition capable of being improved by inhibition of dipeptidyl peptidase IV is an immune disease.

The present invention will be illustrated in detail below, but is not limited to the particular embodiments described herein.

Definitions and illustrative examples of the following selected functional groups, which are used throughout the specification and the claims, are provided for illustrative purposes only and are not intended to be limiting.

The term "optionally substituted $C_{1-6}$ alkyl group" refers to a substituted or unsubstituted linear or branched $C_{1-6}$ alkyl group. Substituents for the $C_{1-6}$ alkyl group refer to one or more (e.g., 1 to 6, preferably 1 to 4, more preferably 1 or 2) members selected from the group consisting of a halogen atom, a hydroxyl group, a carboxyl group, a cyano group, an amino group, an aminocarbonyl group, a $C_{3-5}$ cycloalkyloxy group and a $C_{1-6}$ alkoxy group. Examples of preferred substituents include a halogen atom and a hydroxyl group. Examples of such an alkyl group include a methyl group, an ethyl group, a propyl group, an isopropyl group, a butyl group, an isobutyl group, a sec-butyl group, a tert-butyl group, a pentyl group, an isopentyl group, a 1-ethylpropyl group, a trifluoromethyl group, a 2-chloroethyl group, a hydroxymethyl group, a 2-cyanopropyl group, a 2-aminoethyl group, a 4-carboxybutyl group, and an aminocarbonylmethyl group.

The term "halogen atom" refers to a fluorine atom, a chlorine atom, a bromine atom or an iodine atom.

Examples of a $C_{3-5}$ cycloalkyloxy group include a cyclopropyloxy group, a cyclobutyloxy group, and a cyclopentyloxy group.

The term "$C_{1-6}$ alkoxy group" refers to a linear or branched $C_{1-6}$ alkoxy group. Examples of such an alkoxy group include a methoxy group, an ethoxy group, a propoxy group, an isopropoxy group, a butoxy group, an isobutoxy group, a tert-butoxy group, a pentyloxy group, and an isopentyloxy group.

The term "optionally substituted $C_{3-6}$ cycloalkyl group" refers to a substituted or unsubstituted $C_{3-6}$ cycloalkyl group. Substituents for the $C_{3-6}$ cycloalkyl group refer to one or more (e.g., 1 to 6, preferably 1 to 4, more preferably 1 or 2) members selected from the group consisting of a halogen atom, a hydroxyl group, a carboxyl group, a cyano group, an amino group, an aminocarbonyl group, a $C_{1-6}$ alkyl group, a $C_{3-5}$ cycloalkyloxy group and a $C_{1-6}$ alkoxy group. Examples of such a cycloalkyl group include a cyclopropyl group, a cyclobutyl group, a cyclopentyl group, a cyclohexyl group, a 3-cyanocyclobutyl group, a 2-aminocyclopropyl group, a 4-fluorocyclohexyl group, a 3,4-dihydroxycyclopentyl group, a 2-carboxycyclopropyl group, and a 3-aminocarbonylcyclobutyl group.

The term "optionally substituted $C_{4-9}$ cycloalkylalkyl group" refers to a group composed of an optionally substituted $C_{3-6}$ cycloalkyl group and an optionally substituted $C_{1-3}$ alkylene group attached to each other. Examples of such a cycloalkylalkyl group include a cyclopropylmethyl group, a cyclobutylmethyl group, a 2-cyclopentylethyl group, a cyclohexylmethyl group, a 3-cyanocyclobutylmethyl group, a 1-(2-aminocyclopropyl)ethyl group, a 3-(4-fluorocyclohexyl)propyl group, a 3,4-dihydroxycyclopentylmethyl group, a 2-(2-carboxycyclopropyl)propyl group, and a (3-aminocarbonylcyclobutyl)methyl group.

The term "$C_{1-3}$ alkylene group" refers to a linear or branched $C_{1-3}$ alkylene group. The term "optionally substituted $C_{1-3}$ alkylene group" refers to a substituted or unsubstituted linear or branched $C_{1-3}$ alkylene group. Substituents for the alkylene group refer to one or more groups selected from the group consisting of a halogen atom and a hydroxyl group. Examples of such an alkylene group include a methylene group, an ethylene group, a propylene group, a hydroxymethylene group, and a 2-bromopropylene group.

The term "optionally substituted $C_{2-6}$ alkenyl group" refers to a substituted or unsubstituted linear or branched $C_{2-6}$ alkenyl group. Substituents for the alkenyl group refer to one or more (e.g., 1 to 6, preferably 1 to 4, more preferably 1 or 2) members selected from the group consisting of a halogen atom, a hydroxyl group, a carboxyl group, a cyano group, an amino group, an aminocarbonyl group, a $C_{3-5}$ cycloalkyloxy group, a $C_{1-6}$ alkoxy group and a $C_{1-6}$ alkyl group. Examples of such an alkenyl group include a vinyl group, an allyl group, a 1-propenyl group, an isopropenyl group, a 1-butenyl group, a 2-butenyl group, a 3-butenyl group, an isobutenyl group, a pentenyl group, a 2-chlorovinyl group, a 3-hydroxypropenyl group, a 3-carboxypropenyl group, a 3-amino-2-cyanobutenyl group, and a 3-ethoxyisobutenyl group.

The term "optionally substituted $C_{3-6}$ cycloalkenyl group" refers to a substituted or unsubstituted $C_{3-6}$ cycloalkenyl group. Substituents for the cycloalkenyl group refer to one or more (e.g., 1 to 6, preferably 1 to 4, more preferably 1 or 2) members selected from the group consisting of a halogen atom, a hydroxyl group, a carboxyl group, a cyano group, an amino group, an aminocarbonyl group, a $C_{1-6}$ alkyl group, a $C_{3-5}$ cycloalkyloxy group and a $C_{1-6}$ alkoxy group. Examples of such a cycloalkenyl group include a cyclopropenyl group, a cyclobutenyl group, a cyclopentenyl group, a 3-hydroxycyclopropenyl group, a 3-carboxycyclopropenyl group, a 3-amino-2-cyanocyclobutenyl group, and a 3-ethoxycyclobutenyl group.

The term "optionally substituted $C_{4-9}$ cycloalkenylalkyl group" refers to a group composed of an optionally substituted $C_{3-6}$ cycloalkenyl group and an optionally substituted $C_{1-3}$ alkylene group attached to each other. Examples of such a cycloalkenylalkyl group include a 2-cyanocyclobutenylmethyl group, and a 3-methoxycyclopropenylmethyl group.

The optionally substituted $C_{3-10}$ cycloalkyl group formed by $R^1$ and $R^2$ together with the adjacent carbon atom refers to a substituted or unsubstituted $C_{3-10}$ cycloalkyl group. Substituents for the cycloalkyl group refer to one or more (e.g., 1 to 6, preferably 1 to 4, more preferably 1 or 2) members selected from the group consisting of a halogen atom, a hydroxyl group, a carboxyl group, a cyano group, an amino group, an aminocarbonyl group, a $C_{1-6}$ alkyl group, a $C_{3-5}$ cycloalkyloxy group and a $C_{1-6}$ alkoxy group. Examples of such a cycloalkyl group include a cyclopropyl group, a cyclobutyl group, a cyclopentyl group, a cyclohexyl group, a cycloheptyl group, a cyclooctyl group, a bromocyclopropyl group, a 2-ethyl-3-hydroxycyclohexyl group, a 3-amino-2-cyanocyclobutyl group, and a 4-methoxycyclooctyl group.

The phrase "heteroaryl group which contains at least one oxygen and/or sulfur atom and which may further contain a nitrogen atom" refers to, for example, a 5- or 6-membered heteroaryl or an 8- to 11-membered condensed ring thereof, which contains at least one oxygen and/or sulfur atom and which may further contain a nitrogen atom. Examples include a furyl group, a thienyl group, an oxazolyl group, an isoxazolyl group, a thiazolyl group, an isothiazolyl group, a 1,3,5-oxadiazolyl group, a 1,2,4-oxadiazolyl group, a 1,2,4-thiadiazolyl group, a benzoxazolyl group, a benzisoxazolyl group, a benzothiazolyl group, a benzoisothiazolyl group, a thianaphthenyl group, an isothianaphthenyl group, a benzofuranyl group, an isobenzofuranyl group, a benzothienyl group, a chromenyl group, a 2,1,3-benzoxadiazolyl group, and a benzoxazinyl group. In terms of prolonged DPPIV inhibition activity, preferred are monocyclic groups including a furyl group and a thienyl group. A more preferred example is a furyl group. Substituents for the heteroaryl group which contains at least one oxygen and/or sulfur atom and which may further contain a nitrogen atom refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$,$-N(R^9)SO_2R^{10}$, $-NR^9R^{10}$, $-SO_2R^9$, $-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group or an optionally substituted phenyl group), as well as an optionally substituted $C_{1-6}$ alkyl group and an optionally substituted phenyl group. Examples of a substituted heteroaryl group which contains at least one oxygen and/or sulfur atom and which may further contain a nitrogen atom include a 4-methyl-1,2,3-thiadiazol-5-yl group, a 3-(2-chlorophenyl)-5-methyl-isoxazol-4-yl group, and a 5-methyl-2-trifluoromethylfuran-3-yl group.

The term "optionally substituted phenyl group" refers to a substituted or unsubstituted phenyl group. Substituents for the phenyl group refer to one or more (e.g., 1 to 6, preferably 1 to 4, more preferably 1 or 2) members selected from the group consisting of a halogen atom, a hydroxyl group, a carboxyl group, a cyano group, an amino group, an aminocarbonyl group, a $C_{3-5}$ cycloalkyloxy group, a $C_{1-6}$ alkoxy group and a $C_{1-6}$ alkyl group. Examples of such a phenyl group include a phenyl group, and a 3-aminocarbonyl-4-bromophenyl group.

Examples of a 6-membered nitrogen-containing aromatic ring or a 9- to 11-membered condensed ring thereof include a pyridyl group, a pyrazinyl group, a pyrimidinyl group, a pyridazinyl group, a 1,2,4-triazinyl group, a 1,2,3-triazinyl

group, a 1,3,5-triazinyl group, an isoquinolyl group, a quinolyl group, a phthalazinyl group, a quinoxalinyl group, a quinazolinyl group, and a cinnolinyl group. Preferred are monocyclic groups, and more preferred is a pyridyl group. Substituents for a heteroaryl composed of a 6-membered nitrogen-containing aromatic ring or a 9-to 11-membered condensed ring thereof refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group or an optionally substituted phenyl group), as well as an optionally substituted $C_{1-6}$ alkyl group and an optionally substituted phenyl group. Examples of a substituted 6-membered nitrogen-containing aromatic ring or a substituted 9- to 11-membered condensed ring thereof include a 5-cyanopyridin-2-yl group, and a 6-(aminocarbonyl)quinoxalin-2-yl group.

The term "optionally substituted $C_{1-10}$ alkyl group" refers to a substituted or unsubstituted linear or branched $C_{1-10}$ alkyl group. Substituents for the alkyl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted phenyl group. Examples of such an alkyl group include a methyl group, an ethyl group, a propyl group, an isopropyl group, a butyl group, an isobutyl group, a sec-butyl group, a tert-butyl group, a pentyl group, an isopentyl group, a hexyl group, a heptyl group, an octyl group, a nonyl group, a decyl group, a 2-hydroxyethyl group, an aminocarbonylmethyl group, a cyanomethyl group, a chloroethyl group, a 3-(N,N-dimethylamino)propyl group, a 4-(methanesulfonylamino)butyl group, and a 2-dimethylamide-4-hydroxyheptyl group.

The term "optionally substituted arylalkyl group" refers to a group composed of an optionally substituted aryl group and an optionally substituted $C_{1-3}$ alkylene group attached to each other. Examples of such an arylalkyl group include a benzyl group, a phenethyl group, a 3-phenylpropyl group, a 1-naphthylmethyl group, a 2-(1-naphthyl)ethyl group, a 2-(2-naphthyl)ethyl group, a 3-(2-naphthyl)propyl group, a 4-cyanobenzyl group, and a 2-(3-dimethylaminophenyl)-1-hydroxyethyl group.

The term "optionally substituted $C_{3-10}$ cycloalkyl group" refers to a substituted or unsubstituted $C_{3-10}$ cycloalkyl group. Substituents for the cycloalkyl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted $C_{1-6}$ alkyl group and an optionally substituted phenyl group. Examples of such a cycloalkyl group include a cyclopropyl group, a cyclobutyl group, a cyclopentyl group, a cyclohexyl group, a cycloheptyl group, a cyclooctyl group, a 3-(acetylamino)cyclopentyl group, a 4-(N,N-dimethylaminocarbonyloxy)cyclohexyl group, and a 3-ethylsulfonyl-4-methoxycyclohexyl group.

Examples of $-(C_1-C_3$ alkylene$)-Q$ wherein Q represents an optionally substituted $C_{3-10}$ cycloalkyl group include a cyclopropylmethyl group, a cyclopropylethyl group, a cyclobutylmethyl group, a cyclopentylmethyl group, and a cyclohexylmethyl group.

The term "optionally substituted $C_{4-10}$ bridged cyclic alkyl group" refers to a substituted or unsubstituted $C_{4-10}$ bridged cyclic alkyl group. Substituents for the bridged cyclic alkyl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$,$-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted $C_{1-6}$ alkyl group and an optionally substituted phenyl group. Examples of such a bridged cyclic alkyl group include a bicyclopentyl group, a bicyclohexyl group, a bicycloheptyl group, a bicyclooctyl group, a bicyclononyl group, a bicyclodecyl group, an adamantyl group, a bornyl group, a norbornyl group, a pinanyl group, a thujyl group, a caryl group, a camphanyl group, a 2-hydroxyadamantyl group, and a 3-methylbicyclopentyl group.

The term "optionally substituted $C_{2-10}$ alkenyl group" refers to a substituted or unsubstituted linear or branched $C_{2-10}$ alkenyl group. Substituents for the alkenyl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$,

$-SO_2R^9$, $-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted $C_{1-6}$ alkyl group and an optionally substituted phenyl group. Examples of such an alkenyl group include a vinyl group, an allyl group, a 1-propenyl group, an isopropenyl group, a butenyl group, an isobutenyl group, a pentenyl group, a hexenyl group, a heptenyl group, an octenyl group, and a 4-acetylamino-2-cyanoheptenyl group.

The term "optionally substituted $C_{3-10}$ cycloalkenyl group" refers to a substituted or unsubstituted $C_{3-10}$ cycloalkenyl group. Substituents for the cycloalkenyl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted $C_{1-6}$ alkyl group and an optionally substituted phenyl group. Examples of such a cycloalkenyl group include a cyclobutenyl group, a cyclopentenyl group, a cyclohexenyl group, a cycloheptenyl group, a cyclooctenyl group, and a 3-(N,N-dimethylureido)cyclohexenyl group.

Examples of $-(C_1-C_3$ alkylene)-Q wherein Q represents an optionally substituted $C_{3-10}$ cycloalkenyl group include a cyclobutenylmethyl group, a cyclopentenylmethyl group, and a cyclohexenylmethyl group.

The term "optionally substituted $C_{4-10}$ bridged cyclic alkenyl group" refers to a substituted or unsubstituted $C_{4-10}$ bridged cyclic alkenyl group. Substituents for the bridged cyclic alkenyl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$,$-N(R^9)CONR^{10}R^{11}$,- $N(R^9)SO_2R^{10}$, $-NR^9R^{10}$, $-SO_2R^9$, $-SO_2NR^9R^{10}$,$-SO_2N{=}CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted $C_{1-6}$ alkyl group and an optionally substituted phenyl group. Examples of such a bridged cyclic alkenyl group include a bicyclopentenyl group, a bicyclohexenyl group, a bicycloheptenyl group, a bicyclooctenyl group, a bicyclononenyl group, a bicyclodecenyl group, a 2-cyanobicyclooctenyl group, and a 2-chlorobicyclononenyl group.

The term "optionally substituted aryl group" refers to a substituted or unsubstituted aryl group. Substituents for the aryl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$,$-N(R^9)SO_2R^{10}$, $-NR^9R^{10}$, $-SO_2R^9$, $-SO_2NR^9R^{10}$, $-SO_2N{=}CHNR^9R^{10}$ and$-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group or an optionally substituted phenyl group), as well as an optionally substituted $C_{1-6}$ alkyl group and an optionally substituted phenyl group. Examples of such an aryl group include a phenyl group, a naphthyl group, a 3,4-methylenedioxyphenyl group, a 3-(methylsulfonyl)phenyl group, a 2-trifluoromethylphenyl group, a 3-cyanophenyl group, a 2-fluorophenyl group, a 2-ethoxynaphthyl group, a 2-dimethylaminophenyl group, a 3-butylsulfonylaminonaphthyl group, a 2-carboxyphenyl group, a 3,4-dimethoxyphenyl group, and a 4-[(N,N-dimethylaminomethylene)aminosulfonyl]phenyl group.

Examples of $-(C_1-C_3$ alkylene)-Q wherein Q represents an optionally substituted aryl group include a benzyl group, a phenethyl group, a 3-phenylpropyl group, a 1-naphthylmethyl group, a 2-(1-naphthyl)ethyl group, a 2-(2-naphthyl)ethyl group, a 3-(2-naphthyl)propyl group, a 4-cyanobenzyl group, and a 2-(3-dimethylaminophenyl)-1-hydroxyethyl group.

The term "optionally substituted 4- to 10-membered heterocyclic ring" refers to an aromatic or non-aromatic (saturated or unsaturated) monocyclic or polycyclic heterocyclic ring, which contains one or more heteroatoms selected from O, S and N and which has 4 to 10 ring member atoms, unless otherwise specified. The aromatic heterocyclic ring is also referred to herein as a heteroaryl and will be described later as a heteroaryl. The above heterocyclic ring may be C-bonded or N-bonded, if possible.

In terms of prolonged DPPIV inhibition activity, the heterocyclic ring of the present invention is preferably a monocyclic heterocyclic ring.

Substituents for the heterocyclic ring refer to one or more (e.g., 1 to 6, preferably 1 to 4, more preferably 1 or 2) members selected from the group consisting of an oxo group, a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$,$-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted

$C_{3-6}$ cycloalkyl group; or an optionally substituted $C_{4-9}$ cycloalkylalkyl group or an optionally substituted phenyl group), as well as an optionally substituted $C_{1-6}$ alkyl group and an optionally substituted phenyl group. Examples of such a non-aromatic heterocyclic ring include an aziridinyl group, an azetidinyl group, a pyrrolidinyl group, an imidazolidinyl group, an oxazolidinyl group, a thiazolidinyl group, a piperidinyl group, a piperazinyl group, a morpholinyl group, an azabicycloheptyl group, an azabicyclooctyl group, a 2,6-dimethylmorpholino group, a 4-cyanopiperidinyl group, a diketopiperazinyl group, a 2-oxopiperidinyl group, a 1,1-dioxo-isothiazolidinyl group, a 1,1-dioxo-thiazinanyl group, and a 1,1-dioxo-thiazepanyl group.

The term "optionally substituted heteroaryl group" refers to a substituted or unsubstituted heteroaryl group. Substituents for the heteroaryl group refer to one or more (e.g., 1 to 6, preferably 1 to 4, more preferably 1 or 2) members selected from the group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$,$-N(R^9)CONR^{10}R^{11}$, $-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; or an optionally substituted $C_{4-9}$ cycloalkylalkyl group or an optionally substituted phenyl group), as well as an optionally substituted $C_{1-6}$ alkyl group and an optionally substituted phenyl group. Examples of such a heteroaryl group include a pyrrolyl group, a furyl group, a thienyl group, an oxazolyl group, an isoxazolyl group, an imidazolyl group, a thiazolyl group, an isothiazolyl group, a pyrazolyl group, a triazolyl group, a tetrazolyl group, a 1,3,5-oxadiazolyl group, a 1,2,4-oxadiazolyl group, a 1,2,4-thiadiazolyl group, a pyridyl group, a pyrazinyl group, a pyrimidinyl group, a pyridazinyl group, a 1,2,4-triazinyl group, a 1,2,3-triazinyl group, a 1,3,5-triazinyl group, a benzoxazolyl group, a benzisoxazolyl group, a benzothiazolyl group, a benzoisothiazolyl group, a benzoimidazolyl group, a thianaphthenyl group, an isothianaphthenyl group, a benzofuranyl group, an isobenzofuranyl group, a benzothienyl group, a chromenyl group, an isoindolyl group, an indolyl group, an indazolyl group, an isoquinolyl group, a quinolyl group, a phthalazinyl group, a quinoxalinyl group, a quinazolinyl group, a cinnolinyl group, a 2,1,3-benzoxadiazolyl group, a benzoxazinyl group, a 4-methyl-1,2,3-thiadiazol-5-yl group, a 3-(2-chlorophenyl)-5-methyl-isoxazol-4-yl group, a 5-methyl-2-phenyl-1,2,3-triazol-4-yl group, a 2-phenyl-3-propyl-pyrazol-4-yl group, a 5-methyl-2-trifluoromethylfuran-3-yl group, a 5-cyanopyridin-2-yl group, and a 6-(aminocarbonyl)quinoxalin-2-yl group.

Examples of $-(C_1-C_3$ alkylene)-Q wherein Q represents an optionally substituted heteroaryl group include a 2-furylmethyl group, and a 3-isoxazolylmethyl group.

In a case where in the aryl group or heterocyclic ring found in $R^5$, adjacent substituents attached to the ring member atoms together form a 5- to 8-membered ring which may contain one or more heteroatoms in its ring, it is preferred that the adjacent substituents together form an alkyleneoxy group or an alkylenedioxy group, which in turn forms such a 5- to 8-membered ring together with the adjacent ring member atoms constituting the aryl group or the heterocyclic ring. Examples include 3,4-methylenedioxyphenyl, 3,4-ethylenedioxyphenyl, and 2,3-dihydrobenzo[b]furan-5-yl.

The "optionally substituted 4- to 10-membered nitrogen-containing ring formed together with the adjacent nitrogen atom" found in $R^4$ and $R^6$ is intended to mean a substituted or unsubstituted cyclic amino group which has one or more nitrogen atoms in its ring and which may further contain one or more oxygen and/or sulfur atoms. Examples include cyclic amino groups such as an aziridinyl group, an azetidinyl group, a pyrrolidinyl group, an imidazolidinyl group, an oxazolidinyl group, a thiazolidinyl group, a piperidinyl group, a piperazinyl group, a morpholinyl group, an azabicycloheptyl group, and an azabicyclooctyl group. Substituents for the above 4- to 10-membered nitrogen-containing ring refer to a group consisting of an oxo group, a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$,$-N(R^9)SO_2R^{10}$, $-NR^9R^{10}$, $-SO_2R^9$, $-SO_2NR^9R^{10}$, $-SO_2N=CHNR^9R^{10}$ and $-OCONR^9R^{10}$ (wherein $R^9$, $R^{10}$ and $R^{11}$, which may be the same or different, each represent a hydrogen atom; an optionally substituted $C_{1-6}$ alkyl group; an optionally substituted $C_{3-6}$ cycloalkyl group; an optionally substituted $C_{4-9}$ cycloalkylalkyl group; or an optionally substituted phenyl group), as well as an optionally substituted $C_{1-6}$ alkyl group and an optionally substituted phenyl group.

The term "pharmaceutically acceptable salt" refers to a salt with a mineral or organic acid. Examples include an acetate salt, a propionate salt, a butyrate salt, a formate salt, a trifluoroacetate salt, a maleate salt, a tartrate salt, a citrate salt, a stearate salt, a succinate salt, an ethylsuccinate salt, a lactobionate salt, a gluconate salt, a glucoheptate salt, a benzoate salt, a methanesulfonate salt, an ethanesulfonate salt, a 2-hydroxyethanesulfonate salt, a benzenesulfonate salt, a paratoluenesulfonate salt, a lauryl sulfate salt, a malate salt, an aspartate salt, a glutamate salt, an adipate salt, a salt with cysteine, a salt with N-acetylcysteine, a hydrochloride salt, a hydrobromide salt, a phosphate salt, a sulfate salt, a hydroiodide salt, a nicotinate salt, an oxalate salt, a picrate salt, a thiocyanate salt, an undecanoate salt, a salt with an acrylate polymer and a salt with a carboxyvinyl polymer.

Since certain compounds according to the present invention have an asymmetric center, they are present in various enantiomer forms. All the optical isomers and stereoisomers of the compounds of the present invention as well as mixtures thereof fall within the scope of the present invention. The present invention encompasses a racemate, one or more enantiomers, one or more diastereomers or mixtures thereof. Some of the compounds of the present invention

also exist, e.g., as keto-enol tautomers. The present invention encompasses all of these tautomers and mixtures thereof.

Preferred embodiments of the compound of the present invention will now be described below.

In a compound wherein $R^3$ is $-N(R^4)COR^5$ (wherein $R^5$ is an optionally substituted aryl group), a preferred embodiment of $R^5$ is a substituted or unsubstituted phenyl group and a preferred substituent for the phenyl group is one or more (e.g., 1 to 6, preferably 1 to 4, more preferably 1 or 2) members selected from the group consisting of a nitro group, a cyano group, a hydroxyl group, a halogen (preferably fluorine, chlorine)-substituted $C_{1-6}$ alkyl group (e.g., $-CF_3$, $-CCl_3$), $-COR^9$,$-CO_2R^9$,- $SO_2R^9$, $-SO_2NR^9R^{10}$, $-SO_2N=CHNR^9R^{10}$ (wherein $R^9$ and $R^{10}$, which may be the same or different, each represent a hydrogen atom, a $C_{1-6}$ alkyl group or a phenyl group), a $C_{1-6}$ alkyl group, a halogen atom and a $C_{1-6}$ alkoxy group.

Likewise, another example of a preferred substituent for the phenyl group as $R^5$ is a 5- to 8-membered ring formed as follows: substituents attached to the adjacent atoms constituting the phenyl group together form a $C_{2-3}$ alkyleneoxy group (e.g., an ethyleneoxy group, a propyleneoxy group) or a $C_{1-3}$ alkylenedioxy group (e.g., a methylenedioxy group, an ethylenedioxy group), which in turn forms the 5- to 8-membered ring together with the adjacent atoms constituting the phenyl group. Examples include 3,4-methylenedioxyphenyl, 3,4-ethylenedioxyphenyl, and 2,3-dihydrobenzo[b]furan-5-yl.

In terms of prolonged DPPIV inhibition activity, $R^5$ is preferably a substituted or unsubstituted phenyl group and a preferred substituent for the phenyl group is one or more (e.g., 1 to 6, preferably 1 to 4, more preferably 1 or 2) members selected from the group consisting of a nitro group, a cyano group, a hydroxyl group, a halogen (preferably fluorine, chlorine)-substituted $C_{1-6}$ alkyl group (e.g., $-CF_3$, $-CCl_3$), $-COR^9$, $-CO_2R^9$, $-SO_2R^9$ (wherein $R^9$ is a hydrogen atom, a $C_{1-6}$ alkyl group or a phenyl group), a $C_{1-6}$ alkyl group and a halogen atom, or alternatively, a 5- to 8-membered ring formed as follows: substituents attached to the adjacent atoms constituting the phenyl group together form a $C_{2-3}$ alkyleneoxy group or a $C_{1-3}$ alkylenedioxy group, which in turn forms the 5- to 8-membered ring together with the adjacent atoms constituting the phenyl group.

Specific examples of preferred compounds for the above embodiment include:

(2S,4S)-2-cyano-4-fluoro-1-[[2-(3,4-methylenedioxybenzoyl)amino-1,1-dimethyl]ethylamino]acetylpyrrolidine;
(2S,4S)-2-cyano-4-fluoro-1-[[2-[3-(methylsulfonyl)-benzoyl]amino-1,1-dimethyl]ethylamino]acetylpyrrolidine;
(2S,4S)-2-cyano-1-[[2-(3-cyanobenzoyl)amino-1,1-dimethyl]ethylamino]acetyl-4-fluoropyrrolidine;
(2S,4S)-2-cyano-4-fluoro-1-[[2-(2-fluorobenzoyl)-amino-1,1-dimethyl]ethylamino]acetylpyrrolidine;
(2S,4S)-1-[(2-benzoylamino-1,1-dimethyl)ethylamino]-acetyl-2-cyano-4-fluoropyrrolidine; and
(2S,4S)-2-cyano-1-[[2-(2,3-dihydrobenzo[b]furan-5-yl)carbonylamino-1,1-dimethyl]ethylamino]acetyl-4-fluoropyrrolidine.

In a compound wherein $R^3$ is $-N(R^4)COR^5$ (wherein $R^5$ is an optionally substituted heteroaryl group), a preferred embodiment of $R^5$ is a substituted or unsubstituted monocyclic heteroaryl group, more preferably a thienyl group, in terms of prolonged DPPIV inhibition activity.

A preferred substituent for the heteroaryl group as $R^5$ may be one or more (e.g., 1 to 6, preferably 1 to 4, more preferably 1 or 2) members selected from the group consisting of a nitro group, a cyano group, a hydroxyl group, a halogen (preferably fluorine, chlorine)-substituted $C_{1-6}$ alkyl group (e.g., $-CF_3$, $-CCl_3$), $-COR^9$,$-CO_2R^9$,- $SO_2R^9$, $-SO_2NR^9R^{10}$, $-SO_2N=CHNR^9R^{10}$ (wherein $R^9$ and $R^{10}$, which may be the same or different, each represent a hydrogen atom, a $C_{1-6}$ alkyl group or a phenyl group), a $C_{1-6}$ alkyl group, a halogen atom, a $C_{1-6}$ alkoxy group and an optionally substituted phenyl group. More preferred are one or more (e.g., 1 to 6, preferably 1 to 4, more preferably 1 or 2) members selected from the group consisting of a nitro group, a cyano group, a hydroxyl group, a halogen (preferably fluorine, chlorine)-substituted $C_{1-6}$ alkyl group (e.g., $-CF_3$, $-CCl_3$), $-COR^9$,$-CO_2R^9$, $-SO_2R^9$ (wherein $R^9$ and $R^{10}$, which may be the same or different, each represent a hydrogen atom, a $C_{1-6}$ alkyl group or a phenyl group), a $C_{1-6}$ alkyl group and a halogen atom. Even more preferred is a $C_{1-6}$ alkyl group which may be substituted with one or more (e.g., 1 to 6, preferably 1 to 4, more preferably 1 or 2) hydroxyl groups.

Specific examples of preferred compounds for the above embodiment include:

(2S,4S)-2-cyano-4-fluoro-1-[[2-(4-methyl-1,2,3-thiadiazol-5-yl)carbonylamino-1,1-dimethyl]ethylamino]-acetylpyrrolidine;
(2S,4S)-2-cyano-4-fluoro-l-[[2-(2-pyridyl)carbonylamino-1,1-dimethyl]ethylaminolacetylpyrrolidine;
(2S,4S)-2-cyano-4-fluoro-1-[[2-(furan-2-yl)carbonylamino-1,1-dimethyl]ethylamino]acetylpyrrolidine;
(2S,4S)-2-cyano-1-[[2-(3,5-dimethylisoxazol-4-yl)-carbonylamino-1,1-dimethyl]ethylaminolacetyl-4-fluoropyrrolidine;
(2S,4S)-2-cyano-4-fluoro-1-[[2-(thiophen-2-yl)-carbonylamino-1,1-dimethyl]ethylamino]acetyl-pyrrolidine;
(2S,4S)-1-[[2-(1H-1,2,3-benzotriazol-5-yl)carbonylamino-1,1-dimethyl]ethylamino]acetyl-2-cyano-4-fluoropyrrolidine;

(2S,4S)-2-cyano-4-fluoro-1-[[2-(thiophen-3-yl)-carbonylamino-1,1-dimethyl]ethylamino]acetylpyrrolidine;
(2S,4S)-2-cyano-4-fluoro-1-[[2-(5-methylthiophen-2-yl)carbonylamino-1,1-dimethyl]ethylamino]acetylpyrrolidine; and
(2S,4S)-2-cyano-4-fluoro-1-[[2-(3-methylthiophen-2-yl)carbonylamino-1,1-dimethyl]ethylamino]acetylpyrrolidine.

In a compound wherein $R^3$ is $-N(R^4)COR^5$ (wherein $R^5$ is an optionally substituted $C_{1-6}$ alkyl group or an optionally substituted $C_{3-6}$ cycloalkyl group), a preferred embodiment of $R^5$ may be a $C_{1-6}$ alkyl or $C_{3-6}$ cycloalkyl group which may be substituted with one or more (e.g., 1 to 6, preferably 1 to 4, more preferably 1 or 2) hydroxyl groups.
Specific examples of preferred compounds for the above embodiment include:

(2S,4S)-2-cyano-4-fluoro-1-[(2-pivaloylamino-1,1-dimethyl)ethylamino]acetylpyrrolidine;
(2S,4S)-2-cyano-4-fluoro-1-[[2-(3-hydroxy-2-methylpropan-2-yl)carbonylamino-1,1-dimethyl]ethylamino]acetylpyrrolidine;
(2S,4S)-2-cyano-1-[(2-cyclopropanecarbonylamino-1,1-dimethyl)ethylamino]acetyl-4-fluoropyrrolidine; and
(2S,4S)-1-[[2-(1-methylcyclopropan-1-yl)carbonylamino-1,1-dimethyl]ethylamino]acetyl-2-cyano-4-fluoropyrrolidine.

In a compound wherein $R^3$ is a heteroaryl group which contains at least one oxygen and/or sulfur atom and which may further contain a nitrogen atom (wherein the heteroaryl group may be substituted), the heteroaryl is preferably monocyclic, more preferably a thienyl group or a furanyl group, in terms of prolonged DPPIV inhibition activity. A preferred substituent for the heteroaryl group as $R^3$ may be a $C_{1-6}$ alkyl or $C_{3-6}$ cycloalkyl group which may be substituted with one or more (e.g., 1 to 6, preferably 1 to 4, more preferably 1 or 2) hydroxyl groups.
Specific examples of preferred compounds for the above embodiment include:

(2S,4S)-2-cyano-4-fluoro-1-[[1-(furan-2-yl)-1-methyl]ethylamino]acetylpyrrolidine.

In a compound wherein $R^3$ is $-N(R^4)SO_2R^5$, a preferred embodiment of $R^5$ is the same as already mentioned for the cases where $R^3$ is $-N(R^4)COR^5$ (wherein $R^5$ is an optionally substituted aryl group, an optionally substituted heteroaryl group, and an optionally substituted $C_{1-6}$ alkyl group or an optionally substituted $C_{3-6}$ cycloalkyl group, respectively).
The compound of the present invention can inhibit dipeptidyl peptidase IV, thus enhancing insulin action and improving glucose metabolism. The compound of the present invention can also contribute to inhibition of neuropeptide Y metabolism, inhibition of T cell activation, inhibition of cancer cell adhesion to the endothelium, and prevention of invasion of HIV virus into lymphocytes.
Accordingly, the present invention provides such a pharmaceutical preparation for preventing or treating diseases or conditions capable of being improved by inhibition of dipeptidyl peptidase IV, as exemplified by diabetes mellitus (especially type 2), immune diseases, arthritis, obesity, osteoporosis, conditions of impaired glucose tolerance, benign prostatic hypertrophy and skin diseases.
Examples of pharmaceutical preparations for immune diseases include immunosuppressive agents for use in tissue transplantation, for example, cytokine release inhibitors for various autoimmune diseases such as inflammatory enteritis, multiple sclerosis and chronic rheumatoid arthritis (RA), as well as agents useful for preventing or treating AIDS by preventing invasion of HIV into T-cells and agents for preventing metastasis, especially metastasis of breast and prostate tumors to the lung.
The pharmaceutical preparation of the present invention can be administered systemically or topically via oral route or parenteral (e.g., intrarectal, subcutaneous, intramuscular, intravenous, percutaneous) route.
For use as a pharmaceutical preparation, the compound of the present invention may be formulated into any desired dosage form selected from solid compositions, liquid compositions and other compositions, as appropriate for the intended purpose. The pharmaceutical preparation of the present invention can be prepared by blending the compound of the present invention with pharmaceutically acceptable carrier(s). More specifically, the compound of the present invention may be supplemented with commonly used excipients, extenders, binders, disintegrating agents, coating agents, sugar-coating agents, pH regulators, solubilizers, aqueous or non-aqueous solvents and so on, and then formulated using standard techniques into tablets, pills, capsules, granules, powders, solutions, emulsions, suspensions, injections, etc. Examples of excipients and extenders include, for example, lactose, magnesium stearate, starch, talc, gelatin, agar, pectin, gum arabic, olive oil, sesame oil, cacao butter, ethylene glycol and other commonly used materials.
Also, the compound of the present invention may be modified to form an inclusion compound with, e.g., $\alpha$-, $\beta$- or $\gamma$-cyclodextrin or methylated cyclodextrin before being formulated.
The dose of the compound of the present invention will vary depending on the disease or symptom to be treated,

body weight, age, sex, the route of administration, etc. The adult dose is preferably about 1 to about 1000 mg/person/day, preferably about 5 to about 500 mg/person/day, and more preferably about 10 to about 200 mg/person/day, given as a single dose or in divided doses per day.

How to prepare the compound of the present invention will be explained in more detail below, but is not limited to the particularly embodiments illustrated herein. Any solvent may be used in reactions as long as it does not inhibit each reaction. The reaction solvents used are not limited in any way by the following description.

A compound of Formula (I) can be prepared by the general procedures shown below.

[General procedures for preparation]

[Scheme 1]

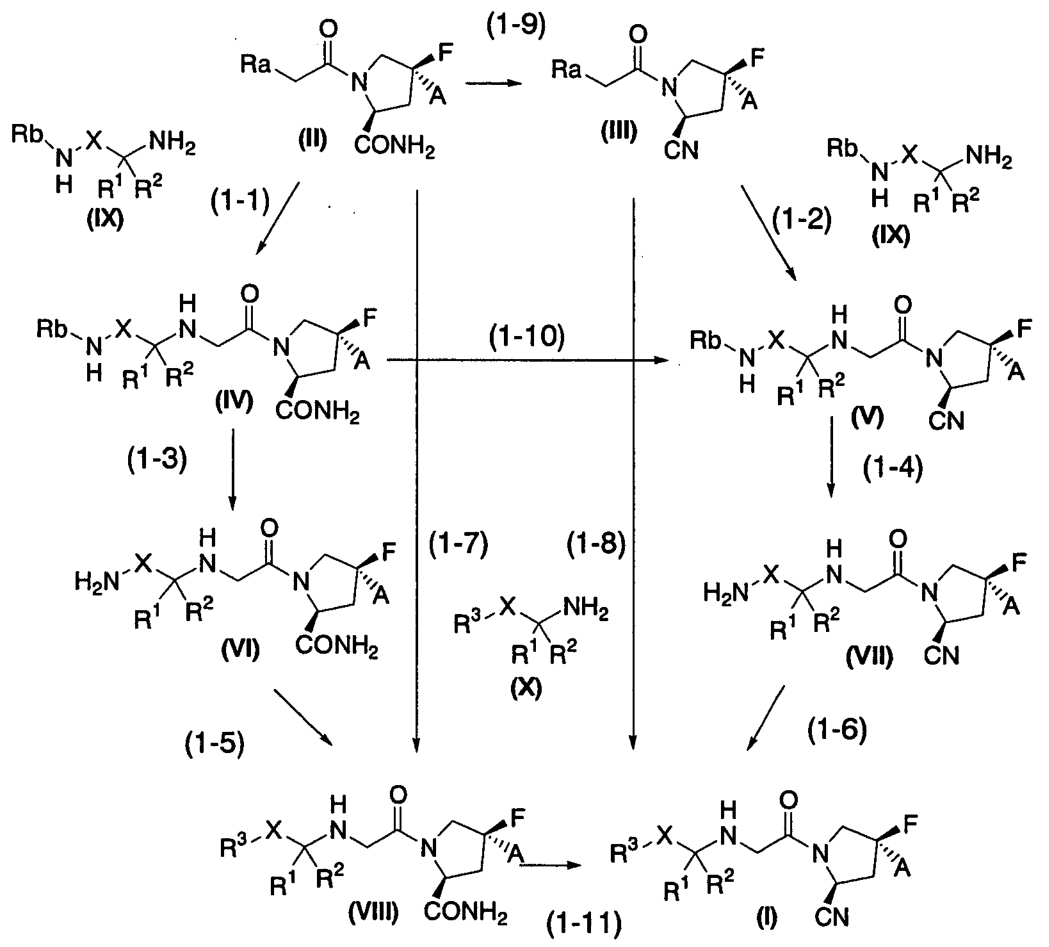

(wherein A, $R^1$, $R^2$, $R^3$ and X are as defined above, Ra represents a leaving group such as a halogen atom or a sulfonyloxy group, and Rb represents a protecting group for an amino group. With respect to Compounds (II) and (III) used as starting materials, procedures for their production can be found in WO0238541. Preparation of Compounds (IX) and (X) will be described later.)

Step (1-1), (1-2), (1-7) or (1-8): In these steps, Compound (II) or (III) having the leaving group Ra is reacted with Compound (IX) or (X) (which is a primary amine derivative) to obtain Compound (IV), (V), (VIII) or (I) (which is a secondary amine derivative). Substitution reaction may be accomplished by using a compound having, as Ra, a leaving group such as a chlorine atom, a bromine atom, an iodine atom, a methanesulfonyloxy group or a p-toluenesulfonyloxy group, along with primary amines. In this case, these amines are used either alone in excessive amounts or in combination

with another base. Examples of a base to be added include amines such as triethylamine and diisopropylethylamine or inorganic bases such as potassium carbonate. Alternatively, in some cases, sodium iodide or the like may be added in order to accelerate the reaction. Examples of a solvent available for use in this reaction include N,N-dimethylformamide, tetrahydrofuran, dioxane, dichloromethane, and chloroform. The reaction may be performed at 0°C to 100°C.

Steps (1-3) and (1-4): In these steps, the protecting group on the amino group is removed. This deprotection may be accomplished by using the procedures described in PROTECTIVE GROUPS IN ORGANIC SYNTHESIS, written by THEODORA W. GREENE and PETER G. M. WU TS.

For example, Compound (IV) or (V) wherein Rb is a group capable of being removed by the action of an acid (e.g., a tert-butoxycarbonyl group, a trityl group, o-nitrobenzenesulfenyl group) may be deprotected using an acid such as hydrochloric acid, sulfuric acid, trifluoroacetic acid, p-toluenesulfonic acid or methanesulfonic acid to synthesize Compound (VI) or (VII) having a primary amino group. In this case, the deprotection may be accomplished by diluting or dissolving the acid with an organic solvent or water and the reaction may be performed at -50°C to 50°C. Examples of an organic solvent include ethanol, methanol, tetrahydrofuran, N,N-dimethylformamide, dichloromethane, chloroform, and 1,2-dichloroethane. Moreover, for example, a compound wherein Rb is a group capable of being removed by hydrogenolysis (e.g., a benzyloxycarbonyl group) may be deprotected by hydrogenolysis using a metal catalyst (e.g., palladium), by reaction using a hydrogen gas, or by reaction using a reagent combination such as formic acid-ammonium formate. Examples of a solvent available for use in this reaction include ethanol, methanol, tetrahydrofuran, and ethyl acetate. The reaction may be performed at 0°C to 100°C. Moreover, for example, a compound wherein Rb is a group capable of being removed by the action of a base (e.g., a fluorenyloxycarbonyl group) may be deprotected using a base such as diethylamine, piperidine, ammonia, sodium hydroxide or potassium carbonate. These bases may be used alone or by diluting, dissolving or suspending in a solvent. Examples of a solvent available for use in this reaction include water, ethanol, methanol, tetrahydrofuran, N,N-dimethylformamide, dichloromethane, chloroform, and 1,2-dichloroethane. The reaction may be performed at 0°C to 100°C. Moreover, for example, a compound wherein Rb is a group capable of being removed by the action of a metal catalyst (e.g., an allyloxycarbonyl group) may be deprotected using tetrakis(triphenylphosphine)palladium or the like as a catalyst or reagent. Examples of a solvent available for use in this reaction include dichloromethane, chloroform, and tetrahydrofuran. The reaction may be performed at 0°C to 100°C.

Steps (1-5) and (1-6): In these steps, Compound (VI) or (VII) having a primary amino group or Compound (VIII) or (I) wherein $R^3$ represents the formula $-NHR^4$ is converted into Compound (VIII) or (I) wherein $R^3$ represents the formula $-N(R^4)COR^5$, $-N(R^4)SO_2R^5$ or $-NR^4R^6$. The reaction to be used will vary depending on the compound to be synthesized. Examples will be given below. These compounds may be converted either in a single step or through a combination of multiple steps.

(Procedure for acylation of amino group): In this step, Compound (VI) or (VII) having a primary amino group or Compound (VIII) or (I) wherein $R^3$ represents the formula $-NHR^4$ is used and converted into Compound (VIII) or (I) wherein $R^3$ represents the formula $-N(R^4)COR^5$.

An example of amidation is a reaction using an acyl halide such as acyl chloride or acyl bromide. Examples of a solvent available for use in this reaction include dichloromethane, chloroform, 1,2-dichloroethane, tetrahydrofuran, dioxane, toluene, and ethyl acetate. The reaction may be performed at -50°C to 100°C. In this case, the reaction may be performed using an appropriate base; and examples of a base include amines (e.g., triethylamine, diisopropylethylamine), organic acid salts (e.g., sodium 2-ethylhexanoate, potassium 2-ethylhexanoate) or inorganic bases (e.g., sodium hydroxide, potassium carbonate).

Another example of amidation is a reaction using an active ester such as 1-benzotriazolyl ester or succinimidyl ester. Examples of a solvent available for use in this reaction include dichloromethane, chloroform, 1,2-dichloroethane, N,N-dimethylformamide, tetrahydrofuran, dioxane, toluene, and ethyl acetate. The reaction may be performed at -50°C to 50°C.

Alternatively, amidation may be accomplished, e.g., by using a carboxylic acid and a dehydration condensing agent. Examples of a dehydration condensing agent include 1-ethyl-3-(3-dimethylaminopropyl)carbodiimide hydrochloride, dicyclohexylcarbodiimide, diphenylphosphorylazide, and carbonyldiimidazole. If necessary, it is possible to use an activating agent such as 1-hydroxybenzotriazole or hydroxysuccinimide. Examples of a solvent available for use in this reaction include dichloromethane, chloroform, 1,2-dichloroethane, N,N-dimethylformamide, tetrahydrofuran, dioxane, toluene, and ethyl acetate. The reaction may be performed at -50°C to 50°C. In this case, the reaction may be performed using an appropriate base; and examples of a base include amines (e.g., triethylamine, diisopropylethylamine), organic acid salts (e.g., sodium 2-ethylhexanoate, potassium 2-ethylhexanoate) or inorganic bases (e.g., sodium hydroxide, potassium carbonate). Alternatively, amidation may be accomplished, e.g., by using a mixed acid anhydride obtained from a carboxylic acid and a chlorocarbonate ester, etc. Examples of a solvent available for use in these reactions include tetrahydrofuran, dioxane, dichloromethane, chloroform, N,N-dimethylformamide, toluene, and ethyl acetate. The reactions may be performed at -50°C to 50°C. In this case, the reactions may be performed using an appropriate base; and examples of a base include amines (e.g., triethylamine, diisopropylethylamine), organic acid salts (e.g., sodium 2-ethylhexanoate, potassium 2-ethylhexanoate) or inorganic bases (e.g., sodium hydroxide, potassium carbonate).

An example of aminocarbonylation of an amino group is a process using an aminocarbonyl halide such as morpholine-4-carbonyl chloride to effect aminocarbonylation of an amino group. Examples of a solvent available for use in such a reaction include dichloromethane, chloroform, 1,2-dichloroethane, tetrahydrofuran, dioxane, toluene, and ethyl acetate. The reaction may be performed at -50°C to 100°C. In this case, the reaction may be performed using an appropriate base; and examples of a base include amines (e.g., triethylamine, diisopropylethylamine), organic acid salts (e.g., sodium 2-ethylhexanoate, potassium 2-ethylhexanoate) or inorganic bases (e.g., potassium carbonate).

(Procedure for sulfonylation of amino group): In this step, Compound (VI) or (VII) having a primary amino group or Compound (VIII) or (I) wherein $R^3$ represents the formula $-NHR^4$ is used and converted into Compound (VIII) or (I) wherein $R^3$ is $-N(R^4)SO_2R^5$. For example, when using sulfonyl chloride and an amine starting material, a sulfonamide form can be obtained. Examples of a solvent available for use in this reaction include dichloromethane, chloroform, 1,2-dichloroethane, tetrahydrofuran, dioxane, toluene, and ethyl acetate. The reaction may be performed at -50°C to 100°C. In this case, the reaction may be performed using an appropriate base; and examples of a base include amines (e.g., triethylamine, diisopropylethylamine), organic acid salts (e.g., sodium 2-ethylhexanoate, potassium 2-ethylhexanoate) or inorganic bases (e.g., potassium carbonate).

(Procedure for alkylation of amino group): In this step, for example, Compound (VI) or (VII) having a primary amino group or Compound (VIII) or (I) wherein $R^3$ represents the formula $-NHR^4$ is used and converted into Compound (VIII) or (I) wherein $R^3$ represents the formula $-NR^4R^6$.

For example, except for using different starting materials, the same procedure as explained in Step (1-1), (1-2), (1-7) or (1-8) may be repeated to synthesize an N-alkyl derivative.

Alternatively, for example, reductive amination may be employed to effect N-alkylation. This is a process using an amino derivative and an aldehyde or ketone derivative to perform the reaction under conditions using an appropriate reduction method. Examples of the reduction method to be used include those using a reducing agent (e.g., sodium borohydride, sodium cyanoborohydride, sodium triacetoxyborohydride), and those using hydrogenation in the presence of palladium or the like. Examples of a solvent available for use in this reaction include ethanol, methanol, tetrahydrofuran, dioxane, and water. The reaction may be performed at -20°C to 100°C.

(Procedure for N-alkylation of amide form): In this step, Compound (VIII) or (I) wherein $R^3$ represents the formula $-NHCOR^5$ is used and converted into Compound (VIII) or (I) wherein $R^3$ represents the formula $-N(R^4)COR^5$.

For example, the compound may be treated with an alkylating reagent such as an alkyl halide to obtain the product of interest. In this case, the reaction is performed in the presence of an appropriate base; and examples of a base include sodium hydride, potassium tert-butoxide, n-butyllithium, and lithium diisopropylamide. Examples of a solvent available for use in this reaction include N,N-dimethylformamide, tetrahydrofuran, and dioxane. The reaction may be performed at -50°C to 50°C.

(Procedure for N-alkylation of sulfonamide form): In this step, Compound (VIII) or (I) wherein $R^3$ represents the formula $-NHSO_2R^5$ is used and converted into Compound (VIII) or (I) wherein $R^3$ represents the formula $-N(R^4)SO_2R^5$.

For example, the compound may be treated with an alkylating reagent such as an alkyl halide to obtain the product of interest. In this case, the reaction is performed in the presence of an appropriate base; and examples of a base include sodium hydride, potassium tert-butoxide, n-butyllithium, and lithium diisopropylamide. Examples of a solvent available for use in this reaction include N,N-dimethylformamide, tetrahydrofuran, and dioxane. The reaction may be performed at -50°C to 50°C.

Alternatively, the Mitsunobu reaction may be used to synthesize the product of interest. In this case, for example, an alcohol form, diethyl azodicarboxylate and triphenylphosphine are used for the reaction. Examples of a solvent available for use in this reaction include N,N-dimethylformamide, tetrahydrofuran, and dioxane. The reaction may be performed at -50°C to 50°C.

Steps (1-9), (1-10) and (1-11): In these steps, the carbamoyl group located at the 2-position of the pyrrolidine ring is converted into a nitrile group, e.g., using trifluoroacetic anhydride. Examples of a solvent available for use in this reaction include dichloromethane, chloroform, 1,2-dichloroethane, tetrahydrofuran, dioxane, and N,N-dimethylformamide. The reaction may be performed at -50°C to 50°C. In this case, a base such as triethylamine, diisopropylethylamine, sodium bicarbonate or potassium carbonate may be added, if necessary. Another example is a process using phosphorus oxychloride. Examples of a solvent available for use in this reaction include dichloromethane, chloroform, 1,2-dichloroethane, tetrahydrofuran, dioxane, and pyridine, which may be used either alone or in combination. The reaction may be performed at -50°C to 50°C. This reaction may also be performed in the presence of imidazole, etc.

Another example is a process using cyanuric chloride and N,N-dimethylformamide. Examples of a solvent available for use in this reaction include dichloromethane, chloroform, 1,2-dichloroethane, tetrahydrofuran, dioxane, and pyridine, which may be used either alone or in combination. The reaction may be performed at -50°C to 50°C.

<u>Compounds (IX) and (X)</u>

[Scheme 2]

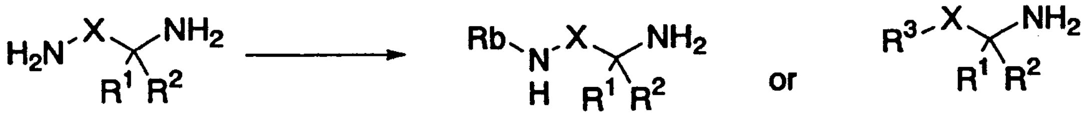

or

(XI) (IX) (X)

(wherein $R^1$, $R^2$, Rb and X are as defined above, $R^3$ represents the formula $-N(R^4)COR^5$, $-N(R^4)SO_2R^5$ or $-NR^4R^6$ among those listed above, and $R^4$, $R^5$ and $R^6$ are as defined above)

This step starts with a diamine form (XI) to obtain an amine form (IX) having a protecting group Rb or an amine form (X) in which $-NH_2$ is converted into a functional group.

For example, the step of obtaining Compound (IX), i.e., a process for introducing a protecting group onto an amino group may be accomplished by using the procedures described in PROTECTIVE GROUPS IN ORGANIC SYNTHESIS, written by THEODORA W. GREENE and PETER G. M. WU TS. For example, in a case where Rb is a tert-butoxycarbonyl group, a benzyloxycarbonyl group, a fluorenylcarbonyl group, a trityl group, an o-nitrobenzenesulfenyl group or the like, such a protecting group may be introduced using, e.g., di-tert-butyl dicarbonate, benzyloxycarbonyl chloride, fluorenylcarbonyl chloride, trityl chloride or o-nitrobenzenesulfenyl chloride in a single or mixed solvent such as dichloromethane, chloroform, 1,2-dichloroethane, tetrahydrofuran, dioxane, toluene, ethyl acetate and/or water. The reaction may be performed at -50°C to 100°C. In this case, the reaction may be performed using an appropriate base; and examples of a base include amines (e.g., triethylamine, diisopropylethylamine), organic acid salts (e.g., sodium 2-ethylhexanoate, potassium 2-ethylhexanoate) or inorganic bases (e.g., sodium hydroxide, potassium carbonate).

For example, in the case of Compound (X) wherein $R^3$ represents the formula $-N(R^4)COR^5$, $-N(R^4)SO_2R^5$ or $-NR^4R^6$, this compound can be prepared by combining the procedures described in Step (1-5) or (1-6) of Scheme 1.

Compound (X)

[Scheme 3]

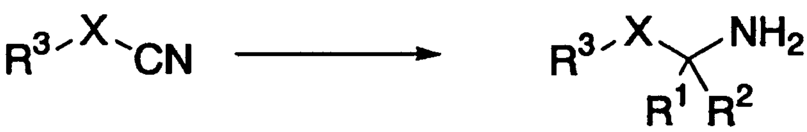

(XII) (X)

{wherein $R^1$, $R^2$ and X are as defined above, $R^3$ represents the formula $-CH=CH-R^7$ or $-C\equiv C-R^7$ or an optionally substituted heteroaryl group among those listed above, and $R^7$ is as defined above}

This step starts with a cyano form (XII) to obtain an amine form (X).

For example, in a case where $R^1$ and $R^2$ are each a methyl group, the amine form (X) can be obtained from the cyano form (XII) using, e.g., methylmagnesium bromide or methyllithium. In this case, anhydrous cerium chloride or the like may be added. Examples of a solvent available for use in this reaction include dichloromethane, chloroform, 1,2-dichloroethane, tetrahydrofuran, dioxane, toluene, and diethyl ether. The reaction may be performed at -78°C to 100°C.

EXAMPLES

The present invention will be further described in more detail by way of the following examples and reference example, which are not intended to limit the scope of the invention.

Reference Example 1

Synthesis of (2S,4S)-1-chloroacetyl-2-cyano-4-fluoropyrrolidine

(2S,4S)-2-Aminocarbonyl-4-fluoropyrrolidine hydrochloride (43.0 g) synthesized as described in W00238541 was suspended in N,N-dimethylformamide (255 mL) and cooled with ice-salt. Chloroacetyl chloride (22.3 mL) was added in one portion and, after 10 minutes, triethylamine (74.7 mL) was added dropwise over 1 hour while maintaining the internal temperature at -7°C to -2°C. Stirring was continued for an additional 1 hour while maintaining the internal temperature at -7°C to +2°C. Cyanuric chloride (28.2 g) in powder form was added over 5 minutes and the reaction mixture was gradually warmed. After 50 minutes, the solidified reaction mixture was poured into a mixture of water (1000 mL) and ice (500 g). The precipitated crystal was collected by filtration, washed with water (400 mL) and dried to give the titled compound (41.94 g) as a colorless powder.
MS(ESI pos.)m/z: 213 ($[M+Na]^+$).
HRMS(ESI pos.): calcd for $C_7H_8ClFN_2ONa[N+Na]^+$ 213.0207, found 213.0201.
$^1$H-NMR (300 MHz, DMSO-d6) δ 5.50 (1H, d, J=51.8 Hz), 5.02-4.96 (1H, m), 4.51 & 4.39 (2H, ABq., J=14.2 Hz), 3.97 (1H, dd like, J=23.6, 12.4 Hz), 3.76 (1H, ddd, J=39.3, 12.4, 3.4 Hz), 2.6-2.3 (2H, m).

Example 1

Synthesis of (2S,4S)-2-cyano-4-fluoro-1-[[2-(3,4-methylenedioxybenzoyl)amino-1,1-dimethyl]ethylamino]acetylpyrrolidine

(1) Synthesis of [2-(3,4-methylenedioxybenzoyl)amino-1,1-dimethyl]ethylamine

1,2-Diamino-2-methylpropane (298 mg) was dissolved in dichloromethane (3.4 mL), followed by addition of 3,4-methylenedioxybenzoyl chloride (312 mg) in small portions under ice cooling. The resulting mixture was stirred under ice cooling for 10 minutes and then at room temperature for 10 minutes. The reaction mixture was concentrated under reduced pressure and the residue was diluted with diethyl ether (15 mL), followed by dropwise addition of 6 M aqueous hydrochloric acid (10 mL) under ice cooling. After the reaction mixture was partitioned, the aqueous phase was further washed with ether (15 mL). The aqueous phase was cooled with ice and 5 M aqueous sodium hydroxide (12 mL) was added thereto, followed by extraction with chloroform (20 mL). The extracted solution was dried over anhydrous magnesium sulfate, filtered to remove the desiccant and then concentrated under reduced pressure to give the titled compound (349 mg) as a colorless oil.
MS(ESI pos.)m/z: 237 ($[M+H]^+$), 259 ($[M+Na]^+$), (ESI neg.)m/z: 235 ($[M-1]^-$).
$^1$H-NMR (300 MHz, $CDCl_3$) δ 7.34 (1H, dd, J=8.0, 1.7 Hz), 7.31 (1H, d, J=1.7 Hz), 6.83 (1H, brd, J=7.9 Hz), 6.66 (1H, brs), 6.02 (2H, s), 3.30 (2H, d, J=5.8 Hz), 1.32 (2H, brs), 1.17 (6H, s).

(2) Synthesis of (2S,4S)-2-cyano-4-fluoro-1-[[2-(3,4-methylenedioxybenzoyl)amino-1,1-dimethyl]ethylamino]acetylpyrrolidine

[2-(3,4-Methylenedioxybenzoyl)amino-1,1-dimethyl]ethylamine (242 mg) was dissolved in methanol (7.3 mL). To this solution, (2S,4S)-1-chloroacetyl-2-cyano-4-fluoropyrrolidine (98 mg) and potassium iodide (127 mg) were added at room temperature and stirred for 3 days. The reaction mixture was concentrated under reduced pressure and the resulting residue was purified by silica gel column chromatography (developing solvent; chloroform:methanol:28% aqueous ammonia = 30:1:0.1) to give the titled compound (174 mg) as a colorless amorphous substance.
MS(ESI pos.)m/z: 391 ($[M+H]^+$), 413 ($[M+Na]^+$), (ESI neg.)m/z: 389 ($[M-1]^-$).
HRMS(ESI pos.): calcd for $C_{19}H_{24}FN_4O_4$ $[M+H]^+$ 391.1782, found 391.1764.
$^1$H-NMR (300 MHz, DMSO-d6) δ 8.08 (1H, m), 7.44 (1H, dd, J=8.1, 1.7 Hz), 7.39 (1H, d, J=1.7 Hz), 6.97 (1H, brd, J=8.1 Hz), 6.09 (2H, s), 5.49 (1H, brd, J=53.2 Hz), 5.00-4.93 (1H, m), 3.96 (1H, dd, J=23.8, 12.6 Hz), 3.74 (1H, ddd, J=39.3, 12.8, 3.6 Hz), 3.52-3.26 (2H, m), 3.24-3.16 (2H, m), 2.62-2.26 (2H, m), 1.98 (1H, brs), 1.01 (3H, s), 1.00 (3H, s).

Example 2

Synthesis of (2S,4S)-2-cyano-4-fluoro-l-[[2-[3-(methylsulfonyl)benzoyl]amino-1,1-dimethyl]ethylamino]acetylpyrrolidine

(1) Synthesis of [2-[3-(methylsulfonyl)benzoyl]amino-1,1-dimethyl]ethylamine

3-(Methylsulfonyl)benzoic acid (296 mg) was suspended in acetone (3.0 mL), followed by addition of triethylamine (206 μL) under ice cooling. After addition of cyanuric chloride (139 mg), acetone (3.0 mL) was further added and stirring was continued at room temperature for 3 hours. The reaction mixture was concentrated under reduced pressure and the residue was diluted with chloroform (5.0 mL). After addition of 1,2-diamino-2-methylpropane (296 mg) and stirring for 10 minutes under ice cooling, the reaction mixture was warmed to room temperature and stirred for 20 minutes. The reaction mixture was concentrated under reduced pressure and the resulting residue was diluted with diethyl ether (20 mL). After addition of 6 M aqueous hydrochloric acid (8.0 mL) and stirring under ice cooling, the organic phase was separated. Under ice cooling, 5 M aqueous sodium hydroxide (8.0 mL) was added to the aqueous phase, which was then extracted three times with chloroform (20 mL). The extracted solution was dried over anhydrous magnesium sulfate, filtered to remove the desiccant and then concentrated under reduced pressure. The resulting residue was purified by silica gel column chromatography (developing solvent; chloroform:methanol:28% aqueous ammonia = 15:1:0.1) to give the titled compound (225 mg) as a colorless oil.
MS(ESI pos.)m/z: 271 ($[M+H]^+$), 293 ($[M+Na]^+$), 541 ($[2M+H]^+$), (ESI neg.) m/z: 269 ($[M-1]^-$).
$^1$H-NMR (300 MHz, $CDCl_3$) δ 8.35 (1H, brs), 8.13 (1H, d, J=7.8 Hz), 8.08 (1H, d, J=7.8 Hz), 7.68 (1H, t, J=7.8 Hz), 6.95 (1H, brs), 3.35 (2H, d, J=5.6 Hz), 3.10 (3H, s), 1.35 (2H, brs), 1.20 (6H, s).

(2) Synthesis of (2S,4S)-2-cyano-4-fluoro-1-[[2-[3-(methylsulfonyl)benzoyl]amino-1,1-dimethyl]ethylamino]acetylpyrrolidine

The same procedure as shown in Example 1(2) was repeated using [2-[3-(methylsulfonyl)benzoyl]amino-1,1-dimethyl]ethylamine (210 mg) and (2S,4S)-1-chloroacetyl-2-cyano-4-fluoropyrrolidine (67 mg) to give the titled compound (113 mg) as a colorless amorphous substance.
MS(ESI pos.)m/z: 425 ($[M+H]^+$), 447 ($[M+Na]^+$), (ESI neg.)m/z: 423 ($[M-1]^-$).
HRMS(ESI pos.): calcd for $C_{19}H_{26}FN_4O_4S$ $[M+H]^+$ 425.1659, found 425.1646.
$^1$H-NMR (300 MHz, DMSO-d6) δ 8.60 (1H, m), 8.36 (1H, brs), 8.19 (1H, d, J=6.8 Hz), 8.08 (1H, brd, J=7.5 Hz), 7.76 (1H, brt, J=7.9 Hz), 5.49 (1H, brd, J=53.2 Hz), 5.00-4.92 (1H, m), 3.97 (1H, dd, J=23.9, 12.4 Hz), 3.87-3.18 (8H, m), 2.62-2.26 (2H, m), 1.96 (1H, brs), 1.04 (6H, s).

Example 3

Synthesis of (2S,4S)-2-cyano-4-fluoro-1-[[2-(2-trifluoromethylbenzoyl)amino-1,1-dimethyl]ethylamino]acetylpyrrolidine

(1) Synthesis of (2-amino-2-methyl-propyl)-carbamic acid tert-butyl ester

1,2-Diamino-2-methylpropane (2.42 mg) was dissolved in tetrahydrofuran (50 mL) and cooled with ice. While stirring, a solution of di-tert-butyl dicarbonate (3.00 g) in tetrahydrofuran (10 mL) was added dropwise over 5 minutes. After the reaction mixture was stirred under ice cooling for 30 minutes, the insoluble materials were filtered off. The filtrate was concentrated under reduced pressure and the residue was partitioned by addition of diethyl ether (50 mL) and 0.4 M hydrochloric acid (50 mL). 5 M aqueous sodium hydroxide (5 mL) was added to the aqueous phase, which was then extracted twice with chloroform (30 mL). The extracted solution was dried over anhydrous sodium sulfate, filtered to remove the desiccant and then concentrated under reduced pressure to give the titled compound (2.44 g) as a colorless solid.
MS(ESI pos.)m/z: 189 ($[M+H]^+$), 211 ($[M+Na]^+$).
$^1$H-NMR (300 MHz, DMSO-d6) δ 6.70 (1H, brt, J=6.1 Hz), 2.80 (2H, d, J=6.1 Hz), 1.38 (9H, s), 1.30 (2H, brs), 0.92 (6H, s).

(2) Synthesis of (2S,4S)-1-[[2-(tert-butoxycarbonyl)amino-1,1-dimethyl]ethylamino]acetyl-2-cyano-4-fluoropyrrolidine

(2S,4S)-1-Chloroacetyl-2-cyano-4-fluoropyrrolidine (0.95 g) and (2-amino-2-methyl-propyl)-carbamic acid tert-butyl ester (1.88 g) were dissolved in methanol (20 mL). To this solution, potassium iodide (0.83 g) was added and stirred overnight at room temperature. After stirring at 50°C for an additional 2 hours, the solvent was distilled off under reduced pressure. The residue was purified by silica gel column chromatography (developing solvent; chloroform:methanol:25% aqueous ammonia = 100:2:0.2). Since (2-amino-2-methyl-propyl)-carbamic acid tert-butyl ester could not be

removed, the residue obtained by distilling off the solvent from the eluted fractions was dissolved in tetrahydrofuran (30 mL). To this solution, di-tert-butyl dicarbonate (1.09 g), 4-dimethylaminopyridine (12 mg) and 0.5 M aqueous sodium hydroxide (10 mL) were added and stirred at room temperature for 2 hours. The reaction mixture was partitioned by addition of ethyl acetate (50 mL) and saturated aqueous sodium chloride (50 mL). The extracted solution was dried over anhydrous sodium sulfate, filtered to remove the desiccant and then concentrated under reduced pressure. The residue was purified by silica gel column chromatography (developing solvent; chloroform:methanol:25% aqueous ammonia = 100:2:0.2) to give the titled compound (1.08 g) as a light-yellow amorphous substance.
MS(ESI pos.)m/z: 343 ($[M+H]^+$), 365 ($[M+Na]^+$), (ESI neg.)m/z: 341 ($[M-H]^-$).
HRMS(ESI pos.): calcd for $C_{16}H_{28}FN_4O_3[M+H]^+$ 343.2145, found 343.2134.
$^1$H-NMR (300 MHz, DMSO-d6) δ 6.64 (1H, brt, J=6.1 Hz), 5.48 (1H, brd, J=53.5 Hz), 4.97-4.91 (1H, m), 3.91 (1H, dd, J=24.6, 12.5 Hz), 3.71 (1H, ddd, J=39.6, 12.5, 3.5 Hz), 3.38 and 3.23 (2H, ABq, J=16.5 Hz), 2.87 (2H, d, J=6.1 Hz), 2.60-2.25 (2H, m), 1.76 (1H, brs), 1.38 (9H, s), 0.94 (6H, s).

(3) Synthesis of (2S,4S)-1-[(2-amino-1,1-dimethyl)ethylamino]acetyl-2-cyano-4-fluoropyrrolidine dihydrochloride

(2S,4S)-1-[[2-(tert-Butoxycarbonyl)amino-1,1-dimethyl]ethylamino]acetyl-2-cyano-4-fluoropyrrolidine (100 mg) was dissolved in ethyl acetate (0.5 mL). To this solution, 4 M hydrochloric acid in ethyl acetate (0.5 mL) was added and stirred at room temperature for 4 hours. The precipitated crystal was collected by filtration and dried to give the titled compound (88 mg) as a colorless powder.
MS(ESI pos.)m/z: 243 ($[M+H]^+$), (ESI neg.)m/z: 277 ($[M+Cl]^-$).
HRMS(ESI pos.): calcd for $C_{11}H_{20}FN_4O$ $[M+H]^+$ 243.1621, found 243.1639.
$^1$H-NMR (300 MHz, DMSO-d6) δ 8.61 (3H, brs), 5.57 (1H, brd, J=50.7 Hz), 5.11-5.04 (1H, m), 4.32-3.72 (4H, m), 3.21 (2H, s), 2.58-2.33 (2H, m), 1.43 (6H, s).

(4) Synthesis of (2S,4S)-2-cyano-4-fluoro-1-[[2-(2-trifluoromethylbenzoyl)amino-1,1-dimethyl]ethylamino]acetylpyrrolidine

(2S,4S)-1-[(2-Amino-1,1-dimethyl)ethylamino]acetyl-2-cyano-4-fluoropyrrolidine dihydrochloride (84 mg) was dissolved in dimethylformamide (0.5 mL). To this solution, triethylamine (111 μL) was added dropwise under ice cooling. After addition of 2-(trifluoromethyl)benzoyl chloride (50 mg) to the suspension and stirring for 10 minutes under ice cooling, the reaction mixture was warmed to room temperature, stirred overnight and then cooled again with ice. After a 1:1 mixture (20 mL) of 10% aqueous sodium bicarbonate and saturated aqueous sodium chloride was added and stirred, the reaction mixture was extracted with ethyl acetate (20 mL). The extracted solution was washed twice with saturated aqueous sodium chloride (20 mL), dried over anhydrous magnesium sulfate, filtered to remove the desiccant and then concentrated under reduced pressure to give the titled compound (68 mg) as a colorless amorphous substance.
MS(ESI pos.)m/z: 415 ($[M+H]^+$), 437 ($[M+Na]^+$), (ESI neg.)m/z: 413 ($[M-1]^-$).
HRMS(ESI pos.): calcd for $C_{19}H_{23}F_4N_4O_2$ $[M+H]^+$415.1757, found 415.1753.
$^1$H-NMR (300 MHz, DMSO-d6) δ 8.36 (1H, m), 7.76 (1H, t, J=7.6 Hz), 7.73 (1H, t, J=7.3 Hz), 7.65 (1H, d, J=7.6 Hz), 7.65 (1H, d, J=7.5 Hz), 5.49 (1H, brd, J=53.2 Hz), 5.00-4.92 (1H, m), 3.94 (1H, dd, J=23.7, 12.2 Hz), 3.72 (1H, ddd, J=39.6, 12.6, 3.4 Hz), 3.52-3.26 (2H, m), 3.25-3.18 (2H, m), 2.62-2.26 (2H, m), 1.05 (6H, s).

Example 4

Synthesis of (2S,4S)-2-cyano-4-fluoro-1-[[2-(3-pyridyl)carbonylamino-1,1-dimethyl]ethylamino]acetylpyrrolidine

(2S,4S)-1-[(2-Amino-1,1-dimethyl)ethylamino]acetyl-2-cyano-4-fluoropyrrolidine dihydrochloride (100 mg) was dissolved in dimethylformamide (0.5 mL). To this solution, triethylamine (177 μL) was added dropwise under ice cooling. After addition of nicotinoyl chloride hydrochloride (51 mg) to the suspension under ice cooling, dimethylformamide (0.5 mL) was further added and stirred for 10 minutes. The reaction mixture was then warmed to room temperature and stirred overnight. After a 1:1 mixture (20 mL) of 10% aqueous sodium bicarbonate and saturated aqueous sodium chloride was added and stirred under ice cooling, the reaction mixture was extracted three times with chloroform (25 mL). The extracted solution was dried over anhydrous magnesium sulfate, filtered to remove the desiccant and then concentrated under reduced pressure. The resulting residue was purified by silica gel column chromatography (developing solvent; chloroform:methanol:28% aqueous ammonia = 20:1:0.1) to give the titled compound (23 mg) as a yellow oil.
MS(ESI pos.)m/z: 348 ($[M+H]^+$), 370 ($[M+Na]^+$), (ESI neg.)m/z: 346 ($[M-1]^-$).
HRMS(ESI pos.): calcd for $C_{17}H_{23}FN_5O_2$ $[M+H]^+$ 348.1836, found 348.1831.
$^1$H-NMR (300 MHz, DMSO-d6) δ 9.00 (1H, d, J=1.6 Hz), 8.70 (1H, dd, J=4.7, 1.6 Hz), 8.46 (1H, m), 8.18 (1H, dt, J=7.9, 2.0 Hz), 7.51 (1H, dd, J=7.9, 4.8 Hz), 5.50 (1H, brd, J=52.7 Hz), 5.00-4.92 (1H, m), 3.96 (1H, dd, J=23.8, 12.3 Hz), 3.74

(1H, ddd, J=39.6, 12.5, 3.5 Hz), 3.48 and 3.30 (2H, ABq, J=16.6 Hz), 3.28-3.22 (2H, m), 2.60-2.25 (2H, m), 1.06-1.03 (6H, m).

Example 5

Synthesis of (2S,4S)-1-[(2-benzenesulfonylamino-1,1-dimethyl)ethylamino]acetyl-2-cyano-4-fluoropyrrolidine

(2S,4S)-1-[(2-Amino-1,1-dimethyl)ethylamino]acetyl-2-cyano-4-fluoropyrrolidine dihydrochloride (95 mg) was dissolved in N,N-dimethylformamide (1 mL) and cooled with ice. To this solution, triethylamine (0.13 mL) and a solution of benzenesulfonyl chloride (48 mg) in N,N-dimethylformamide (0.2 mL) were sequentially added and stirred under ice cooling for 30 minutes. The reaction mixture was partitioned by addition of ethyl acetate (30 mL), 10% aqueous sodium bicarbonate (10 mL) and saturated aqueous sodium chloride (20 mL). The extracted solution was dried over anhydrous sodium sulfate, filtered to remove the desiccant and then concentrated under reduced pressure to give the titled compound (90 mg) as a colorless amorphous substance.
MS(ESI pos.)m/z: 383 ($[M+H]^+$), 405 ($[M+Na]^+$), (ESI neg.)m/z: 381 ($[M-H]^-$).
HRMS(ESI pos.): calcd for $C_{17}H_{24}FN_4O_3S$ $[M+H]^+$ 383.1553, found 383.1551.
$^1$H-NMR (300 MHz, DMSO-d6) δ 7.82 (2H, dd, J=8.1, 1.9 Hz), 7.67-7.55 (4H, m), 5.49 (1H, brd, J=53.0 Hz), 4.98-4.92 (1H, m), 3.87 (1H, dd, J=24.3, 12.1 Hz), 3.66 (1H, ddd, J=39.4, 12.5, 3.4 Hz), 3.33 and 3.18 (2H, ABq, J=16.5 Hz), 2.63 (2H, s), 2.53-2.30 (2H, m), 0.96 (6H, s).

Example 6

Synthesis of (2S,4S)-1-[[2-(N-benzenesulfonyl-N-methyl)amino-1,1-dimethyl]ethylamino]acetyl-2-cyano-4-fluoropyrrolidine

(2S,4S)-1-[(2-Benzenesulfonylamino-1,1-dimethyl)ethylamino]acetyl-2-cyano-4-fluoropyrrolidine (57 mg) and triphenylphosphine (59 mg) were dissolved in tetrahydrofuran (3 mL). To this solution, methanol (0.009 mL) and diethyl azodicarboxylate (98 mg as a 40% toluene solution) were added at room temperature. The resulting mixture was stirred overnight at room temperature and the solvent was distilled off under reduced pressure. The residue was purified by silica gel column chromatography (developing solvent; chloroform:methanol:25% aqueous ammonia = 100:3:0.3 to 100:5:0.5) to give the titled compound (25 mg) as a colorless amorphous substance.
MS(ESI pos.)m/z: 397 ($[M+H]^+$), 419 ($[M+Na]^+$),(ESI neg.)m/z: 395 ($[M-H]^-$).
HRMS(ESI pos.): calcd for $C_{18}H_{26}FN_4O_3S$ $[M+H]^+$ 397.1710, found 397.1718.
$^1$H-NMR (300 MHz, DMSO-d6) δ 7.79 (2H, brd, J=8.4 Hz), 7.72-7.60 (3H, m), 5.49 (1H, brd, J=52.7 Hz), 4.98-4.92 (1H, m), 3.94 (1H, dd, J=24.1, 12.0 Hz), 3.70 (1H, ddd, J=39.5, 12.5, 3.2 Hz), 3.46 and 3.30 (2H, ABq, J=17.4 Hz), 2.92 (2H, s), 2.78 (3H, s), 2.60-2.26 (2H, m), 1.07 (6H, s).

Example 7

Synthesis of (2S,4S)-2-cyano-1-[[2-(4-cyanobenzyl)amino-1,1-dimethyl]ethylamino]acetyl-4-fluoropyrrolidine

(2S,4S)-1-[(2-Amino-1,1-dimethyl)ethylamino]acetyl-2-cyano-4-fluoropyrrolidine dihydrochloride (315 mg) and 4-cyanobenzaldehyde (131 mg) were suspended in chloroform (5 mL) and stirred at room temperature for 30 minutes. After addition of sodium triacetoxyborohydride (424 mg), stirring was continued at room temperature for an additional 30 minutes. The reaction mixture was partitioned by addition of chloroform (50 mL), 10% aqueous sodium bicarbonate (20 mL) and saturated aqueous sodium chloride (20 mL). The extracted solution was dried over anhydrous sodium sulfate, filtered to remove the desiccant and then concentrated under reduced pressure. The resulting residue was purified by silica gel column chromatography (developing solvent; chloroform:methanol:25% aqueous ammonia = 100:3:0.3 to 100:5:0.5) to give the titled compound (217 mg) as a colorless gum.
MS(ESI pos.)m/z: 358 ($[M+H]^+$), 380 ($[M+Na]^+$), (ESI neg.)m/z: 356 ($[M-H]^-$).
HRMS(ESI pos.): calcd for $C_{19}H_{25}FN_5O$ $[M+H]^+$ 358.2043, found 358.2044.
$^1$H-NMR (300 MHz, DMSO-d6) δ 7.77 (2H, d, J=8.4 Hz), 7.54 (2H, d, J=8.2 Hz), 5.49 (1H, brd, J=52.8 Hz), 4.99-4.93 (1H, m), 3.89 (1H, dd, J=24.1, 11.6 Hz), 3.68 (1H, ddd, J=39.6, 12.4, 3.3 Hz), 3.32 and 3.17 (2H, ABq, J=16.3 Hz), 2.60-2.25 (4H, m), 0.98 (6H, s).

Example 8

Synthesis of (2S,4S)-1-[[2-[N-benzoyl-N-(4-cyanobenzyl)]amino-1,1-dimethyl]ethylamino]acetyl-2-cyano-4-fluoropyrrolidine

(2S,4S)-2-Cyano-1-[[2-(4-cyanobenzyl)amino-1,1-dimethyl]ethylamino]acetyl-4-fluoropyrrolidine (62 mg) was dissolved in chloroform (1.0 mL). To this solution, triethylamine (24 μL) and a solution of benzoyl chloride (62 mg) in chloroform (200 μL) were sequentially added dropwise under ice cooling and stirred at room temperature for 2 hours. After a 1:1 mixture (20 mL) of 5% aqueous sodium bicarbonate and saturated aqueous sodium chloride was added and stirred under ice cooling, the reaction mixture was extracted with ethyl acetate (25 mL). The extracted solution was washed sequentially with water (20 mL) and saturated aqueous sodium chloride (20 mL), dried over anhydrous magnesium sulfate, filtered to remove the desiccant and then concentrated under reduced pressure. The resulting residue was purified by silica gel column chromatography (developing solvent; chloroform:methanol:28% aqueous ammonia = 30:1:0.1) to give the titled compound (61 mg) as a colorless amorphous substance.
MS(ESI pos.)m/z: 462 ($[M+H]^+$), 484 ($[M+Na]^+$), (ESI neg.)m/z: 460 ($[M-1]^-$).
HRMS(ESI pos.): calcd for $C_{26}H_{29}FN_5O_2$ $[M+H]^+$ 462.2305, found 462.2307.
$^1$H-NMR (300 MHz, DMSO-d6) δ 7.88-7.72 (2H, m), 7.60-7.20 (7H, m), 5.61-5.30 (1H, brd, J=51.8 Hz), 5.08-4.92 (1H, m), 4.81 (2H, brs), 3.89 (1H, dd, J=24.0, 12.5 Hz), 3.80-3.54 (1H, m), 3.50-3.20 (4H, m), 2.60-2.25 (2H, m), 1.11 (4.3H, s), 0.85 (1.7H, s).

With respect to this compound, rotational isomers were observed by $^1$H-NMR. The abundance ratio was about 5:2, as estimated from the integrated value of dimethyl peaks. When heated in DMSO-d6 up to 100°C, the peaks were found to have a tendency to converge.

Example 9

Synthesis of (2S,4S)-2-cyano-4-fluoro-1-[[1-(furan-2-yl)-1-methyl]ethylamino]acetylpyrrolidine

(1) Synthesis of [1-(furan-2-yl)-1-methyl]ethylamine

2-Furonitrile (2 g) was dissolved in toluene (136 mL). To this solution, methylmagnesium bromide (3 M in diethyl ether, 21.4 mL) was added dropwise at room temperature. After heating under reflux for 7 hours in an oil bath, the reaction mixture was cooled with ice and ethanol (13.6 mL) was slowly added dropwise thereto. The suspension was filtered through celite and the filtrate was concentrated under reduced pressure. The resulting residue was purified by silica gel column chromatography (developing solvent; chloroform:methanol:25% aqueous ammonia = 60:1:0.1) to give the titled compound (131 mg) as a brown liquid.
MS(ESI pos.)m/z: 126 ($[M+H]^+$), 109 ($[M-NH_2]^+$).
$^1$H-NMR (300 MHz, $CDCl_3$) δ 7.35 (1H, dd, J=1.8, 0.9 Hz), 6.30 (1H, dd, J=3.3, 1.9 Hz), 6.22 (1H, dd, J=3.3, 0.9 Hz), 1.62 (6H, s).

(2) Synthesis of (2S,4S)-2-cyano-4-fluoro-1-[[1-(furan-2-yl)-1-methyl]ethylamino]acetylpyrrolidine

The same procedure as shown in Example 1(2) was repeated using [1-(furan-2-yl)-1-methyl]ethylamine (110 mg) and (2S,4S)-1-chloroacetyl-2-cyano-4-fluoropyrrolidine (67 mg) to give the titled compound (20 mg) as a brown gum.
MS(ESI pos.)m/z: 302 ($[M+Na]^+$), (ESI neg.)m/z: 278 ($[M-1]^-$).
HRMS(ESI pos.): calcd for $C_{14}H_{18}FN_3O_2Na$ $[M+Na]^+$ 302.1281, found 302.1277.
$^1$H-NMR (300 MHz, DMSO-d6) δ 7.55 (1H, dd, J=1.8, 0.9 Hz), 6.35 (1H, m), 6.21 (1H, dd, J=3.2, 0.9 Hz), 5.40 (1H, brd, J=50.8 Hz), 4.95-4.87 (1H, m), 3.84 (1H, dd, J=23.9, 12.6 Hz), 3.61 (1H, ddd, J=39.6, 12.5, 3.3 Hz), 3.21 and 3.02 (2H, ABq, J=16.0), 2.60-2.22 (2H, m), 1.44-1.32 (6H, m).

Example 10

Synthesis of (2S,4S)-2-cyano-4-fluoro-1-[[1-(thiophen-3-yl)-1-methyl]ethylamino]acetylpyrrolidine

(1) Synthesis of [1-(thiophen-3-yl)-1-methyl]ethylamine

Anhydrous cerium chloride (5.0 g) was suspended in tetrahydrofuran (40 mL) and stirred overnight at room temperature. While cooling with dry ice and acetone, methyllithium (1.2 M in diethyl ether, 16.3 mL) was slowly added dropwise to the suspension, followed by stirring for 30 minutes. To this reaction system, a solution of 3-cyanothiophene

(710 mg) in tetrahydrofuran (1.0 mL) was added dropwise at the same temperature. The reaction mixture was further stirred while gradually warming to room temperature over 5 hours. While stirring the reaction mixture under ice cooling, 25% aqueous ammonia (12.5 mL) was added dropwise. The suspension was filtered through celite and the resulting filtrate was extracted with diethyl ether (25 mL). The extracted solution was washed with saturated aqueous sodium chloride (20 mL), dried over anhydrous magnesium sulfate, filtered to remove the desiccant and then concentrated under reduced pressure to give the titled compound (203 mg) as a black liquid.
MS(ESI pos.)m/z: 142 ([M+H]$^+$), 164 ([M+Na]$^+$), 125 ([M-NH$_2$]$^+$).
$^1$H-NMR (300 MHz, CDCl$_3$) δ 7.27 (1H, dd, J=5.2, 3.2 Hz), 7.13 (1H, d, J=1.4 Hz), 7.11 (1H, dd, J=3.2, 1.3 Hz), 1.49 (6H, s).

(2) Synthesis of (2S,4S)-2-cyano-4-fluoro-1-[[1-(thiophen-3-yl)-1-methyl]ethylamino]acetylpyrrolidine

The same procedure as shown in Example 1(2) was repeated using [1-(thiophen-3-yl)-1-methyl]ethylamine (174 mg) and (2S,4S)-1-chloroacetyl-2-cyano-4-fluoropyrrolidine (106 mg) to give the titled compound (80 mg) as a black oil.
MS(ESI pos.)m/z: 318 ([M+Na]$^+$), (ESI neg.)m/z: 294 ([M-1]$^-$).
HRMS(ESI pos.): calcd for $C_{14}H_{18}FN_3ONaS$[M+Na]$^+$318.1052, found 318.1060.
$^1$H-NMR (300 MHz, DMSO-d6) δ 7.45 (1H, dd, J=5.0, 3.0 Hz), 7.23 (1H, dd, J=3.0, 1.4 Hz), 7.13 (1H, dd, J=5.0, 1.4 Hz), 5.40 (1H, brd, J=52.1 Hz), 4.95-4.87 (1H, m), 3.82 (1H, dd, J=24.1, 12.4 Hz), 3.60 (1H, ddd, J=39.7, 12.4, 3.4 Hz), 3.18 and 3.00 (2H, ABq, J=16.1 Hz), 2.56-2.22 (2H, m), 1.40-1.35 (6H, m).

Example 11

Synthesis of (2S,4S)-2-cyano-4-fluoro-1-[[2-(4-methyl-1,2,3-thiadiazol-5-yl)carbonylamino-1,1-dimethyl]ethylamino] acetylpyrrolidine

(1) Synthesis of [2-(4-methyl-1,2,3-thiadiazol-5-yl)-1,1-dimethyl]ethylamine

The same procedure as shown in Example 1(1) was repeated using 4-methyl-1,2,3-thiadiazole-5-carbonyl chloride (304 mg) and 1,2-diamino-2-methylpropane (329 mg) to give the titled compound (338 mg) as a colorless oil.
MS(ESI pos.)m/z: 215 ([M+H]$^+$), (ESI neg.)m/z: 213 ([M-1]$^-$).

(2) Synthesis of (2S,4S)-2-cyano-4-fluoro-1-[[2-(4-methyl-1,2,3-thiadiazol-5-yl)carbonylamino-1,1-dimethyl]ethylamino] acetylpyrrolidine

The same procedure as shown in Example 1(2) was repeated using [2-(4-methyl-1,2,3-thiadiazol-5-yl)carbonylamino-1,1-dimethyl]ethylamine (256 mg) and (2S,4S)-1-chloroacetyl-2-cyano-4-fluoropyrrolidine (103 mg) to give the titled compound (107 mg) as a colorless amorphous substance.
MS(ESI pos.)m/z: 369 ([M+H]$^+$), 391 ([M+Na]$^+$), (ESI neg.)m/z: 367 ([M-1]$^-$).
HRMS(ESI pos.): calcd for $C_{15}H_{22}FN_6O_2S$[M+H]$^+$ 369.1509, found 369.1516.
$^1$H-NMR (300 MHz, DMSO-d6) δ 8.75-8.60 (1H, m), 5.61-5.30 (1H, m), 4.99-4.92 (1H, m), 3.94 (1H, dd, J=23.9, 12.6 Hz), 3.83-3.61 (1H, m), 3.45-3.21 (4H, m), 2.78 (3H, s), 2.62-2.25 (2H, m), 1.90-1.80 (1H, brs), 1.04 (6H, s).

Example 12

Synthesis of (2S,4S)-2-cyano-1-[[2-(3-cyanobenzoyl)amino-1,1-dimethyl]ethylamino]acetyl-4-fluoropyrrolidine

The same procedure as shown in Example 3 was repeated using 3-cyanobenzoyl chloride (45 mg) and (2S, 4S)-1-[(2-amino-1,1-dimethyl)ethylamino]acetyl-2-cyano-4-fluoropyrrolidine dihydrochloride (93 mg) to give the titled compound (28 mg) as a colorless oil.
MS(ESI pos.)m/z: 371 ([M+H]$^+$), 394 ([M+Na]$^+$), (ESI neg.)m/z: 370 ([M-1]$^-$). HRMS(ESI pos.): calcd for $C_{19}H_{23}FN_5O_2$ [M+H]$^+$ 372.1836, found 372.1848.
$^1$H-NMR (300 MHz, DMSO-d6) δ 8.52-8.44 (1H, m), 8.29 (1H, t, J=1.4 Hz), 8.50 (1H, dt, J=7.9, 1.4 Hz), 8.03-7.98 (1H, m), 7.69 (1H, t, J=7.9 Hz), 5.46 (1H, brd, J=52.8 Hz), 4.99-4.92 (1H, m), 3.96 (1H, dd, J=25.0, 12.2 Hz), 3.73 (1H, ddd, J=39.6, 12.7, 3.3 Hz), 3.52-3.23 (4H, m), 2.62-2.25 (2H, m), 1.04 (6H, s).

Example 13

Synthesis of (2S,4S)-2-cyano-4-fluoro-l-[[2-(2-fluorobenzoyl)amino-1,1-dimethyl]ethylamino]acetylpyrrolidine

The same procedure as shown in Example 3 was repeated using 2-fluorobenzoyl chloride (43 mg) and (2S, 4S)-1-[(2-amino-1,1-dimethyl)ethylamino]acetyl-2-cyano-4-fluoropyrrolidine dihydrochloride (95 mg) to give the titled compound (71 mg) as a colorless amorphous substance.
MS(ESI pos.)m/z: 365 ($[M+H]^+$), 387 ($[M+Na]^+$), (ESI neg.)m/z: 363 ($[M-1]^-$).
HRMS(ESI pos.): calcd for $C_{18}H_{23}F_2N_4O_2$ $[M+H]^+$ 365.1789, found 365.1772.
$^1$H-NMR (300 MHz, DMSO-d6) δ 8.20-8.05 (1H, m), 7.63 (1H, td, J=7.6, 1.7 Hz), 7.58-7.48 (1H, m), 7.34-7.24 (2H, m), 5.49 (1H, brd, J=53.0 Hz), 4.98-4.92 (1H, m), 3.94 (1H, dd, J=23.4, 12.7 Hz), 3.73 (1H, ddd, J=41.2, 12.0, 3.4 Hz), 3.51-3.27 (2H, m), 3.26-3.20 (2H, m), 2.60-2.26 (2H, m), 1.04 (6H, s).

Example 14

Synthesis of (2S,4S)-2-cyano-4-fluoro-1-[[2-(2-quinoxalyl)carbonylamino-1,1-dimethyl]ethylamino]acetylpyrrolidine

(1) Synthesis of [2-(2-quinoxaline)carbonylamino-1,1-dimethyl]ethylamine

The same procedure as shown in Example 1(1) was repeated using 1,2-diamino-2-methylpropane (176 mg) and 2-quinoxalinecarbonyl chloride (193 mg) to give the titled compound (140 mg) as a colorless solid.
MS(ESI pos.)m/z: 267 ($[M+Na]^+$).
$^1$H-NMR (300 MHz, DMSO-d6) δ 8.76 (1H, brt, J=6.1 Hz), 8.26-8.18 (2H, m), 8.04-7.95 (2H, m), 3.28 (2H, d, J=6.1 Hz), 1.07 (6H, s).

(2) Synthesis of (2S,4S)-2-cyano-4-fluoro-1-[[2-(2-quinoxaline)carbonylamino-1,1-dimethyl]ethylamino]acetylpyrrolidine

The same procedure as shown in Example 1(2) was repeated using (2S,4S)-1-chloroacetyl-2-cyano-4-fluoropyrrolidine (48 mg) and [2-(2-quinoxaline)carbonylamino-1,1-dimethyl]ethylamine (123 mg) to give the titled compound (86 mg) as a light-yellow amorphous substance.
MS(ESI pos.)m/z: 399 ($[M+H]^+$), 421 ($[M+Na]^+$), (ESI neg.)m/z: 397 ($[M-H]^-$).
HRMS(ESI pos.): calcd for $C_{20}H_{24}FN_6O_2$ $[M+H]^+$ 399.1945, found 399.1944.
$^1$H-NMR (300 MHz, DMSO-d6) δ 9.50 (1H, s), 8.78 (1H, brt, J=6.1 Hz), 8.26-8.18 (2H, m), 8.04-7.94 (2H, m), 5.51 (1H, brd, J=51.8 Hz), 5.04-4.97 (1H, m), 4.00 (1H, dd, J=25.0, 12.7 Hz), 3.78 (1H, ddd, J=39.5, 12.5, 3.4 Hz), 3.55 and 3.38 (2H, ABq, J=16.6 Hz), 3.36 (2H, d, J=6.1 Hz), 2.62-2.30 (2H, m), 2.01 (1H, brs), 1.09 (6H, s).

Example 15

Synthesis of (2S,4S)-1-[[2-[3-(2-chlorophenyl)-5-methylisoxazol-4-yl]carbonylamino-1,1-dimethyl]ethylamino]acetyl-2-cyano-4-fluoropyrrolidine

The same procedure as shown in Example 3 was repeated using 3-(2-chlorophenyl)-5-methylisoxazole-4-carbonyl chloride (82 mg) and (2S,4S)-1-[(2-amino-1,1-dimethyl)ethylamino]acetyl-2-cyano-4-fluoropyrrolidine dihydrochloride (111 mg) to give the titled compound (86 mg) as a colorless amorphous substance.
MS(ESI pos.)m/z: 462 ($[M+H]^+$), 484 ($[M+Na]^+$), (ESI neg.)m/z: 460 ($[M-1]^-$).
HRMS(ESI pos.): calcd for $C_{22}H_{26}FN_5O_3Cl$ $[M+H]^+$462.1708, found 462.1726.
$^1$H-NMR (300 MHz, DMSO-d6) δ 7.61-7.42 (4H, m), 7.41-7.35 (1H, m), 5.46 (1H, brd, J=53.2 Hz), 4.99-4.93 (1H, m), 3.89 (1H, dd, J=24.0, 12.0 Hz), 3.79-3.57 (1H, m), 3.36-3.10 (2H, m), 3.10-3.00 (2H, m), 2.66 (3H, s), 2.60-2.26 (2H, m), 0.88 (6H, s).

Example 16

Synthesis of (2S,4S)-1-[[2-(1-adamantyl)carbonylamino-1,1-dimethyl]ethylamino]acetyl-2-cyano-4-fluoropyrrolidine

The same procedure as shown in Example 3 was repeated using 1-adamantanecarbonyl chloride (67 mg) and (2S,4S)-1-[(2-amino-1,1-dimethyl)ethylamino]acetyl-2-cyano-4-fluoropyrrolidine dihydrochloride (117 mg) to give the titled compound (80 mg) as a colorless amorphous substance.
MS(ESI pos.)m/z: 405 ($[M+H]^+$), 427 ($[M+Na]^+$), (ESI neg.)m/z: 403 ($[M-1]^-$).

HRMS(ESI pos.): calcd for $C_{22}H_{34}FN_4O_2$ $[M+H]^+$405.2666, found 405.2660.
$^1$H-NMR (300 MHz, DMSO-d6) δ 7.16-7.05 (1H, m), 5.50 (1H, brd, J=53.0 Hz), 5.05-4.93 (1H, m), 4.10-3.85 (1H, m), 3.84-3.62 (1H, m), 3.53-3.20 (2H, m), 3.05-2.97 (2H, m), 2.60-2.25 (2H, m), 2.02-1.92 (3H, m), 1.86-1.60 (12H, m), 0.94 (6H, s).

Example 17

Synthesis of (2S,4S)-2-cyano-4-fluoro-1-[[2-(5-methyl-2-phenyl-1,2,3-triazol-4-yl)carbonylamino-1,1-dimethyl]ethylamino]acetylpyrrolidine

The same procedure as shown in Example 3 was repeated using 4-methyl-2-phenyl-1,2,3-triazole-5-carbonyl chloride (62 mg) and (2S,4S)-1-[(2-amino-1,1-dimethyl)ethylamino]acetyl-2-cyano-4-fluoropyrrolidine dihydrochloride (97 mg) to give the titled compound (64 mg) as a light-yellow amorphous substance.
MS(ESI pos.)m/z: 428 ($[M+H]^+$), 450 ($[M+Na]^+$), (ESI neg.)m/z: 426 ($[M-1]^-$).
HRMS(ESI pos.): calcd for $C_{21}H_{27}FN_7O_2$ $[M+H]^+$428.2210, found 428.2200.
$^1$H-NMR (300 MHz, DMSO-d6) δ 8.35-8.25 (1H, m), 8.07 (2H, d, J=7.8 Hz), 7.59 (2H, t, J=7.8 Hz), 7.50-7.42 (1H, m), 5.46 (1H, brd, J=52.9 Hz), 5.02-4.95 (1H, m), 3.97 (1H, dd, J=24.1, 12.3 Hz), 3.86-3.65 (1H, m), 3.53-3.17 (4H, m), 2.62-2.25 (2H, m), 2.53 (3H, s), 1.05 (6H, s).

Example 18

Synthesis of (2S,4S)-2-cyano-4-fluoro-1-[[2-(2-phenyl-3-propyl-pyrazol-4-yl)carbonylamino-1,1-dimethyl]ethylamino] acetylpyrrolidine

The same procedure as shown in Example 3 was repeated using 1-phenyl-5-N-propylpyrazole-4-carbonyl chloride (66 mg) and (2S,4S)-1-[(2-amino-1,1-dimethyl)ethylamino]acetyl-2-cyano-4-fluoropyrrolidine dihydrochloride (92 mg) to give the titled compound (79 mg) as a colorless amorphous substance.
MS(ESI pos.)m/z: 455 ($[M+H]^+$), 477 ($[M+Na]^+$), (ESI neg.)m/z: 453 ($[M-1]^-$).
HRMS(ESI pos.): calcd for $C_{24}H_{32}FN_6O_2$ $[M+H]^+$455.2571, found 455.2555.
$^1$H-NMR (300 MHz, DMSO-d6) δ 8.14 (1H, s), 7.95-7.85 (1H, m), 7.61-7.43 (5H, m), 5.48 (1H, brd, J=52.5 Hz), 5.02-4.94 (1H, m), 3.96 (1H, dd, J=23.7, 12.3 Hz), 3.86-3.64 (1H, m), 3.58-3.30 (2H, m), 3.26-3.16 (2H, m), 2.97-2.85 (2H, m), 2.62-2.26 (2H, m), 1.46-1.32 (2H, m), 1.04 (6H, s), 0.70 (3H, t, J=7.0 Hz).

Example 19

Synthesis of (2S,4S)-2-cyano-4-fluoro-1-[[2-(2-pyridyl)carbonylamino-1,1-dimethyl]ethylamino]acetylpyrrolidine

The same procedure as shown in Example 4 was repeated using picolinoyl chloride hydrochloride (51 mg) and (2S,4S)-1-[(2-amino-1,1-dimethyl)ethylamino]acetyl-2-cyano-4-fluoropyrrolidine dihydrochloride (100 mg) to give the titled compound (31 mg) as a yellow oil.
MS(ESI pos.)m/z: 348 ($[M+H]^+$), 370 ($[M+Na]^+$), (ESI neg.)m/z: 346 ($[M-1]^-$).
HRMS(ESI pos.): calcd for $C_{17}H_{23}FN_5O_2$ $[M+H]^+$348.1836, found 348.1831.
$^1$H-NMR (300 MHz, DMSO-d6) δ 8.69-8.63 (1H, m), 8.61-8.52 (1H, m), 8.05 (1H, m), 8.00 (1H, td, J=7.3, 1.7 Hz), 7.65-7.57 (1H, m), 5.46 (1H, brd, J=52.8 Hz), 5.01-4.94 (1H, m), 3.96 (1H, dd, J=23.9, 11.8 Hz), 3.86-3.64 (1H, m), 3.54-3.24 (4H, m), 2.62-2.25 (2H, m), 1.04 (6H, s).

Example 20

Synthesis of (2S,4S)-2-cyano-1-[[2-[4-[(N, N-dimethylaminomethylene)aminosulfonyl]benzoyl]amino-1,1-dimethyl] ethylamino]acetyl-4-fluoropyrrolidine

The same procedure as shown in Example 4 was repeated using sulfamidobenzoyl chloride/N,N-dimethylformamide complex (72 mg) and (2S,4S)-1-[(2-amino-1,1-dimethyl)ethylamino]acetyl-2-cyano-4-fluoropyrrolidine dihydrochloride (81 mg) to give the titled compound (55 mg) as a colorless amorphous substance.
MS(ESI pos.)m/z: 481 ($[M+H]^+$), 503 ($[M+Na]^+$), (ESI neg.)m/z: 479 ($[M-1]^-$).
HRMS(ESI pos.): calcd for $C_{21}H_{30}FN_6O_4S$ $[M+H]^+$481.2033, found 481.2027.
$^1$H-NMR (300 MHz, DMSO-d6) δ 8.44-8.36 (1H, m), 8.24 (1H, s), 7.95 (2H, d, J=8.4 Hz), 7.84 (2H, d, J=8.4 Hz), 5.60-5.35 (1H, m), 4.99-4.92 (1H, m), 3.95 (1H, dd, J=24.1, 12.4 Hz), 3.84-3.63 (1H, m), 3.52-3.19 (4H, m), 3.15 (3H, s), 2.91 (3H,

s), 2.62-2.25 (2H, m), 2.00-1.98 (1H, brs), 1.03 (3H, s), 1.02 (3H, s).

Example 21

Synthesis of (2S,4S)-2-cyano-4-fluoro-1-[[2-(5-methyl-2-trifluoromethylfuran-3-yl)carbonylamino-1,1-dimethyl]ethylamino]acetylpyrrolidine

The same procedure as shown in Example 4 was repeated using 5-methyl-2-(trifluoromethyl)furan-3-carbonyl chloride (51 mg) and (2S,4S)-1-[(2-amino-1,1-dimethyl)ethylamino]acetyl-2-cyano-4-fluoropyrrolidine dihydrochloride (83 mg) to give the titled compound (60 mg) as a colorless amorphous substance.
MS(ESI pos.)m/z: 419 ([M+H]$^+$), 441 ([M+Na]$^+$), (ESI neg.)m/z: 417 ([M-1]$^-$).
HRMS(ESI pos.): calcd for $C_{18}H_{23}F_4N_4O_3S$ [M+H]$^+$419.1706, found 419.1691.
$^1$H-NMR (300 MHz, DMSO-d6) δ 8.30-8.22 (1H, m), 6.63 (1H, s), 5.49 (1H, brd, J=53.0 Hz), 4.98-4.92 (1H, m), 3.94 (1H, dd, J=24.1, 12.3 Hz), 3.72 (1H, ddd, J=39.7, 12.4, 3.4 Hz), 3.48-3.24 (2H, m), 3.24-3.08 (2H, m), 2.62-2.25 (2H, m), 2.36 (3H, s), 1.95-1.85 (1H, brs), 1.00 (6H, s).

Example 22

Synthesis of (2S,4S)-2-cyano-4-fluoro-1-[[2-(4-morpholino)carbonylamino-1,1-dimethyl]ethylamino]acetylpyrrolidine

The same procedure as shown in Example 4 was repeated using morpholine-4-carbonyl chloride (50 mg) and (2S,4S)-1-[(2-amino-1,1-dimethyl)ethylamino]acetyl-2-cyano-4-fluoropyrrolidine dihydrochloride (117 mg) to give the titled compound (105 mg) as a colorless amorphous substance.
MS(ESI pos.)m/z: 356 ([M+H]$^+$), 378 ([M+Na]$^+$), (ESI neg.)m/z: 354 ([M-1]$^-$).
HRMS(ESI pos.): calcd for $C_{16}H_{27}FN_5O_3$ [M+H]$^+$356.2098, found 356.2105.
$^1$H-NMR (300 MHz, DMSO-d6) δ 6.36-6.26 (1H, m), 5.46 (1H, brd, J=53.0 Hz), 4.99-4.92 (1H, m), 3.94 (1H, dd, J=24.0, 12.5 Hz), 3.72 (1H, ddd, J=39.4, 12.8, 3.4 Hz), 3.61-3.47 (4H, m), 3.46-3.19 (6H, m), 3.00 (2H, d, J=5.8 Hz), 2.62-2.25 (2H, m), 1.90-1.80 (1H, brs), 0.95 (6H, s).

Example 23

Synthesis of (2S,4S)-1-[[2-(2-carboxyphenyl)carbonylamino-1,1-dimethyl]ethylamino]acetyl-2-cyano-4-fluoropyrrolidine

(2S,4S)-1-[(2-Amino-1,1-dimethyl)ethylamino]acetyl-2-cyano-4-fluoropyrrolidine dihydrochloride (158 mg) was suspended in dioxane (2 mL). To this suspension, triethylamine (0.14 mL) and N,N-dimethylformamide (2 mL) were added. After further addition of phthalic anhydride (74 mg), the resulting mixture was stirred overnight at room temperature. The solvent was distilled off under reduced pressure and the residue was purified by resin column chromatography (resin: Toyopearl, developing solvent; 0.1 M aqueous hydrochloric acid) to give the titled compound (123 mg) as a colorless amorphous substance.
MS(ESI pos.)m/z: 391 ([M+H]$^+$), 413 ([M+Na]$^+$), (ESI neg.)m/z: 389 ([M-H]$^-$).
HRMS(ESI pos.): calcd for $C_{19}H_{24}FN_4O_4$ [M+H]$^+$ 389.1625, found 389.1640.
$^1$H-NMR (300 MHz, DMSO-d6) δ: 8.64 (1H, brt, J=6.1 Hz), 7.95-7.51 (4H, m), 5.57 (1H, brd, J=52.8 Hz), 5.13-5.05 (1H, m), 4.34-3.42 (6H, m), 2.58-2.40 (2H, m), 1.35 (3H, s), 1.34 (3H, s).

Example 24

Synthesis of (2S,4S)-2-cyano-1-[[2-(2-cyanobenzene)sulfonylamino-1,1-dimethyl]ethylamino]acetyl-4-fluoropyrrolidine

The same procedure as shown in Example 5 was repeated using (2S,4S)-1-[(2-amino-1,1-dimethyl)ethylamino]acetyl-2-cyano-4-fluoropyrrolidine dihydrochloride (95 mg), triethylamine (0.13 mL) and 2-cyanobenzenesulfonyl chloride (54 mg) to give the titled compound (41 mg) as a light-yellow amorphous substance.
MS(ESI pos.)m/z: 408 ([M+H]$^+$), 430 ([M+Ma]$^+$), (ESI neg.)m/z: 406 ([M-H]$^-$).
HRMS(ESI pos.): calcd for $C_{18}H_{23}FN_5O_3S$ [M+H]$^+$ 408.1506, found 408.1512.
$^1$H-NMR (300 MHz, DMSO-d6) δ 8.20-7.78 (5H, m), 5.48 (1H, brd, J=53.0 Hz), 4.97-4.91 (1H, m), 3.88 (1H, dd, J=23.8, 12.0 Hz), 3.67 (1H, ddd, J=39.6, 12.3, 3.4 Hz), 3.33 and 3.18 (2H, ABq, J=16.5 Hz), 2.86 (2H, s), 2.60-2.26 (2H, m), 0.96 (6H, s).

Example 25

Synthesis of (2S,4S)-2-cyano-4-fluoro-1-[(2-methanesulfonylamino-1,1-dimethyl)ethylamino]acetylpyrrolidine

The same procedure as shown in Example 5 was repeated using methanesulfonyl chloride (24 μL) and (2S, 4S)-1-[(2-amino-1,1-dimethyl)ethylamino]acetyl-2-cyano-4-fluoropyrrolidine dihydrochloride (108 mg) to give the titled compound (51 mg) as a colorless amorphous substance.
MS(ESI pos.)m/z: 321 ([M+H]$^+$), 343 ([M+Na]$^+$), (ESI neg.)m/z: 319 ([M-1]$^-$).
HRMS(ESI pos.): calcd for $C_{12}H_{22}FN_4O_3S$[M+H]$^+$ 321.1397, found 321.1405.
$^1$H-NMR (300 MHz, DMSO-d6) δ 6.90-6.76 (1H, m), 5.46 (1H, brd, J=51.0 Hz), 4.99-4.91 (1H, m), 3.93 (1H, dd, J=24.4, 12.6 Hz), 3.72 (1H, ddd, J=39.6, 12.6, 3.4 Hz), 3.45-3.20 (2H, m), 2.89 (3H, s), 2.87-2.81 (2H, m), 2.62-2.26 (2H, m), 1.00 (6H, s).

Example 26

Synthesis of (2S,4S)-2-cyano-1-(1,1-diethylpropargylamino)acetyl-4-fluoropyrrolidine

The same procedure as shown in Example 1(2) was repeated using (2S,4S)-1-chloroacetyl-2-cyano-4-fluoropyrrolidine (191 mg) and 1,1-diethylpropargylamine (333 mg) to give the titled compound (215 mg) as a colorless solid.
MS(ESI pos.)m/z: 288 ([M+Na]$^+$).
HRMS(ESI pos.): calcd for $C_{14}H_{21}FN_3O$ [M+H]$^+$ 266.1669, found 266.1654.
$^1$H-NMR (300 MHz, DMSO-d6) δ 5.48 (1H, brd, J=51.5 Hz), 4.99-4.93 (1H, m), 3.95 (1H, dd, J=24.6, 12.6 Hz), 3.72 (1H, ddd, J=39.6, 12.6, 3.4 Hz), 3.50-3.27 (2H, m), 3.18 (1H, s), 2.62-2.28 (2H, m), 2.06 (1H, t, J=5.9 Hz), 1.53 (4H, q, J=7.4 Hz), 0.87 (6H, t, J=7.4 Hz).

Example 27

Synthesis of (2S,4S)-2-cyano-1-(1,1-dimethylcinnamylamino)acetyl-4-fluoropyrrolidine

(1) Synthesis of 1,1-dimethylcinnamylamine

The same procedure as shown in Example 10(1) was repeated using cinnamonitrile (500 mg) to give the titled compound (210 mg) as a brown oil.
MS(ESI pos.)m/z: 162 ([M+H]$^+$), 184 ([M+Na]$^+$), 145 ([M-$NH_2$]$^+$).

(2) Synthesis of (2S,4S)-2-cyano-1-(1,1-dimethylcinnamylamino)acetyl-4-fluoropyrrolidine

The same procedure as shown in Example 1(2) was repeated using 1,1-dimethylcinnamylamine (200 mg) and (2S,4S)-1-chloroacetyl-2-cyano-4-fluoropyrrolidine (107 mg) to give the titled compound (74 mg) as a colorless powder.
MS(ESI pos.)m/z: 338 ([M+Na]$^+$).
HRMS(ESI pos.): calcd for $C_{18}H_{22}FN_3ONa$[M+Na]$^+$ 338.1645, found 338.1641.
$^1$H-NMR (300 MHz, DMSO-d6) δ 7.44-7.38 (2H, m), 7.35-7.27 (2H, m), 7.25-7.17 (1H, m), 6.40 (1H, d, J=16.3 Hz), 6.21 (1H, d, J=16.3 Hz), 5.42 (1H, brd, J=51.8 Hz), 4.91 (1H, d, J=8.9 Hz), 3.90 (1H, dd, J=23.5, 12.4 Hz), 3.78-3.56 (1H, m), 3.44-3.14 (2H, m), 2.60-2.20 (2H, m), 2.06-1.98 (1H, m), 1.22 (6H, s).

Example 28

Synthesis of (2S,4S)-2-cyano-4-fluoro-1-[[2-(pyridin-2-yl)-1,1-bis(hydroxymethyl)]ethylamino]acetylpyrrolidine

(1) Synthesis of 2-[N-(tert-butoxycarbonyl)amino]-2-(pyridin-2-yl)methylmalonic acid diethyl ester

Under a nitrogen atmosphere, 60% sodium hydride in oil (0.88 g) was suspended in N,N-dimethylformamide (10 mL). To this suspension, a solution of 2-[N-(tert-butoxycarbonyl)amino]malonic acid diethyl ester (2.75 g) in N, N-dimethylformamide (110 mL) was added at room temperature. A solution of 2-(chloromethyl)pyridine hydrochloride (1.64 g) in N,N-dimethylformamide (10 mL) was then added and stirring was continued overnight at room temperature. The reaction mixture was partitioned by addition of ethyl acetate (100 mL) and saturated aqueous sodium chloride (100 mL). The extracted solution was washed four times with saturated aqueous sodium chloride (50 mL), dried over anhydrous magnesium sulfate, filtered to remove the desiccant and then concentrated under reduced pressure. The residue was

purified by silica gel column chromatography (developing solvent; ethyl acetate:hexane = 1:7 to 1:5) to give the titled compound (2.61 g) as a colorless oil.
MS(ESI pos.)m/z: 389 ([M+Na]$^+$).
$^1$H-NMR (300 MHz, DMSO-d6) δ 8.44 (1H, ddd, J=4.8, 1.7, 0.9 Hz), 7.57 (1H, td, J=7.6, 1.9 Hz), 7.14-7.08 (2H, m), 5.95 (1H, brs), 4.38-4.20 (4H, m), 3.80 (2H, s), 1.42 (9H, s), 1.28 (6H, t, J=7.1 Hz).

(2) Synthesis of N-(tert-butoxycarbonyl)-2-(pyridin-2-yl)-1,1-bis(hydroxymethyl)ethylamine

Under a nitrogen atmosphere, lithium borohydride (0.419 g) was suspended in tetrahydrofuran (30 mL) and cooled with ice. To this suspension, a solution of 2-[N-(tert-butoxycarbonyl)amino]-2-(pyridin-2-yl)methylmalonic acid diethyl ester (2.35 g) in tetrahydrofuran (15 mL) was added dropwise over 15 minutes. The reaction mixture was warmed to room temperature and stirred overnight. After cooling again with ice, 10% aqueous potassium carbonate (20 mL) was added. The reaction mixture was partitioned by addition of ethyl acetate (100 mL) and saturated aqueous sodium chloride (50 mL). The extracted solution was washed with saturated aqueous sodium chloride (50 ml), dried over anhydrous magnesium sulfate, filtered to remove the desiccant and then concentrated under reduced pressure. The residue was purified by silica gel column chromatography (developing solvent; ethyl acetate:hexane = 3:2 to 7:1) to give the titled compound (0.22 g) as a colorless oil.
MS(ESI pos.)m/z: 305 ([M+Na]$^+$), (ESI neg.)m/z: 281 ([M-H]$^-$).
$^1$H-NMR (300 MHz, $CDCl_3$) δ 8.50 (1H, d like, J=5.0 Hz), 7.66 (1H, t like, J=7.8 Hz), 7.30 (1H, d, J=7.9 Hz), 7.21 (1H, t like, J=5.0 Hz), 5.49 (1H, brs), 5.15 (2H, brs), 3.63 and 3.49 (4H, ABq, J=11.7 Hz), 3.27 (2H, s), 1.39 (9H, s).

(3) Synthesis of 2-(pyridin-2-yl)-1,1-bis(hydroxymethyl)ethylamine dihydrochloride

When adding 4 M hydrochloric acid in dioxane (3 mL) at room temperature, N-(tert-butoxycarbonyl)-2-(pyridin-2-yl)-1,1-bis(hydroxymethyl)ethylamine (210 mg) was converted into a white waxy product. When adding methanol (0.6 mL), this product was gradually converted into a powder suspension. After stirring for 30 minutes at room temperature, the precipitate was collected by filtration to give the titled compound (166 mg) as a colorless powder.
MS(ESI pos.)m/z: 182.9 ([M+H]$^+$), 204.9 ([M+Na]$^+$).
$^1$H-NMR (300 MHz, DMSO-d6) δ 8.72 (1H, d, J=5.1 Hz), 8.35-8.10 (4H, m), 7.84-7.67 (2H, m), 3.53 and 3.48 (4H, ABq, J=11.5 Hz), 3.28 (2H, s).

(4) Synthesis of (2S,4S)-2-cyano-4-fluoro-1-[[2-(pyridin-2-yl)-1,1-bis(hydroxymethyl)]ethylamino]acetylpyrrolidine

The same procedure as shown in Example 1(2) was repeated using 2-(pyridin-2-yl)-1,1-bis(hydroxymethyl) ethylamine dihydrochloride (160 mg), (2S,4S)-1-chloroacetyl-2-cyano-4-fluoropyrrolidine (80 mg), potassium iodide (70 mg) and triethylamine (0.23 mL) to give the titled compound (30 mg) as a colorless powder.
MS(ESI pos.)m/z: 337 ([M+H]$^+$), 359 ([M+Na]$^+$), (ESI neg.)m/z: 335 ([M-H]$^-$).
HRMS(ESI pos.): calcd for $C_{16}H_{22}FN_4O_3$ [M+H]$^+$ 337.1676, found 337.1680.
$^1$H-NMR (300 MHz, DMSO-d6) δ 8.45 (1H, d like, J=5.2 Hz), 7.69 (1H, td, J=6.0, 1.7 Hz), 7.31 (1H, d, J=7.9 Hz), 7.21 (1H, dd, J=6.9, 5.2 Hz), 5.48 (1H, brd, J=50.2 Hz), 4.97-4.91 (1H, m), 4.70-4.63 (2H, m), 3.99 (1H, dd, J=24.3, 12.5 Hz), 3.80-3.64 (1H, m), 3.60 and 3.46 (2H, ABq, J=16.4 Hz), 3.31-3.20 (4H, m), 2.80 (2H, s), 2.60-2.25 (2H, m).

Example 29

Synthesis of (2S,4S)-1-[[1-(benzofuran-2-yl)-1-methyl]ethylamino]acetyl-2-cyano-4-fluoropyrrolidine

(1) Synthesis of [1-(benzofuran-2-yl)-1-methyl]ethylamine

The same procedure as shown in Example 9(1) was repeated using 2-benzofurancarbonitrile (1000 mg) to give the titled compound (284 mg) as a brown oil.
MS(ESI pos.)m/z: 159 ([M-$NH_2$]$^+$).

(2) Synthesis of (2S,4S)-1-[[1-(benzofuran-2-yl)-1-methyl]ethylamino]acetyl-2-cyano-4-fluoropyrrolidine

The same procedure as shown in Example 9(2) was repeated using [1-(benzofuran-2-yl)-1-methyl]ethylamine (204 mg) and (2S,4S)-1-chloroacetyl-2-cyano-4-fluoropyrrolidine (89 mg) to give the titled compound (93 mg) as a light-yellow solid.
MS(ESI pos.)m/z: 352 ([M+Na]$^+$), (ESI neg.)m/z: 328 ([M-1]$^-$).

HRMS(ESI pos.): calcd for $C_{18}H_{20}FN_3O_2Na[M+Na]^+$ 352.1437 found 352.1454.
$^1$H-NMR (300 MHz, DMSO-d6) δ 7.59-7.48 (2H, m), 7.28-7.26 (2H, m), 6.70 (1H, s), 5.38 (1H, brd, J=52.2 Hz), 4.90-4.83 (1H, m), 3.85 (1H, dd, J=23.2, 12.3 Hz), 3.60 (1H, ddd, J=39.6, 12.5, 3.4 Hz), 3.38-3.04 (2H, m), 2.60-2.18 (2H, m), 1.48 (6H, s).

Example 30

Synthesis of (2S,4S)-2-cyano-4-fluoro-1-[[1-(pyridin-2-yl)-1-methyl]ethylamino]acetylpyrrolidine

(1) Synthesis of [1-(pyridin-2-yl)-1-methyl]ethylamine

The same procedure as shown in Example 10(1) was repeated using 2-cyanopyridine (685 mg) to give the titled compound (196 mg) as a brown oil.
MS(ES+)m/z: 137 ($[M+H]^+$), 159 ($[M+Na]^+$), 120 ($[M-NH_2]^+$).

(2) Synthesis of (2S,4S)-2-cyano-4-fluoro-1-[[1-(pyridin-Z-yl)-1-methyl]ethylamino]acetylpyrrolidine

The same procedure as shown in Example 1(2) was repeated using [1-(pyridin-2-yl)-1-methyl]ethylamine (177 mg) and (2S,4S)-1-chloroacetyl-2-cyano-4-fluoropyrrolidine (112 mg) to give the titled compound (106 mg) as a light brown amorphous substance. For purification, preparative TLC (developing solvent; chloroform:methanol = 5:1) was used.
MS(ESI pos.)m/z: 291 ($[M+H]^+$), 313 ($[M+Na]^+$), (ESI neg.)m/z: 289 ($[M-1]^-$).
HRMS(ESI pos.): calcd for $C_{15}H_{20}FN_4O[M+H]^+$ 291.1621, found 291.1612.
$^1$H-NMR (300 MHz, $CDCl_3$) δ 8.61-8.54 (1H, m), 7.67 (1H, td, J=7.7, 1.9 Hz), 7.49-7.39 (1H, m), 7.19-7.12 (1H, m), 5.48-5.16 (1H, m), 4.92 (1H, d, J=9.2 Hz), 4.03-3.26 (4H, m), 2.63 (1H, t, J=15.8 Hz), 2.40-2.12 (2H, m), 1.53 (6H, s).

Table 1

| Example No. | Stractural formula | Example No. | Stractural formula |
|---|---|---|---|
| 1 | 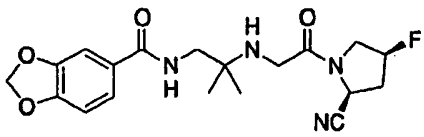 | 7 | 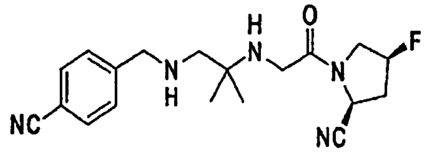 |
| 2 | 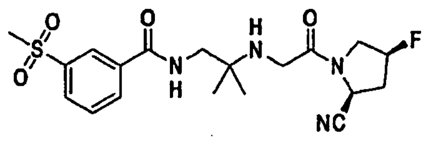 | 8 | 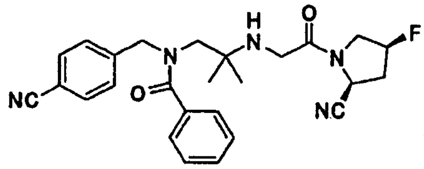 |
| 3 | 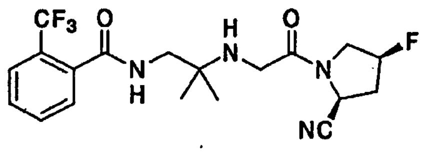 | 9 | 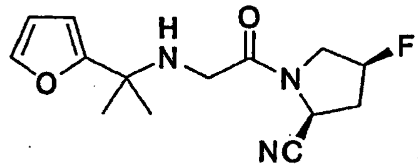 |
| 4 | 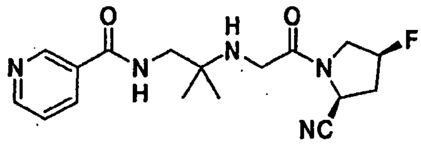 | 10 | 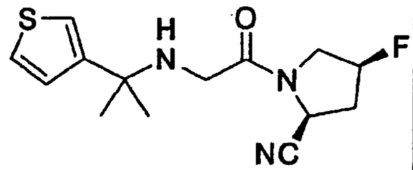 |
| 5 | 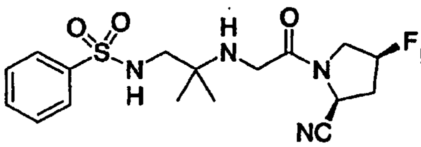 | 11 | 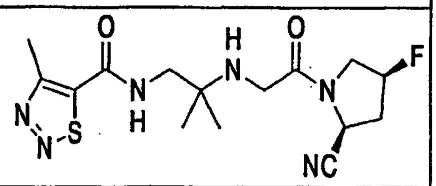 |
| 6 | 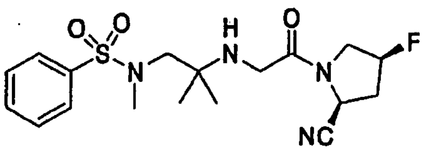 | 12 | 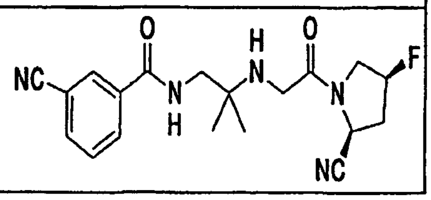 |

Table 1 (continued-1)

| Example No. | Stractural formula | Example No. | Stractural formula |
|---|---|---|---|
| 13 | 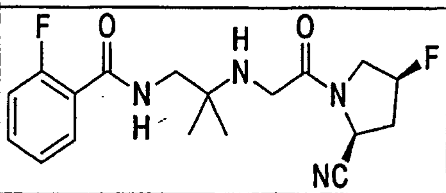 | 19 | 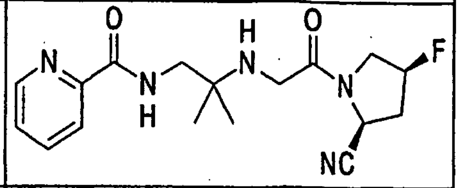 |
| 14 | 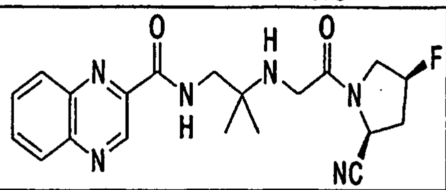 | 20 | 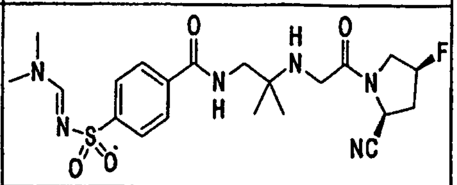 |
| 15 | 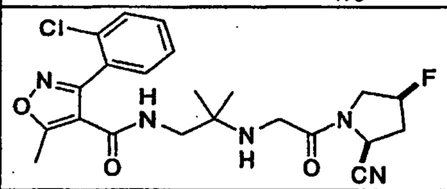 | 21 | 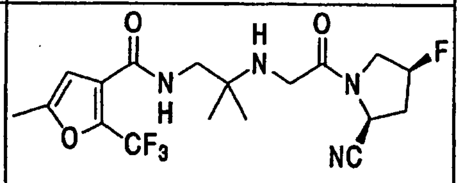 |
| 16 | 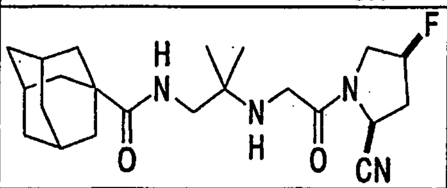 | 22 | 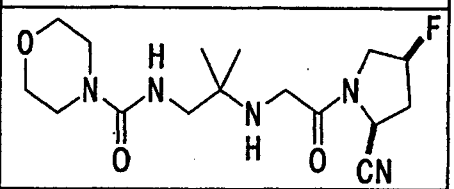 |
| 17 | 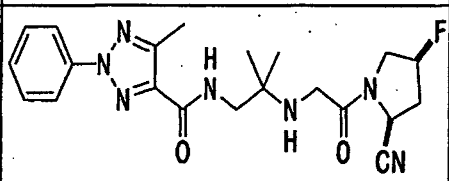 | 23 | 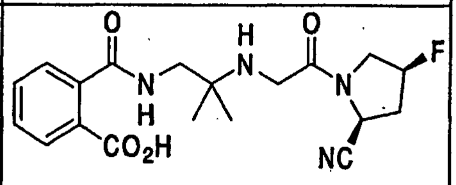 |
| 18 | 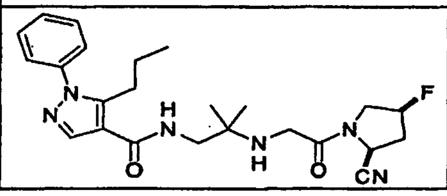 | 24 | 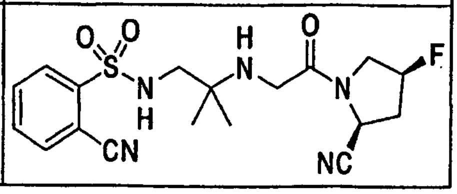 |

Table 1 (continued-2)

| Example No. | Stractural formula | Example No. | Stractural formula |
|---|---|---|---|
| 25 | 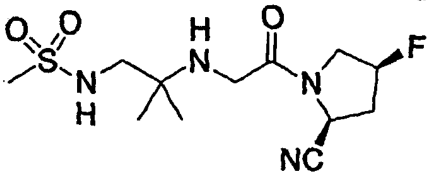 | 28 | 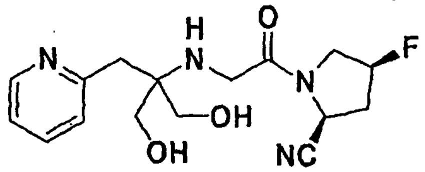 |
| 26 | 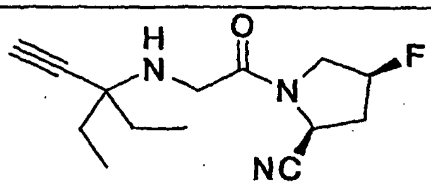 | 29 | 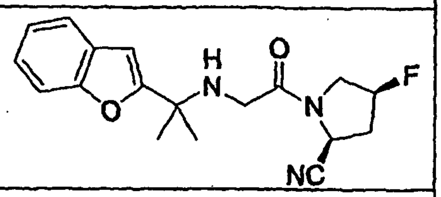 |
| 27 | 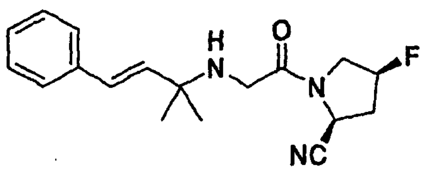 | 30 | 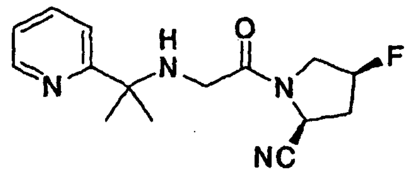 |

Examples 31 to 47

Table 2 below shows the compounds obtained in the same manner as used in Example 3(4).

Table 2

| Example No. | Structural formula | $^1$H-NMR (300 MHz, DMSO-d6) $\delta$ |
|---|---|---|
| 31 | 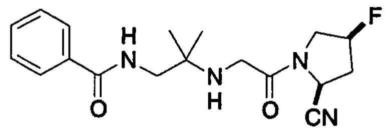 | 8.23 (1H, m), 7.86 (1H, brd), 7.83 (1H, brd), 7.55-7.43 (3H, m), 5.50 (1H, brd, J=52.8 Hz), 4.98-4.95 (1H, m), 3.96 (1H, dd, J=23.8, 12.4 Hz), 3.74 (1H, ddd, J=39.7, 12.5, 3.3 Hz), 3.55-3.30 (2H, m), 3.27-3.20 (2H, m), 2.59-2.28 (2H, m), 1.98 (1H, brs), 1.02 (6H, brs). |
| 32 | 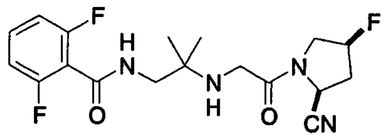 | 8.59 (1H, m), 7.50 (1H, m), 7.16 (2H, brt, J=8.0 Hz), 5.49 (1H, brd, J=52.2 Hz), 4.96-4.93 (1H, m), 3.93 (1H, dd, J=24.7, 12.8 Hz), 3.72 (1H, m), 3.58-3.40 (2H, m), 3.24 (2H, m), 2.40-2.28 (2H, m), 1.80 (1H, brs), 1.03 (6H, brs). |
| 33 | 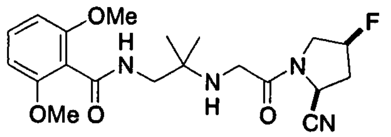 | 7.88 (1H, m), 7.27 (1H, t, J=8.4 Hz), 6.66 (2H, d, J=8.4 Hz), 5.48 (1H, brd, J=51.0 Hz), 4.95-4.92 (1H, m), 3.92 (1H, dd, J=24.4, 12.3 Hz), 3.72 (6H, s), 3.70 (1H, m), 3.57-3.26 (2H, m), 3.14 (2H, brd, J=6.1 Hz), 2.46-2.27 (2H, m), 1.83 (1H, brs), 1.02 (6H, brs). |
| 34 | 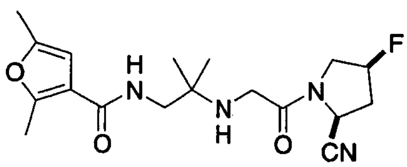 | 7.65-7.55 (1H, m), 6.44 (1H, s), 5.46 (1H, brd, J=52.8 Hz), 4.99-4.91 (1H, m), 3.95 (1H, dd, J=24.2, 12.0 Hz), 3.73 (1H, ddd, J=39.5, 12.6, 3.3 Hz), 3.52-3.25 (2H, m), 3.19-3.08 (2H, d, J=6.1 Hz), 2.62-2.25 (2H), 2.44 (3H, s), 2.22 (3H, s), 0.99 (3H, s), 0.98 (3H, s). |
| 35 | 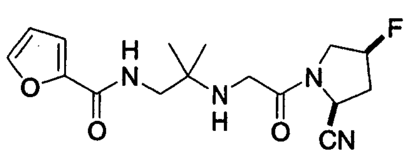 | 8.07-7.95 (1H, m), 7.83 (1H, m), 7.11 (1H, d, J=3.4 Hz), 6.62 (1H, m), 5.46 (1H, brd, J=51.1 Hz), 5.00-4.93 (1H, m), 3.95 (1H, dd, J=24.1, 12.7 Hz), 3.73 (1H, ddd, J=39.4, 12.7, 3.1 Hz), 3.52-3.27 (2H, m), 3.25-3.10 (2H, m), 2.62-2.25 (2H), 1.96-1.84 (1H, brs), 1.01 (6H, s). |

| | | |
|---|---|---|
| 36 | 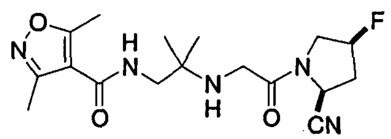 | 7.90-7.81 (1H, m), 5.46 (1H, brd, J=50.2 Hz), 4.98-4.91 (1H, m), 3.94 (1H, dd, J=24.6, 12.4 Hz), 3.71 (1H, ddd, J=39.6, 12.4, 3.3 Hz), 3.51-3.26 (2H, m), 3.25-3.10 (2H, m), 2.62-2.25 (2H, m), 2.52 (3H, s), 2.30 (3H, s), 1.98-1.84 (1H, brs), 1.03 (6H, s). |
| 37 | 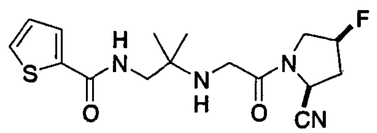 | 8.30-8.21 (1H, m), 7.80 (1H, d, J=3.7 Hz), 7.74 (1H, d, J=4.8 Hz), 7.15 (1H, dd, J=4.8, 3.7 Hz), 5.45 (1H, brd, J=50.4 Hz), 5.00-4.93 (1H, m), 3.96 (1H, dd, J=23.8, 12.4 Hz), 3.73 (1H, ddd, J=39.5, 12.4, 3.4 Hz), 3.52-3.28 (2H, m), 3.26-3.12 (2H, m), 2.62-2.25 (2H, m), 1.98-1.86 (1H, brs), 1.02 (3H, s), 1.01 (3H, s). |
| 38 | 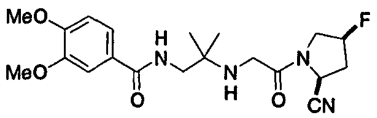 | 8.20-8.10 (1H, m), 7.48-7.43 (2H, m), 7.05-6.98 (1H, d, J=8.2 Hz), 5.46 (1H, brd, J=52.4 Hz), 5.00-4.93 (1H, m), 3.96 (1H, dd, J=24.2, 12.7 Hz), 3.86-3.63 (1H), 3.82 (3H, s), 3.80 (3H, s), 3.54-3.30 (2H), 3.25-3.18 (2H, m), 2.62-2.25 (2H, m), 2.02-1.90 (1H, brs), 1.02 (3H, s), 1.01 (3H, s). |
| 39 | 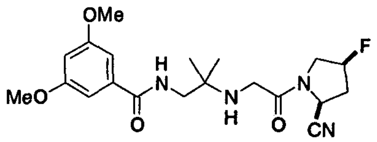 | 8.30-8.21 (1H, m), 7.03-6.99 (2H, m), 6.66-6.61 (1H, t, J=2.3 Hz), 5.62-5.30 (1H, m), 5.00-4.92 (1H, m), 3.96 (1H, dd, J=23.9, 12.9 Hz), 3.85-3.63 (1H), 3.79 (6H, s), 3.54-3.26 (2H), 3.25-3.17 (2H, m), 2.62-2.25 (2H, m), 1.02 (3H, s), 1.01 (3H, s). |
| 40 | 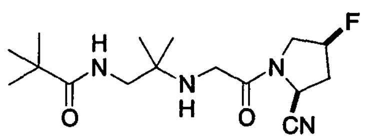 | 7.27-7.15 (1H, m), 5.62-5.30 (1H, m), 4.99-4.92 (1H, m), 3.94 (1H, dd, J=24.1, 11.9 Hz), 3.72 (1H, ddd, J=39.6, 12.5, 3.5 Hz), 3.47-3.21 (2H, m), 3.08-2.92 (2H, m), 2.56-2.26 (2H, m), 1.12 (9H, s), 0.94 (6H, d, J=2.5 Hz). |
| 41 | 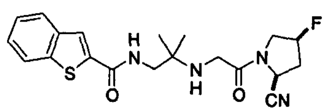 | 8.58-8.48 (1H, m), 8.15 (1H, s), 8.05-7.98 (1H, m), 7.97-7.90 (1H, m), 7.50-7.40 (2H, m), 5.46 (1H, brd, J=51.6 Hz), 5.00-4.94 (1H, m), 3.96 (1H, dd, J=25.1, 11.9 Hz), 3.75 (1H, ddd, J=39.6, 12.5, 3.3 Hz), 3.40-3.17 (4H, m), 2.59-2.26 (2H, m), 1.96 (1H, brs), 1.05 (6H, s). |
| 42 | 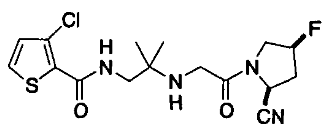 | 8.00-7.92 (1H, m), 7.85 (1H, d, J=5.3 Hz), 7.16 (1H, d, J=5.3 Hz), 5.60-5.30 (1H, m), 5.00-4.92 (1H, m), 3.94 (1H, dd, J=24.2, 11.8 Hz), 3.72 (1H, ddd, J=39.5, 12.5, 3.4 Hz), 3.52 (4H, m), 2.62-2.26 (2H, m), 1.93 (1H, brs), 1.05 (6H, s). |

| | | |
|---|---|---|
| 43 | 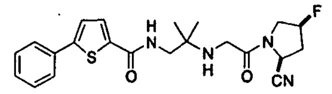 | 8.35-8.28 (1H, m), 7.82 (1H, d, J=3.9 Hz), 7.74-7.68 (2H, m), 7.55 (1H, d, J=3.9 Hz), 7.50-7.33 (3H, m), 5.46 (1H, brd, J=49.7 Hz), 5.00-4.93 (1H, m), 3.96 (1H, dd, J=24.3, 12.0 Hz), 3.74 (1H, ddd, J=39.6, 12.5, 3.3 Hz), 3.54-3.27 (2H, m), 3.26-3.14 (2H, m), 2.62-2.25 (2H, m), 1.03 (3H, s), 1.02 (3H, s). |
| 44 | 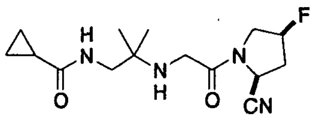 | 7.86 (1H, t, J=5.8 Hz), 5.46 (1H, brd, J=51.5 Hz), 4.99-4.91 (1H, m), 3.92 (1H, dd, J=23.6, 12.3 Hz), 3.82-3.60 (1H, m), 3.45-3.20 (2H, m), 3.07-3.00 (2H, d, J=6.0 Hz), 2.62-2.26 (2H, m), 1.95-1.75 (1H, m), 1.70-1.59 (1H, m), 0.97 (3H, s), 0.69-0.57 (7H, m). |
| 45 | 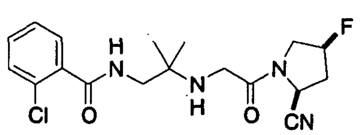 | 8.35-8.25 (1H, m), 7.52-7.34 (4H, m), 5.45 (1H, brd, J=50.7 Hz), 4.98-4.91 (1H, m), 3.93 (1H, dd, J=24.1, 12.6 Hz), 3.72 (1H, ddd, J=39.5, 12.6, 3.4 Hz), 3.25-3.16 (2H), 3.25-3.14 (2H, m), 2.62-2.25 (2H, m), 1.05 (6H, s). |
| 46 | 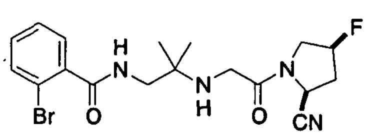 | 8.35-8.24 (1H, m), 7.65 (1H, d, J=7.4 Hz), 7.47-7.31 (3H, m), 5.45 (1H, brd, J=52.0 Hz), 4.98-4.90 (1H, m), 3.93 (1H, dd, J=23.0, 12.0 Hz), 3.72 (1H, ddd, J=39.6, 12.5, 3.4 Hz), 3.49-3.26 (2H, m), 3.25-3.16 (2H, m), 2.62-2.25 (2H, m), 1.06 (6H, s). |
| 47 | 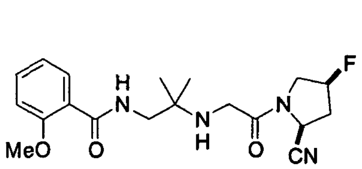 | 8.35-8.25 (1H, m), 7.80 (1H, dd, J=7.6, 1.9 Hz), 7.51-7.43 (1H, m), 7.15 (1H, d, J=8.4 Hz), 7.03 (1H, t, J=7.6 Hz), 5.45 (1H, brd, J=51.7 Hz), 5.01-4.93 (1H, m), 4.03-3.86 (1H, m), 3.90 (3H, s), 3.73 (1H, ddd, J=39.5, 12.5, 3.4 Hz), 3.52-3.21 (4H), 2.62-2.25 (2H, m), 1.95-1.82 (1H, m), 1.04 (6H, s). |

Example 48

Synthesis of (2S,4S)-2-cyano-4-fluoro-1-[(2-isobutylamino-1,1-dimethyl)ethylamino]acetylpyrrolidine

The same procedure as shown in Example 7 was repeated using isobutyl aldehyde (34 mg) and (2S,4S)-1-[(2-amino-1,1-dimethyl)ethylamino]acetyl-2-cyano-4-fluoropyrrolidine dihydrochloride (150 mg) to give the titled compound (99 mg) as a colorless oil.
$^1$H-NMR (300 MHz, DMSO-d6) δ 5.60-5.28 (1H, m), 4.98-4.90 (1H, m), 3.93 (1H, dd, J=23.5, 12.4 Hz), 3.71 (1H, ddd, J=39.6, 12.4, 3.4 Hz), 3.41-3.16 (2H, m), 2.62-2.28 (6H), 1.64 (1H, m, J=6.7 Hz), 0.97 (6H, s), 0.85 (6H, d, J=6.7 Hz).

Example 49

Synthesis of (2S,4S)-2-cyano-1-[(2-diethylamino-1,1-dimethyl)ethylamino]acetyl-4-fluoropyrrolidine

The same procedure as shown in Example 7 was repeated using acetoaldehyde (23 mg) and (2S,4S)-1-[(2-amino-1,1-dimethyl)ethylamino]acetyl-2-cyano-4-fluoropyrrolidine dihydrochloride (150 mg) to give the titled compound (38 mg) as a colorless oil and (2S,4S)-2-cyano-4-fluoro-1-[(2-ethylamino-1,1-dimethyl)ethylamino]acetylpyrrolidine (23 mg) as a colorless oil.
$^1$H-NMR (300 MHz, DMSO-d6) δ 5.60-5.30 (1H, m), 4.99-4.90 (1H, m), 3.93 (1H, dd, J=23.3, 12.5 Hz), 3.71 (1H, ddd, J=39.6, 12.5, 3.4 Hz), 3.44-3.20 (2H, m), 2.62-2.30 (6H), 2.28-2.18 (2H, m), 1.01-0.85 (12H, m).

Example 50

Synthesis of (2S,4S)-2-cyano-1-[(2-dihexylamino-1,1-dimethyl)ethylamino]acetyl-4-fluoropyrrolidine

The same procedure as shown in Example 7 was repeated using hexanal (46 mg) and (2S,4S)-1-[(2-amino-1,1-dimethyl)ethylamino]acetyl-2-cyano-4-fluoropyrrolidine dihydrochloride (150 mg) to give the titled compound (75 mg) as a colorless oil.
$^1$H-NMR (300 MHz, DMSO-d6) δ 5.60-5.30 (1H, m), 4.99-4.90 (1H, m), 4.08-2.80 (6H), 2.60-2.20 (4H), 1.98-1.82 (2H, m), 1.50-1.10 (16H, m), 1.00-0.70 (10H).

Example 51

Synthesis of (2S,4S)-1-[[[2-bis(3,5,5-trimethylhexyl)amino]-1,1-dimethyl]ethylamino]acetyl-2-cyano-4-fluoropyrrolidine

The same procedure as shown in Example 7 was repeated using 3,5,5-trimethylhexanal (69 mg) and (2S,4S)-1-[(2-amino-1,1-dimethyl)ethylamino]acetyl-2-cyano-4-fluoropyrrolidine dihydrochloride (150 mg) to give the titled compound (106 mg) as a colorless oil.
$^1$H-NMR (300 MHz, DMSO-d6) δ 5.46 (1H, brd, J=50.8 Hz), 4.98-4.90 (1H, m), 3.92 (1H, dd, J=24.2, 12.7 Hz), 3.83-3.20 (5H), 2.62-2.25 (1H), 1.49-0.98 (14H, m), 0.97-0.82 (30H, m).

Example 52

Synthesis of (2S,4S)-1-[[2-(N-benzoyl-N-isobutyl)amino-1,1-dimethyl]ethylamino]acetyl-2-cyano-4-fluoropyrrolidine

The same procedure as shown in Example 3(4) was repeated using (2S,4S)-2-cyano-4-fluoro-1-[(2-isobutylamino-1,1-dimethyl)ethylamino]acetylpyrrolidine (67 mg) obtained in Example 48 and benzoyl chloride (32 mg) to give the titled compound (68 mg) as a colorless oil.
$^1$H-NMR (300 MHz, DMSO-d6) δ 7.50-7.28 (5H, m), 5.46 (1H, brd, J=51.1 Hz), 5.01-4.91 (1H, m), 4.06-3.18 (8H), 2.62-2.35 (2H, m), 1.90-1.78 (1H, m), 1.13-0.88 (6H, m), 0.86-0.54 (6H, m).

Example 53

Synthesis of (2S,4S)-1-[[2-(N-benzoyl-N-ethyl)amino-1,1-dimethyl]ethylamino]acetyl-2-cyano-4-fluoropyrrolidine

The same procedure as shown in Example 3(4) was repeated using (2S,4S)-2-cyano-4-fluoro-1-[(2-ethylamino-1,1-dimethyl)ethylamino]acetylpyrrolidine (23 mg) obtained in Example 49 and benzoyl chloride (12 mg) to give the titled compound (18 mg) as a light-yellow oil.
$^1$H-NMR (300 MHz, DMSO-d6) δ 7.47-7.28 (5H, m), 5.46 (1H, brd, J=51.1 Hz), 4.99-4.91 (1H, m), 4.06-3.22 (8H), 2.62-2.26 (2H, m), 1.20-0.74 (9H, m).

Example 54

Synthesis of (2S,4S)-1-[[2-(N-aminocarbonylmethyl-N-benzoyl)amino-1,1-dimethyl]ethylamino]acetyl-2-cyano-4-fluoropyrrolidine

(1) Synthesis of (2S,4S)-1-[[(2-aminocarbonylmethyl)amino-1,1-dimethyl]ethylamino]acetyl-2-cyano-4-fluoropyrrolidine

(2S,4S)-1-[(2-Amino-1,1-dimethyl)ethylamino]acetyl-2-cyano-4-fluoropyrrolidine dihydrochloride (150 mg) was dissolved in N,N-dimethylformamide (3.0 mL), followed by addition of triethylamine (199 μL) and 2-chloroacetamide (45 mg) under ice cooling. The resulting mixture was then warmed to room temperature and stirred for 3 days. The reaction mixture was concentrated under reduced pressure and the resulting residue was purified by silica gel column chromatography (developing solvent; chloroform:methanol:28% aqueous ammonia = 15:1:0.1 to 12:1:0.1) to give the titled

compound (41 mg) as a colorless oil.
$^1$H-NMR (300 MHz, DMSO-d6) δ 7.23 (1H, brs), 7.02 (1H, brs), 5.45 (1H, brd, J=53.3 Hz), 4.99-4.90 (1H, m), 3.94 (1H, dd, J=39.6, 12.7, 3.2 Hz), 3.73 (1H, ddd, J=39.6, 12.7, 3.2 Hz), 3.44-3.20 (2H), 3.04 (2H, s), 2.62-2.26 (4H), 1.03-0.91 (6H, m).

(2) Synthesis of (2S,4S)-1-[[2-(N-aminocarbonylmethyl-N-benzoyl)amino-1,1-dimethyl]ethylamino]acetyl-2-cyano-4-fluoropyrrolidine

The same procedure as shown in Example 3(4) was repeated using (2S,4S)-1-[[(2-aminocarbonylmethyl)amino-1,1-dimethyl]ethylamino]acetyl-2-cyano-4-fluoropyrrolidine (34 mg) and benzoyl chloride (13 mg) to give the titled compound (28 mg) as a light-yellow amorphous substance.
$^1$H-NMR (300 MHz, DMSO-d6) δ 7.47-7.25 (6H, m), 7.00 (1H, brs), 5.65-5.25 (1H, m), 5.08-4.91 (1H, m), 4.03-3.90 (2H, m), 3.86-2.90 (6H), 2.62-2.25 (2H, m), 1.28-1.05 (6H, m).

Example 55

Synthesis of (2S,4S)-2-cyano-1-[[2-(2,6-dimethylpiperidino)-1,1-dimethyl]ethylamino]acetyl-4-fluoropyrrolidine

(1) Synthesis of (2,6-dimethylpiperidino)acetonitrile

2,6-Dimethylpiperidine (1.00 g) was dissolved in N,N-dimethylformamide (15 mL), followed by addition of bromoacetonitrile (1.08 g) and sodium carbonate (983 mg) at room temperature. The resulting mixture was heated to 80°C and stirred for 3 hours, and then cooled to room temperature and stirred overnight. The reaction mixture was concentrated under reduced pressure and the resulting residue was purified by silica gel column chromatography (developing solvent; chloroform:methanol:28% aqueous ammonia = 50:1:0.1) to give the titled compound (791 mg) as a yellow oil.
$^1$H-NMR (300 MHz, $CDCl_3$) δ 3.79 (2H, s), 2.53-2.39 (2H, m), 1.74-1.62 (3H, m), 1.50-1.24 (3H, m), 1.12 (6H, d, J=6.2 Hz).

(2) Synthesis of 1-(2,6-dimethylpiperidino)-2-methyl-2-aminopropane

The same procedure as shown in Example 10(1) was repeated using (2,6-dimethylpiperidino)acetonitrile (781 mg) and methyllithium (1.2 M in diethyl ether, 12.8 mL) to give the titled compound (671 mg) as a yellow oil.
$^1$H-NMR (300 MHz, $CDCl_3$) δ 2.94-2.80 (2H, m), 2.34 (2H, s), 1.74-1.57 (3H, m), 1.51-1.33 (3H, m), 1.07 (3H, s), 1.05 (3H, s), 1.03 (6H, s).

(3) Synthesis of (2S,4S)-2-cyano-1-[[2-(2,6-dimethylpiperidino)-1,1-dimethyl]ethylamino]acetyl-4-fluoropyrrolidine

The same procedure as shown in Example 1(2) was repeated using 1-(2,6-dimethylpiperidino)-2-methyl-2-aminopropane (180 mg) and (2S,4S)-1-chloroacetyl-2-cyano-4-fluoropyrrolidine (84 mg) to give the titled compound (69 mg) as a colorless amorphous substance.
$^1$H-NMR (300 MHz, DMSO-d6) δ 5.50 (1H, brd, J=52.5 Hz), 5.08-5.00 (1H, m), 4.04 (1H, dd, J=23.9, 12.7 Hz), 3.94-3.56 (3H), 3.16-3.06 (2H, m), 2.90-2.74 (2H, m), 2.56-2.24 (2H, m), 1.73-1.30 (6H, m), 1.20-1.14 (12H, m).

Example 56

Synthesis of (2S,4S)-2-cyano-1-[[2-(2,5-dimethylpyrrolidin-1-yl)-1,1-dimethyl]ethylamino]acetyl-4-fluoropyrrolidine

(1) Synthesis of (2,5-dimethyl-1-pyrrolidinyl)acetonitrile

2,5-Dimethylpyrrolidine (491 mg) was dissolved in THF (5 mL), followed by addition of bromoacetonitrile (552 mg) and sodium carbonate (513 mg) at room temperature. The resulting mixture was stirred at room temperature for 1 hour and at 65°C for 1 hour, and then cooled to room temperature and stirred overnight. The reaction mixture was concentrated under reduced pressure and the resulting residue was purified by silica gel column chromatography (developing solvent; chloroform:methanol:28% aqueous ammonia = 50:1:0 to 50:1:0.1) to give the titled compound (357 mg) as a colorless oil.
$^1$H-NMR (300 MHz, $CDCl_3$) δ 3.62 (2H, s), 3.30-3.17 (2H, m), 2.79-2.63 (2H, m), 2.14-1.98 (2H, m), 1.10 (3H, s), 1.08 (3H, s).

(2) Synthesis of 1-(2,5-dimethyl-1-pyrrolidinyl)-2-methyl-2-aminopropane

The same procedure as shown in Example 10(1) was repeated using (2,5-dimethyl-1-pyrrolidinyl)acetonitrile (350 mg) and methyllithium (1.2 M in diethyl ether, 6.3 mL) to give the titled compound (100 mg) as a brown oil.
$^1$H-NMR (300 MHz, $CDCl_3$) δ 3.07-3.01 (2H, m), 2.64 (1H, d, J=14.1 Hz), 2.16 (1H, d, J=14.1 Hz), 2.05-1.89 (2H, m), 1.44-1.26 (2H, m), 1.07 (3H, s), 1.06 (3H, s), 0.99 (3H, s), 0.97 (3H, s).

(3) Synthesis of (2S,4S)-2-cyano-1-[[2-(2,5-dimethylpyrrolidin-1-yl)-1,1-dimethyl]ethylamino]acetyl-4-fluoropyrrolidine

The same procedure as shown in Example 1(2) was repeated using 1-(2,5-dimethyl-1-pyrrolidinyl)-2-methyl-2-aminopropane (88 mg) and (2S,4S)-1-chloroacetyl-2-cyano-4-fluoropyrrolidine (45 mg) to give the titled compound (22 mg) as a dark brown oil.
$^1$H-NMR (300 MHz, DMSO-d6) δ 5.60-5.30 (1H, m), 4.99-4.90 (1H, m), 4.83-3.85 (1H, m), 3.83-3.60 (1H, m), 3.45-3.20 (2H), 3.12-2.98 (2H, m), 2.64-2.54 (1H, dd, J=14.0, 5.8 Hz), 2.53-2.20 (2H), 2.25-2.16 (1H, dd, J=14.0, 5.8 Hz), 1.99-1.75 (3H, m), 1.40-1.20 (2H, m), 1.00-0.90 (12H, m).

Example 57

Synthesis of (2S,4S)-1-[[2-(benzothiazol-6-yl)carbonylamino-1,1-dimethyl]ethylamino]acetyl-2-cyano-4-fluoropyrrolidine

(1) Synthesis of 1-[2-(benzothiazol-6-yl)carbonylamino]-2-methyl-2-aminopropane

The same procedure as shown in Example 2(1) was repeated using benzothiazole-6-carboxylic acid (300 mg) and 1,2-diamino-2-dimethylpropane (295 mg) to give the titled compound (261 mg) as a yellow oil.
$^1$H-NMR (300 MHz, DMSO-d6) δ 9.52 (1H, s), 8.68 (1H, d, J=1.5 Hz), 8.46-8.34 (1H, m), 8.15 (1H, d, J=8.6 Hz), 8.01 (1H, dd, J=8.6, 1.5 Hz), 3.23 (2H, brd, J=5.9 Hz), 1.08-0.98 (6H, m).

(2) Synthesis of (2S,4S)-1-[[2-(benzothiazol-6-yl)carbonylamino-1,1-dimethyl]ethylamino]acetyl-2-cyano-4-fluoropyrrolidine

The same procedure as shown in Example 1(2) was repeated using 1-[2-(benzothiazol-6-yl)carbonylamino]-2-methyl-2-aminopropane (205 mg) and (2S,4S)-1-chloroacetyl-2-cyano-4-fluoropyrrolidine (71 mg) to give the titled compound (96 mg) as a light-yellow amorphous substance.
$^1$H-NMR (300 MHz, DMSO-d6) δ 9.53 (1H, s), 8.67 (1H, s), 8.44-8.34 (1H, m), 8.15 (1H, d, J=8.6 Hz), 8.00 (1H, d, J=8.6 Hz), 5.45 (1H, brd, J=51.6 Hz), 5.00-4.92 (1H, m), 3.96 (1H, dd, J=24.3, 12.8 Hz), 3.86-3.18 (5H), 2.62-2.25 (2H), 1.06 (6H, s).

Example 58

Synthesis of (2S,4S)-1-[[2-(N-benzoyl-N-hexyl)amino-1,1-dimethyl]ethylamino]acetyl-2-cyano-4-fluoropyrrolidine

(1) Synthesis of 1-hexylamino-2-methyl-2-aminopropane

The same procedure as shown in Example 7 was repeated using hexanal (1.13 g) and 1,2-diamino-2-methylpropane (1.00 g) to give the titled compound (1.16 g) as a colorless oil.
$^1$H-NMR (300 MHz, $CDCl_3$) δ 4.35-4.20 (4H, m), 2.69 (1H, t, J=7.5 Hz), 1.60-1.47 (2H, m), 1.38-1.24 (6H, m), 1.20 (6H, s), 0.94-0.84 (3H, m).

(2) Synthesis of (2S,4S)-2-cyano-4-fluoro-1-[(2-hexylamino-1,1-dimethyl)ethylamino]acetylpyrrolidine

The same procedure as shown in Example 1(2) was repeated using 1-hexylamino-2-methyl-2-aminopropane (232 mg) and (2S,4S)-1-chloroacetyl-2-cyano-4-fluoropyrrolidine (117 mg) to give the titled compound (110 mg) as a light-yellow amorphous substance.

(3) Synthesis of (2S,4S)-1-[[2-(N-benzoyl-N-hexyl)amino-1,1-dimethyl]ethylamino]acetyl-2-cyano-4-fluoropyrrolidine

The same procedure as shown in Example 3(4) was repeated using (2S,4S)-2-cyano-4-fluoro-1-[(2-hexylamino-1,1-dimethyl)ethylamino]acetylpyrrolidine (100 mg) and benzoyl chloride (43 mg) to give the titled compound (17 mg) as a colorless oil.
$^1$H-NMR (300 MHz, DMSO-d6) δ 7.47-7.28 (5H, m), 5.46 (1H, brd, J=51.5 Hz), 4.99-4.90 (1H, m), 3.97 (1H, dd, J=23.6, 12.5 Hz), 3.85-3.22 (5H), 2.62-2.25 (2H, m), 1.44-0.70 (19H).

Example 59

Synthesis of (2S,4S)-1-[[2-(benzimidazol-5-yl)carbonylamino-1,1-dimethyl]ethylamino]acetyl-2-cyano-4-fluoropyrrolidine

(1) Synthesis of benzimidazole-5-carbonyl chloride

5-Benzimidazolecarboxylic acid (80 mg) was suspended in benzene (1.5 mL). After addition of thionyl chloride (176 μL) under ice cooling, the resulting mixture was heated under reflux for 6 hours. The reaction mixture was concentrated under reduced pressure to give benzimidazole-5-carbonyl chloride (90 mg).

(2) Synthesis of (2S,4S)-1-[[2-(benzimidazol-5-yl)carbonylamino-1,1-dimethyl]ethylamino]acetyl-2-cyano-4-fluoropyrrolidine

The same procedure as shown in Example 3(4) was repeated using (2S,4S)-1-[(2-amino-1,1-dimethyl)ethylamino]acetyl-2-cyano-4-fluoropyrrolidine dihydrochloride (50 mg) and benzimidazole-5-carbonyl chloride (27 mg) to give the titled compound (48 mg) as a yellow amorphous substance.
$^1$H-NMR (300 MHz, DMSO-d6) δ 12.80-12.50 (1H, m), 8.38-8.18 (3H, m), 7.82-7.70 (1H, m), 5.47 (1H, brd, J=51.8 Hz), 5.04-4.90 (1H, m), 4.10-3.18 (6H), 2.62-2.25 (2H), 1.06 (6H, s).

Example 60

Synthesis of (2S,4S)-1-[[2-(1H-1,2,3-benzotriazol-5-yl)carbonylamino-1,1-dimethyl]ethylamino]acetyl-2-cyano-4-fluoropyrrolidine

1H-1,2,3-Benzotriazole-5-carboxylic acid (57 mg) was dissolved in N,N-dimethylformamide (1.8 mL). To this solution, N,N'-carbonyldiimidazole (62 mg) was added at room temperature and stirred for 3 hours, followed by addition of (2S,4S)-1-[(2-amino-1,1-dimethyl)ethylamino]acetyl-2-cyano-4-fluoropyrrolidine dihydrochloride (100 mg). Triethylamine (133 μL) was then added under ice cooling and the reaction mixture was stirred for 10 minutes, warmed to room temperature and stirred overnight. The reaction mixture was concentrated under reduced pressure and the resulting residue was purified by silica gel column chromatography (developing solvent; chloroform:methanol:28% aqueous ammonia = 15:1:0.1 to 12:1:0.1 to 10:1:0.1) to give the titled compound (61 mg) as a light-yellow powder.
$^1$H-NMR (300 MHz, DMSO-d6) δ 8.49-8.38 (2H, m), 7.99-7.82 (2H, m), 5.46 (1H, brd, J=51.0 Hz), 5.03-4.90 (1H, m), 3.97 (1H, dd, J=24.2, 12.7 Hz), 3.86-3.00 (5H), 2.64-2.25 (2H, m), 1.07 (6H, s).

Example 61

Synthesis of (2S,4S)-2-cyano-1-[[2-(2,3-dihydrobenzo[b]furan-5-yl)carbonylamino-1,1-dimethyl]ethylamino]acetyl-4-fluoropyrrolidine

The same procedure as shown in Example 60 was repeated using (2S,4S)-1-[(2-amino-1,1-dimethyl)ethylamino]acetyl-2-cyano-4-fluoropyrrolidine dihydrochloride (97 mg) and 2,3-dihydrobenzo[b]furan-5-carboxylic acid (56 mg) to give the titled compound (94 mg) as a colorless gum.
$^1$H-NMR (300 MHz, DMSO-d6) δ 8.07-7.95 (1H, m), 7.75-7.73 (1H, m), 7.69-7.61 (1H, m), 6.80 (1H, d, J=8.4 Hz), 5.46 (1H, brd, J=51.4 Hz), 4.99-4.92 (1H, m), 4.59 (2H, t, J=8.8 Hz), 3.96 (1H, dd, J=23.5, 12.6 Hz), 3.74 (1H, ddd, J=39.8, 12.5, 3.3 Hz), 3.52-3.14 (6H, m), 2.62-2.26 (2H, m), 2.10-1.80 (1H, m), 1.01 (3H, s), 1.00 (3H, s).

Example 62

Synthesis of (2S,4S)-2-cyano-4-fluoro-1-[[2-(thiophen-3-yl)carbonylamino-1,1-dimethyl]ethylamino]acetylpyrrolidine

The same procedure as shown in Example 60 was repeated using (2S,4S)-1-[(2-amino-1,1-dimethyl)ethylamino]acetyl-2-cyano-4-fluoropyrrolidine dihydrochloride (134 mg) and 3-thiophenecarboxylic acid (60 mg) to give the titled

compound (72 mg) as a colorless foam.
$^1$H-NMR (300 MHz, DMSO-d6) δ 8.15 (1H, dd, J=3.0, 1.3 Hz), 8.11-8.02 (1H, m), 7.58 (1H, dd, J=5.0, 3.0 Hz), 7.51 (1H, dd, J=5.0, 1.3 Hz), 5.46 (1H, brd, J=50.2 Hz), 5.00-4.93 (1H, m), 3.96 (1H, dd, J=24.5, 11.9 Hz), 3.73 (1H, ddd, J=39.6, 12.6, 3.3 Hz), 3.52-3.25 (2H, m), 3.26-3.12 (2H, m), 2.62-2.25 (2H, m), 2.00-1.85 (1H, brs), 1.02 (3H, s), 1.01 (3H, s).

Example 63

Synthesis of (2S,4S)-2-cyano-4-fluoro-1-[[2-(5-methylthiophen-2-yl)carbonylamino-1,1-dimethyl]ethylamino]acetylpyrrolidine

The same procedure as shown in Example 60 was repeated using (2S,4S)-1-[(2-amino-1,1-dimethyl)ethylamino]acetyl-2-cyano-4-fluoropyrrolidine dihydrochloride (129 mg) and 5-methyl-2-thiophenecarboxylic acid (64 mg) to give the titled compound (92 mg) as a colorless foam.
$^1$H-NMR (300 MHz, DMSO-d6) δ 8.17-8.06 (1H, m), 7.60 (1H, d, J=3.7 Hz), 6.84 (1H, dd, J=3.7, 1.0 Hz), 5.44 (1H, brd, J=51.6 Hz), 5.00-4.92 (1H, m), 3.94 (1H, dd, J=23.6, 12.6 Hz), 3.73 (1H, ddd, J=39.5, 12.6, 3.3 Hz), 3.50-3.26 (2H, m), 3.25-3.08 (2H, m), 2.62-2.22 (2H, m), 2.48-2.44 (3H, m), 1.98-1.84 (1H, brs), 1.00 (3H, s), 0.99 (3H, s)

Example 64

Synthesis of (2S,4S)-2-cyano-4-fluoro-1-[[2-(3-methylthiophen-2-yl)carbonylamino-1,1-dimethyl]ethylamino]acetylpyrrolidine

The same procedure as shown in Example 60 was repeated using (2S,4S)-1-[(2-amino-1,1-dimethyl)ethylamino]acetyl-2-cyano-4-fluoropyrrolidine dihydrochloride (129 mg) and 3-methyl-2-thiophenecarboxylic acid (64 mg) to give the titled compound (74 mg) as a colorless foam.
$^1$H-NMR (300 MHz, DMSO-d6) δ 7.75-7.66 (1H, m), 7.56 (1H, d, J=5.0 Hz), 6.96 (1H, d, J=5.0 Hz), 5.46 (1H, brd, J=53.2 Hz), 5.00-4.92 (1H, m), 3.94 (1H, dd, J=24.2, 11.9 Hz), 3.83-3.61 (1H, m), 3.50-3.25 (2H, m), 3.24-3.10 (2H, m), 2.62-2.25 (2H, m), 2.43 (3H, s), 2.10-1.86 (1H, brs), 1.03 (3H, s), 1.02 (3H, s)

Example 65

Synthesis of (2S,4S)-2-cyano-4-fluoro-1-[[2-(3-hydroxy-2-methylpropan-2-yl)carbonylamino-1,1-dimethyl]ethylamino] acetylpyrrolidine

Hydroxypivaloylic acid (41 mg) and (2S,4S)-1-[(2-amino-1,1-dimethyl)ethylamino]acetyl-2-cyano-4-fluoropyrrolidine dihydrochloride (110 mg) were dissolved in N,N-dimethylformamide (1.1 mL), followed by addition of 1-hydroxybenzotriazole monohydrate (54 mg) at room temperature. After dropwise addition of diisopropylethylamine (128 μL) under ice cooling, 1-ethyl-3-(3'-dimethylaminopropyl)carbodiimide hydrochloride (76 mg) was added and stirred for 10 minutes. After warming to room temperature and stirring overnight, the reaction mixture was concentrated under reduced pressure and the resulting residue was purified by silica gel column chromatography (developing solvent; chloroform: methanol:28% aqueous ammonia = 20:1:0 to 15:1:0.1). The resulting colorless solid was suspended in isopropyl ether and stirred to give the titled compound (60 mg) as a colorless powder.
$^1$H-NMR (300 MHz, DMSO-d6) δ 7.60-7.40 (1H, m), 5.48 (1H, brd, J=50.4 Hz), 5.05-4.96 (1H, m), 4.00 (1H, dd, J=24.4, 12.7 Hz), 3.86-3.47 (3H, m), 3.44-3.24 (2H), 3.22-3.06 (2H, m), 2.56-2.25 (2H, m), 1.18-0.95 (12H, m).

Example 66

Synthesis of (2S,4S)-2-cyano-4-fluoro-1-[[2-(1,3-dihydroxy-2-methylpropan-2-yl)carbonylamino-1,1-dimethyl]ethylamino]acetylpyrrolidine

The same procedure as shown in Example 65 was repeated using (2S,4S)-1-[(2-amino-1,1-dimethyl)ethylamino]acetyl-2-cyano-4-fluoropyrrolidine dihydrochloride (106 mg) and 2,2-bis(hydroxymethyl)propionic acid (45 mg) to give the titled compound (55 mg) as a colorless gum.
$^1$H-NMR (300 MHz, DMSO-d6) δ 7.52-7.40 (1H, m), 5.44 (1H, brd, J=50.7 Hz), 4.98-4.91 (1H, m), 4.86-4.70 (2H, brs), 3.92 (1H, dd, J=23.8, 12.6 Hz), 3.73 (1H, ddd, J=39.8, 12.6, 3.3 Hz), 3.52-3.21 (6H, m), 3.08-2.97 (2H, m), 2.62-2.26 (2H, m), 0.99 (3H, s), 0.96 (6H, s).

Example 67

Synthesis of (2S,4S)-2-cyano-4-fluoro-1-[[2-(cis-4-hydroxycyclohexan-1-yl)carbonylamino-1,1-dimethyl]ethylamino] acetylpyrrolidine

The same procedure as shown in Example 65 was repeated using (2S,4S)-1-[(2-amino-1,1-dimethyl)ethylamino]acetyl-2-cyano-4-fluoropyrrolidine dihydrochloride (128 mg) and cis-4-hydroxycyclohexanecarboxylic acid (59 mg) to give the titled compound (65 mg) as a light-yellow solid.
$^1$H-NMR (300 MHz, DMSO-d6) δ 7.51-7.41 (1H, m), 5.46 (1H, brd, J=53.4 Hz), 4.98-4.91 (1H, m), 4.27 (1H, d, J=3.3 Hz), 3.93 (1H, dd, J=23.4, 12.4 Hz), 3.82-3.61 (1H, m), 3.44-3.21 (2H, m), 3.08-2.91 (2H, m), 2.62-2.26 (2H, m), 2.24-2.08 (1H, m), 1.86-1.69 (2H, m), 1.68-1.55 (2H, m), 1.48-1.32 (4H, m), 0.95 (3H, s), 0.94 (3H, s).

Example 68

Synthesis of (2S,4S)-2-cyano-4-fluoro-1-[[2-(1-methylcyclohexan-1-yl)carbonylamino-1,1-dimethyl]ethylamino] acetylpyrrolidine

The same procedure as shown in Example 65 was repeated using (2S,4S)-1-[(2-amino-1,1-dimethyl)ethylamino]acetyl-2-cyano-4-fluoropyrrolidine dihydrochloride (129 mg) and 1-methyl-1-cyclohexanecarboxylic acid (58 mg) to give the titled compound (74 mg) as a light-yellow oil.
$^1$H-NMR (300 MHz, DMSO-d6) δ 7.29-7.19 (1H, m), 5.43 (1H, brd, J=51.5 Hz), 4.99-4.90 (1H, m), 3.94 (1H, dd, J=24.0, 11.6 Hz), 3.82-3.61 (1H, m), 3.48-3.22 (2H, m), 3.10-2.95 (2H, m), 2.62-2.26 (2H, m), 2.04-1.75 (3H, m), 1.54-1.10 (8H, m), 1.05 (3H, s), 0.96 (3H, s), 0.95 (3H, s).

Example 69

Synthesis of (2S,4S)-1-[[2-(1-methylcyclopropan-1-yl)carbonylamino-1,1-dimethyl]ethylamino]acetyl-2-cyano-4-fluoropyrrolidine

The same procedure as shown in Example 65 was repeated using (2S,4S)-1-[(2-amino-1,1-dimethyl)ethylamino]acetyl-2-cyano-4-fluoropyrrolidine dihydrochloride (146 mg) and 1-methylcyclopropylcarboxylic acid (46 mg) to give the titled compound (51 mg) as a colorless oil.
$^1$H-NMR (300 MHz, DMSO-d6) δ 7.30-7.16 (1H, m), 5.62-5.29 (1H, m), 5.00-4.92 (1H, m), 3.90 (1H, dd, J=24.8, 12.4 Hz), 3.82-3.61 (1H, m), 3.47-3.22 (2H, m), 3.05-2.96 (2H, m), 2.62-2.25 (2H, m), 2.05-1.80 (1H, brs), 1.29 (3H, s), 1.03-0.88 (8H, m), 0.50 (2H, dd, J=6.1, 3.2 Hz).

Example 70

Synthesis of (2S,4S)-1-[[2-[bis(4-chlorophenyl)]acetylamino-1,1-dimethyl]ethylamino]acetyl-2-cyano-4-fluoropyrrolidine

(1) Synthesis of 1-[2-[bis(4-chlorophenyl)]acetylamino]-2-methyl-2-aminopropane

Bis(4-chlorophenyl)acetic acid (320 mg) was dissolved in N,N-dimethylformamide (3.0 mL). To this solution, 1,2-diamino-2-dimethylpropane (100 mg), 1-hydroxybenzotriazole monohydrate (219 mg) and 1-ethyl-3-(3'-dimethylaminopropyl)carbodiimide hydrochloride (301 mg) were added under ice cooling and stirred for 30 minutes. After the reaction mixture was warmed to room temperature and stirred overnight, a 1:1 mixture (20 mL) of 5% aqueous sodium bicarbonate and saturated aqueous sodium chloride was added thereto under ice cooling, followed by extraction with chloroform (30 mL). The organic phase was dried over anhydrous magnesium sulfate and suction-filtered to remove the desiccant, and the filtrate was concentrated under reduced pressure. The resulting residue was purified by silica gel column chromatography (developing solvent; chloroform:methanol:28% aqueous ammonia = 20:1:0.1) to give the titled compound (283 mg) as a light-yellow solid.
$^1$H-NMR (300 MHz, DMSO-d6) δ 8.24-8.13 (1H, m), 7.35 (8H, dd, J=21.5, 8.6 Hz), 5.10 (1H, s), 2.97 (2H, d, J=5.8 Hz), 0.89 (6H, s).

(2) Synthesis of (2S,4S)-1-[[2-[bis(4-chlorophenyl)]acetylamino-1,1-dimethyl]ethylamino]acetyl-2-cyano-4-fluoropyrrolidine

The same procedure as shown in Example 1(2) was repeated using 1-[2-[bis(4-chlorophenyl)]acetylamino]-2-methyl-2-aminopropane (229 mg) and (2S,4S)-1-chloroacetyl-2-cyano-4-fluoropyrrolidine (57 mg) to give the titled compound (119 mg) as a light-yellow foam.
$^1$H-NMR (300 MHz, DMSO-d6) δ 8.22-8.13 (1H, m), 7.41-7.27 (8H, m), 5.45 (1H, brd, J=53.2 Hz), 5.10 (1H, s), 4.97-4.90 (1H, m), 3.90 (1H, dd, J=24.2, 12.0 Hz), 3.78-3.58 (1H, m), 3.48-3.20 (2H, m), 3.15-2.90 (2H, m), 2.60-2.25 (2H, m), 0.94 (6H, s).

Example 71

Synthesis of (2S,4S)-1-[[2-(2-hydroxybenzoyl)amino-1,1-dimethyl]ethylamino]acetyl-2-cyano-4-fluoropyrrolidine

(2S,4S)-1-[(2-Amino-1,1-dimethyl)ethylamino]acetyl-2-cyano-4-fluoropyrrolidine dihydrochloride (195 mg) was dissolved in methanol (0.2 mL). To this solution, a 3 M methanol solution of potassium hydroxide (412 μL) was added dropwise under ice cooling. The precipitated potassium chloride was filtered off and the filtrate was concentrated under reduced pressure. The resulting residue was dissolved in N,N-dimethylformamide (0.8 mL) and added dropwise to a solution of O-acetylsalicyloyl chloride (123 mg) in chloroform (0.8 mL) under ice cooling. Triethylamine (130 μL) was then added dropwise and stirred under ice cooling for 10 minutes. After warming to room temperature and stirring for 3 hours, the reaction mixture was supplemented with a mixture of saturated aqueous sodium bicarbonate (1.0 mL) and methanol (1.5 mL) and allowed to stand overnight in a freezer. Under ice cooling, saturated aqueous sodium chloride (20 mL) was added to the reaction mixture, which was then extracted with chloroform (30 mL). The organic phase was dried over anhydrous magnesium sulfate and suction-filtered to remove the desiccant, and the filtrate was concentrated under reduced pressure. The resulting residue was purified by silica gel column chromatography (developing solvent; chloroform:methanol:28% aqueous ammonia = 20:1:0.1) to give the titled compound (36 mg) as a colorless foam.
$^1$H-NMR (300 MHz, DMSO-d6) δ 8.75-8.60 (1H, m), 7.89 (1H, dd, J=8.1, 1.7 Hz), 7.38 (1H, td, J=7.7, 1.7 Hz), 6.94-6.86 (2H, m), 5.46 (1H, brd, J=51.5 Hz), 4.99-4.92 (1H, m), 3.95 (1H, dd, J=24.3, 12.4 Hz), 3.74 (1H, ddd, J=39.5, 12.4, 3.2 Hz), 3.53-3.19 (4H), 2.62-2.25 (2H, m), 1.04 (6H, s).
Table 3 below summarizes the structures of the compounds obtained in the above Examples 48 to 71.

Table 3

| Example No. | Structural formula |
|---|---|
| **48** | 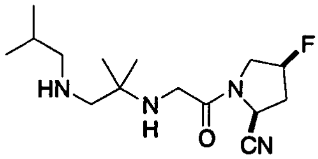 |
| **49** | 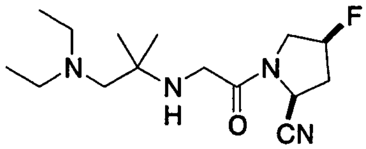 |
| **50** | 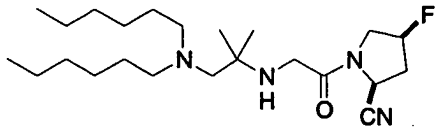 |
| **51** | 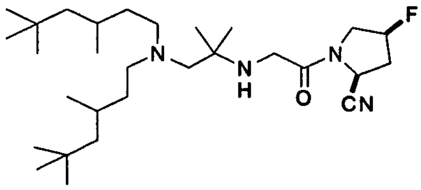 |
| **52** | 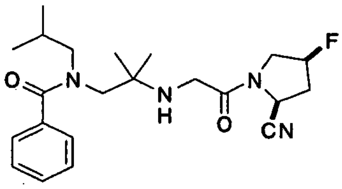 |
| **53** | 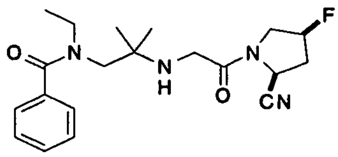 |
| **54** | 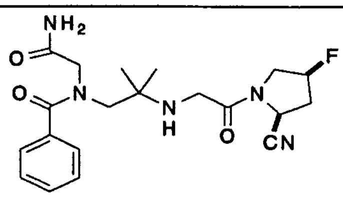 |

| | |
|---|---|
| 55 | 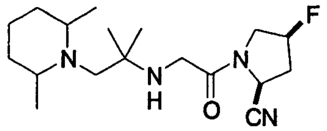 |
| 56 | 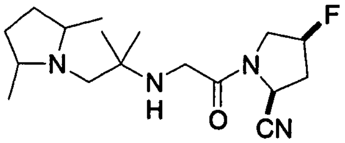 |
| 57 | 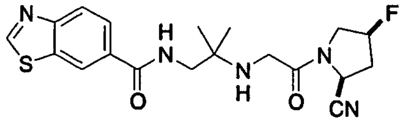 |
| 58 | 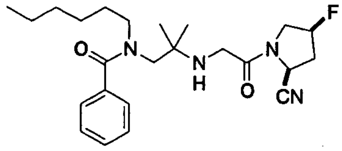 |
| 59 | 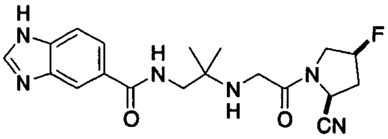 |
| 60 | 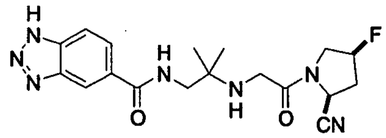 |
| 61 | 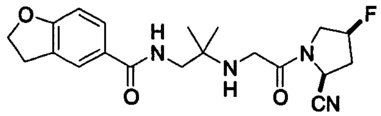 |
| 62 | 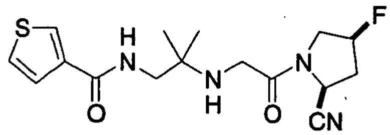 |
| 63 | 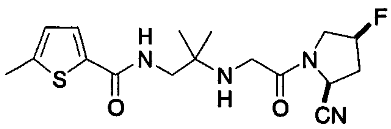 |
| 64 | 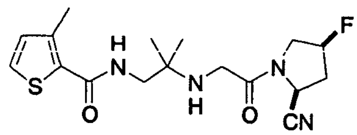 |
| 65 | 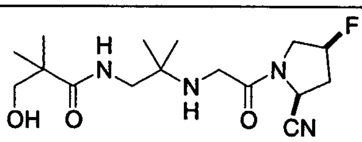 |
| 66 | 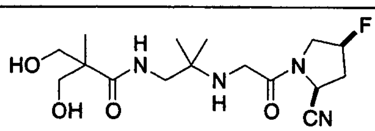 |

| | |
|---|---|
| 67 | 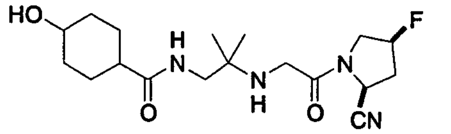 |
| 68 | 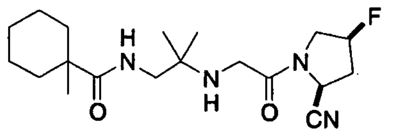 |
| 69 | 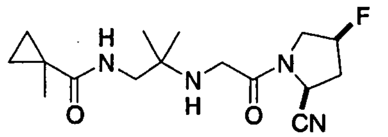 |
| 70 | 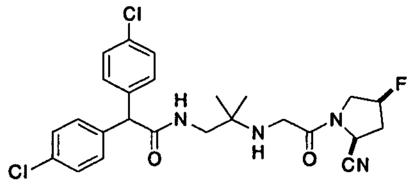 |
| 71 | 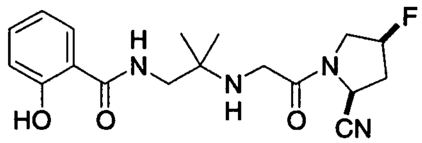 |

Test Example 1 [Dipeptidyl peptidase IV activity inhibition test]

The inhibition test of dipeptidyl peptidase IV (DPPIV) activity was performed as described in Diabetes, 47, 764-769, 1998. Plasma containing dipeptidyl peptidase IV was prepared from the blood of healthy volunteers by centrifugation. The enzymatic reaction was performed using a 96-well flat-bottomed plate in a buffer composed of 25 mM HEPES, 140 mM NaCl and 1% BSA, pH 7.8. A 100 μM solution of Gly-Pro-4-methylcoumaryl-7-amide (25 μl, a product of Peptide Institute, Inc., Japan), a 133 mM solution of magnesium chloride (7.5 μl) and a test compound (5 μl) were mixed and then supplemented with the plasma (12.5 μl) which had been diluted to 1/100 with the above buffer. After reaction at room temperature for 2 hours, a 25% aqueous acetic acid solution (50 μl) was added to stop the reaction. The amount of the liberated 7-amino-4-methylcoumarin was measured as fluorescence intensity at 460 nm using a fluorescence plate reader (1420 ARVO™ Multilabel Counter; Wallac) with an excitation wavelength of 390 nm. The fluorescence intensity obtained for vehicle addition (reaction time: 0 min) was used as a blank value and subtracted from each measured value to obtain the specific fluorescence intensity. The resulting specific fluorescence intensity was used to calculate the % inhibition of dipeptidyl peptidase IV activity according to the following equation. Each test compound was prepared as a 1000-fold concentrated solution in dimethyl sulfoxide and diluted with the above buffer before use. A concentration required for each test compound to produce 50% inhibition ($IC_{50}$) was calculated from % inhibition data at the respective concentrations.

$$\text{Inhibition (\%)} = A/B \times 100$$

where

A = (fluorescence intensity in the presence of vehicle)-(fluorescence intensity in the presence of test compound)
B = fluorescence intensity in the presence of vehicle
Compound of Example No. 37 $IC_{50}$ 5.4 nM
Compound of Example No. 60 $IC_{50}$ 1.5 nM

The compounds of the present invention were confirmed to have an excellent DPPIV inhibition activity. Test Example 2 [Measurement test on DPPIV activity in rat plasma when administered orally]

Male SD(IGS) rats at 8 weeks of age (Charles River Japan, Inc.) were used for this test. A test compound was dissolved at a concentration of 0.2 mg/mL with water for injection under Japanese Pharmacopoeia (Hikari Pharmaceutical Co., Ltd., Japan) and administered orally in a volume of 5 mL/kg (at a dose of 1 mg/kg). Blood samples were collected over time from the orbital vein using heparin-treated blood collection tubes (Drummond Scientific Company) under diethyl ether anesthesia before and after administration of the test compound. Each blood sample was centrifuged at 3,000 rpm for 15 minutes at 4°C to collect a plasma fraction. The resulting plasma fractions were stored at -80°C. It should be noted that the rats were fasted from 16 hours before and until 6 hours after administration of the test compound, but allowed to drink sterile water without any restriction.

DPPIV activity in plasma was determined as follows.

The enzymatic reaction was performed using a 96-well plate in a buffer composed of 25 mM HEPES, 140 mM NaCl and 1% BSA, pH 7.8. As a substrate solution, 10 mM H-Gly-Pro-4-methylcoumaryl-7-amide (BACHEM) was diluted to 1/100 with the buffer. A reaction solution was prepared in advance to have the following composition per well: 100 μM substrate solution (25 μL), buffer (5 μL) and 133 mM magnesium chloride (7.5 μL).

The plasma samples were dispensed at 12.5 μL per well and supplemented with the reaction solution (37.5 μL) to start the enzymatic reaction. After reaction at room temperature for 5 minutes, a 25% aqueous acetic acid solution (50 μL) was added to stop the reaction. The amount of the liberated 7-amino-4-methylcoumarin was measured as fluorescence intensity at 460 nm using a fluorescence plate reader (1420 ARVO™ Multilabel Counter; Wallac) with an excitation wavelength of 390 nm. The rat plasma collected before administration of the test compound (pooled from all rats) was pre-treated with a 25% aqueous acetic acid solution to deactivate the enzyme activity and then supplemented with the reaction solution, followed by determining the fluorescence intensity. The fluorescence intensity thus determined was used as a blank value and subtracted from each measured value to obtain the specific fluorescence intensity. The amount of the produced 7-amino-4-methylcoumarin (nmol, hereinafter referred to as AMC) was calculated from a calibration curve prepared with standards. Assuming that the amount of AMC production before administration of the test compound was set to 100%, DPPIV activity was expressed as follows.

$$\text{DPPIV activity (\% of Control)} = A/B \times 100$$

where

A = amount of AMC production after administration of test compound
B = amount of AMC production before administration of test compound

The results obtained are shown in Table A. The compound of the present invention was confirmed to ensure prolonged inhibition of DPPIV activity when administered orally to rats at a dose of 1 mg/kg.

Table A DPPIV activity (%) in plasma

| | 1 hr after administration | 4 hrs after administration | 8 hrs after administration |
|---|---|---|---|
| Compound of Example No. 37 | 12 | 17 | 22 |

INDUSTRIAL APPLICABILITY

The present invention enables the provision of a compound having an excellent dipeptidyl peptidase IV (DPPIV) inhibition activity. Moreover, the compound of the present invention is also advantageous in having prolonged DPPIV inhibition activity. The compound of the present invention is useful as an agent for preventing or treating diabetes mellitus, immune diseases, etc.

**Claims**

1. A cyanofluoropyrrolidine compound of the following Formula (I) or a pharmaceutically acceptable salt thereof or a hydrate thereof:

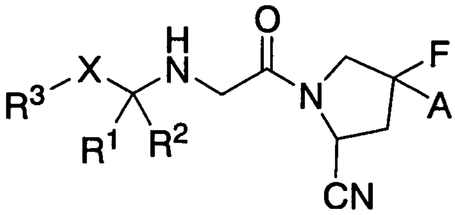

(I)

[wherein

A represents a hydrogen atom or a fluorine atom,

$R^1$ and $R^2$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; a $C_{2-6}$ alkenyl group which may be substituted with one or more substituents selected from the substituent $Y^2$ group; a $C_{3-6}$ cycloalkenyl group which may be substituted with one or more substituents selected from the substituent $Y^2$ group; or a $C_{4-9}$ cycloalkenylalkyl group which may be substituted with one or more substituents selected from the substituent $Y^2$ group; or

$R^1$ and $R^2$ may form, together with the adjacent carbon atom, a $C_{3-10}$ cycloalkyl group which may be substituted with one or more substituents selected from the substituent $Y^2$ group,

X represents a single bond or a $C_{1-3}$ alkylene group,

$R^3$ represents a group represented by the formula: $-N(R^4)COR^5$, $-N(R^4)SO_2R^5$, $-NR^4R^6$, $-SO_2R^5$, $-SO_2NR^4R^5$, $-OCONR^4R^5$, $-CH=CH-R^7$ or $-C\equiv C-R^7$, or represents a heteroaryl group selected from a heteroaryl group which contains at least one oxygen and/or sulfur atom and which may further contain a nitrogen atom, and a 6-membered nitrogen-containing aromatic ring or a 9- to 11-membered condensed ring thereof (wherein the heteroaryl group may be substituted with one or more substituents selected from the substituent $Y^3$ group)

(wherein $R^4$ and $R^6$, which may be the same or different, each represent a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or an arylalkyl group which may be substituted with one or more substituents selected from the substituent $Y^3$ group,

$R^5$ represents a $C_{1-10}$ alkyl group which may be substituted with one or more substituents selected from the substituent $Y^4$ group, or $-(C_{1-3}$ alkylene$)-Q$ or Q, wherein $C_{1-3}$ alkylene may be substituted with one or more substituents selected from a halogen atom and a hydroxyl group, and Q represents an aliphatic or aromatic hydrocarbon selected from a $C_{3-10}$ cycloalkyl group which may be substituted with one or more substituents selected from the substituent $Y^3$ group; a $C_{4-10}$ bridged cyclic alkyl group which may be substituted with one or more substituents selected from the substituent $Y^3$ group; a $C_{2-10}$ alkenyl group which may be substituted with one or more substituents selected from the substituent $Y^3$ group; a $C_{3-10}$ cycloalkenyl group which may be substituted with one or more substituents selected from the substituent $Y^3$ group; a $C_{4-10}$ bridged cyclic alkenyl group which may be substituted with one or more substituents selected from the substituent $Y^3$ group; and an aryl group which may be substituted with one or more substituents selected from the substituent $Y^3$ group; or alternatively, Q represents a heterocyclic ring which may be substituted with one or more substituents selected from the substituent $Y^5$ group, wherein in the aryl group or heterocyclic ring in $R^5$, adjacent substituents attached to the ring member atoms may together form a 5- to 8-membered ring which may contain one or more heteroatoms in its ring,

in $R^4$, $R^5$ or $R^6$, $R^4$ and $R^5$, $R^4$ and $R^6$, as well as $R^5$ and $R^6$ may form, together with the adjacent heteroatom(s), a 4-to 10-membered heterocyclic ring which may be substituted with one or more substituents selected from the substituent $Y^5$ group, and

$R^7$ represents a hydrogen atom; a $C_{1-6}$ alkyl group which may be substituted with one or more substituents selected from the substituent $Y^1$ group; an aryl group which may be substituted with one or more substituents selected from the substituent $Y^3$ group; or a heteroaryl group which may be substituted with one or more substituents selected from the substituent $Y^3$ group),

the substituent $Y^1$ group represents a group consisting of a halogen atom, a hydroxyl group, a carboxyl group, a cyano group, an amino group, an aminocarbonyl group, a $C_{3-5}$ cycloalkyloxy group and a $C_{1-6}$ alkoxy group,

the substituent $Y^2$ group represents a group consisting of a halogen atom, a hydroxyl group, a carboxyl group, a

cyano group, an amino group, an aminocarbonyl group, a $C_{3-5}$ cycloalkyloxy group, a $C_{1-6}$ alkoxy group and a $C_{1-6}$ alkyl group,
the substituent $Y^3$ group represents a group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or a phenyl group which may be substituted with one or more substituents selected from the substituent $Y^2$ group), as well as a $C_{1-6}$ alkyl group which may be substituted with one or more substituents selected from the substituent $Y^1$ group and a phenyl group which may be substituted with one or more substituents selected from the substituent $Y^2$ group,
the substituent $Y^4$ group represents a group consisting of a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or a phenyl group which may be substituted with one or more substituents selected from the substituent $Y^2$ group), as well as a phenyl group which may be substituted with one or more substituents selected from the substituent $Y^2$ group, and
the substituent $Y^5$ group represents a group consisting of an oxo group, a halogen atom, a hydroxyl group, a cyano group, a nitro group, an amino group, $-OR^9$, $-COR^9$, $-CO_2R^9$, $-CONR^9R^{10}$, $-N(R^9)COR^{10}$, $-N(R^9)CONR^{10}R^{11}$, $-N(R^9)SO_2R^{10}$, $-NR^9R^{10}$, $-SO_2R^9$, $-SO_2NR^9R^{10}$, $-SO_2N{=}CHNR^9R^{10}$ and $-OCONR^9R^{10}$ (wherein $R^9$, $R^{10}$ and $R^{11}$, which may be the same or different, each represent a hydrogen atom; a $C_{1-6}$ alkyl group which may be substituted with one or more substituents selected from the substituent $Y^1$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or a phenyl group which may be substituted with one or more substituents selected from the substituent $Y^2$ group), as well as a $C_{1-6}$ alkyl group which may be substituted with one or more substituents selected from the substituent $Y^1$ group and a phenyl group which may be substituted with one or more substituents selected from the substituent $Y^2$ group].

2. The compound according to claim 1 or a salt thereof or a hydrate thereof, which is represented by Formula (I-2):

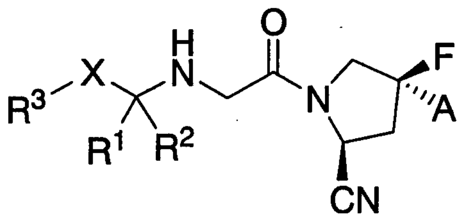

(I-2)

(wherein A, $R^1$, $R^2$, $R^3$ and X are as defined in claim 1).

3. The compound according to claim 1 or 2 or a salt thereof or a hydrate thereof, wherein $R^1$ and $R^2$, which may be the same or different, each represent a $C_{1-6}$ alkyl group which may be substituted with one or more substituents selected from the substituent $Y^1$ group.

4. The compound according to any one of claims 1 to 3 or a salt thereof or a hydrate thereof, wherein $R^1$ and $R^2$ are each a methyl group, an ethyl group or a hydroxymethyl group.

5. The compound according to any one of claims 1 to 4 or a salt thereof or a hydrate thereof, wherein
X is a methylene group or an ethylene group,
$R^3$ is a group represented by the formula $-N(R^4)COR^5$,
$R^4$ represents a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more

substituents selected from the substituent $Y^2$ group,
$R^5$ is a $C_{1-10}$ alkyl group which may be substituted with one or more substituents selected from the substituent $Y^4$ group, or -($C_{1-3}$ alkylene)-Q or Q, wherein $C_{1-3}$ alkylene may be substituted with one or more substituents selected from a halogen atom and a hydroxyl group, and Q is an aliphatic or aromatic hydrocarbon selected from a $C_{3-10}$ cycloalkyl group which may be substituted with one or more substituents selected from the substituent $Y^3$ group; a $C_{4-10}$ bridged cyclic alkyl group which may be substituted with one or more substituents selected from the substituent $Y^3$ group; a $C_{2-10}$ alkenyl group which may be substituted with one or more substituents selected from the substituent $Y^3$ group; a $C_{3-10}$ cycloalkenyl group which may be substituted with one or more substituents selected from the substituent $Y^3$ group; a $C_{4-10}$ bridged cyclic alkenyl group which may be substituted with one or more substituents selected from the substituent $Y^3$ group; and an aryl group which may be substituted with one or more substituents selected from the substituent $Y^3$ group,
in the aryl group in $R^5$, adjacent substituents attached to the ring member atoms may together form a 5- to 8-membered ring which may contain one or more heteroatoms in its ring, and
$R^4$ and $R^5$ may form, together with the adjacent heteroatom(s), a 4- to 10-membered heterocyclic ring which may be substituted with one or more substituents selected from the substituent $Y^5$ group.

6. The compound according to claim 5 or a salt thereof or a hydrate thereof, wherein $R^5$ is a $C_{1-6}$ alkyl group which may be substituted with one or more substituents selected from the substituent $Y^1$ group, or a $C_{3-6}$ cycloalkyl group which may be substituted with one or more substituents selected from the substituent $Y^2$ group.

7. The compound according to claim 5 or a salt thereof or a hydrate thereof, wherein $R^5$ is an aryl group which may be substituted with one or more substituents selected from the substituent $Y^3$ group, wherein in the aryl group, adjacent substituents attached to the ring member atoms may together form a 5- to 8-membered ring which may contain one or more heteroatoms in its ring.

8. The compound according to any one of claims 1 to 4 or a salt thereof or a hydrate thereof, wherein
X is a methylene group or an ethylene group,
$R^3$ is a group represented by the formula -N($R^4$)COR$^5$,
$R^4$ represents a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group, and
$R^5$ is a heteroaryl group which may be substituted with one or more substituents selected from the substituent $Y^3$ group.

9. The compound according to claim 8 or a salt thereof or a hydrate thereof, wherein $R^5$ is a monocyclic heteroaryl group which may be substituted with one or more substituents selected from the substituent $Y^3$ group.

10. The compound according to claim 9 or a salt thereof or a hydrate thereof, wherein $R^5$ is a thienyl group which may be substituted with one or more substituents selected from the substituent $Y^3$ group.

11. The compound according to any one of claims 1 to 4 or a salt thereof or a hydrate thereof, wherein
X is a methylene group or an ethylene group,
$R^3$ is $-N(R^4)SO_2R^5$,
$R^4$ represents a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; or a $C_{4-9}$ cycloalkylalkyl group which may be substituted with one or more substituents selected from the substituent $Y^2$ group,
$R^5$ is a $C_{1-10}$ alkyl group which may be substituted with one or more substituents selected from the substituent $Y^4$ group, or -($C_{1-3}$ alkylene)-Q or Q, wherein $C_{1-3}$ alkylene may be substituted with one or more substituents selected from a halogen atom and a hydroxyl group, and Q is an aliphatic or aromatic hydrocarbon selected from a $C_{3-10}$ cycloalkyl group which may be substituted with one or more substituents selected from the substituent $Y^3$ group; a $C_{4-10}$ bridged cyclic alkyl group which may be substituted with one or more substituents selected from the substituent $Y^3$ group; a $C_{2-10}$ alkenyl group which may be substituted with one or more substituents selected from the substituent $Y^3$ group; a $C_{3-10}$ cycloalkenyl group which may be substituted with one or more substituents selected from the substituent $Y^3$ group; a $C_{4-10}$ bridged cyclic alkenyl group which may be substituted with one or more substituents selected from the substituent $Y^3$ group; and an aryl group which may be substituted with one or more substituents selected from the substituent $Y^3$ group; or alternatively, Q represents a heterocyclic ring which may be substituted

with one or more substituents selected from the substituent $Y^5$ group, wherein in the aryl group or heterocyclic ring in $R^5$, adjacent substituents attached to the ring member atoms may together form a 5- to 8-membered ring which may contain one or more heteroatoms in its ring, and
$R^4$ and $R^5$ may form, together with the adjacent heteroatom(s), a 4- to 10-membered heterocyclic ring which may be substituted with one or more substituents selected from the substituent $Y^5$ group.

**12.** The compound according to any one of claims 1 to 4 or a salt thereof or a hydrate thereof, wherein
X is a methylene group or an ethylene group, and
$R^3$ is $-NR^4R^6$,
(wherein $R^4$ and $R^6$, which may be the same or different, each represent a hydrogen atom; a $C_{1-10}$ alkyl group which may be substituted with one or more substituents selected from the substituent $Y^4$ group; a $C_{3-6}$ cycloalkyl group which may be substituted with one or more substituents selected from the substituent $Y^2$ group; a $C_{4-9}$ cycloalkylalkyl group which may be substituted with one or more substituents selected from the substituent $Y^2$ group; or an arylalkyl group which may be substituted with one or more substituents selected from the substituent $Y^3$ group, or
$R^4$ and $R^6$ may form, together with the adjacent nitrogen atom, a 4- to 10-membered nitrogen-containing ring which may be substituted with one or more substituents selected from the substituent $Y^5$ group).

**13.** The compound according to any one of claims 1 to 4 or a salt thereof or a hydrate thereof, wherein
X represents a single bond or a methylene group, and
$R^3$ is a group represented by the formula $-CH=CH-R^7$ or $-C\equiv C-R^7$
(wherein $R^7$ represents a hydrogen atom; a $C_{1-6}$ alkyl group which may be substituted with one or more substituents selected from the substituent $Y^1$ group; an aryl group which may be substituted with one or more substituents selected from the substituent $Y^3$ group; or a heteroaryl group which may be substituted with one or more substituents selected from the substituent $Y^3$ group).

**14.** The compound according to any one of claims 1 to 4 or a salt thereof or a hydrate thereof, wherein
X represents a single bond or a methylene group, and
$R^3$ is a 5- or 6-membered heteroaryl or an 8- to 11-membered condensed ring thereof, which contains at least one oxygen and/or sulfur atom and may further contain a nitrogen atom and which may be substituted with one or more substituents selected from the substituent $Y^3$ group.

**15.** The compound according to any one of claims 1 to 4 or a salt thereof or a hydrate thereof, wherein
X represents a single bond or a methylene group, and
$R^3$ is a 6-membered nitrogen-containing aromatic ring or a 9- to 11-membered condensed ring thereof, which may be substituted with one or more substituents selected from the substituent $Y^3$ group.

**16.** A pharmaceutical preparation, which comprises the cyanofluoropyrrolidine compound according to any one of claims 1 to 15 or a pharmaceutically acceptable salt thereof or a hydrate thereof as an active ingredient.

**17.** The pharmaceutical preparation according to claim 16 for use in preventing or treating a disease or condition capable of being improved by inhibition of dipeptidyl peptidase IV.

**18.** The pharmaceutical preparation according to claim 16, wherein the disease or condition capable of being improved by inhibition of dipeptidyl peptidase IV is diabetes mellitus.

**19.** The pharmaceutical preparation according to claim 16, wherein the disease or condition capable of being improved by inhibition of dipeptidyl peptidase IV is an immune disease.

**INTERNATIONAL SEARCH REPORT**

International application No.
PCT/JP2004/006983

A. CLASSIFICATION OF SUBJECT MATTER
Int.Cl[7] C07D207/16, 401/12, 403/12, 405/12, 409/12, 413/12, 417/12, A61K31/40, 31/4025, 31/4155, 31/4184, 31/4192, 31/422, 31/428, 31/433, 31/4439, 31/454, 31/498, A61P3/04, 3/10, 5/50, 13/08,

According to International Patent Classification (IPC) or to both national classification and IPC

B. FIELDS SEARCHED

Minimum documentation searched (classification system followed by classification symbols)
Int.Cl[7] C07D207/16, 401/12, 403/12, 405/12, 409/12, 413/12, 417/12, A61K31/40, 31/4025, 31/4155, 31/4184, 31/4192, 31/422, 31/428, 31/433, 31/4439, 31/454, 31/498

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,A | JP 2004-26820 A (Taisho Pharmaceutical Co., Ltd.), 29 January, 2004 (29.01.04), Claims (Family: none) | 1-19 |
| P,A | WO 03/95425 A1 (Taisho Pharmaceutical Co., Ltd.), 20 November, 2003 (20.11.03), Claims (Family: none) | 1-19 |
| A | WO 02/38541 A1 (Taisho Pharmaceutical Co., Ltd.), 16 May, 2002 (16.05.02), Claims & EP 1333025 A1 & US 2004/72892 A1 | 1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 July, 2004 (13.07.04) | 03 August, 2004 (03.08.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/006983

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-511559 A (Novartis AG.), 05 September, 2000 (05.09.00), Claims & WO 98/19998 A2 & EP 937040 A1 & CN 1236361 A | 1-19 |

Form PCT/ISA/210 (continuation of second sheet) (January 2004)

**INTERNATIONAL SEARCH REPORT**

International application No.
PCT/JP2004/006983

Continuation of A. CLASSIFICATION OF SUBJECT MATTER
(International Patent Classification (IPC))

Int.Cl[7] 17/00, 19/02, 29/00, 37/00, 43/00

(According to International Patent Classification (IPC) or to both national classification and IPC)

Form PCT/ISA/210 (extra sheet) (January 2004)